# EUROPEAN PATENT SPECIFICATION

(11) **EP 2 237 794 B1**
(45) Date of publication and mention of the grant of the patent: **10.04.2013**
(21) Application number: 09709108.6
(22) Date of filing: 23.01.2009
(51) Int. Cl.: A61K 38/16, C07K 19/00, A61P 31/18

(54) **CHIMERIC HIV FUSION PROTEINS AS VACCINES**
CHIMÄRE HIV-FUSIONSPROTEINE ALS IMPFSTOFFE
PROTÉINES HYBRIDES VIH CHIMÈRIQUES UTILISÉES EN TANT QUE VACCINS

(30) Priority: 31.01.2008 US 25094 P
(43) Date of publication of application: 13.10.2010
(73) Proprietor: TheVax Genetics Vaccine Co., Ltd., Taipei (TW)
(72) Inventor: LIAO, Chao-wei, Shin-chu City 300 (TW); CHANG, Hsiu-kang, Taipei 106 (TW)
(74) Representative: McNamara, Kathryn
(86) International application number: PCT/US2009/031859
(87) International publication number: WO 2009/099777

(56) References cited:
- US-A1- 2004 247 617
- US-A1- 2005 106 160
- US-B1- 7 314 632
- CRYZ S J ET AL: "Human immunodeficiency virus-1 principal neutralizing domain peptide-toxin A conjugate vaccine", VACCINE, ELSEVIER LTD, GB, vol. 13, no. 1, 1 January 1995 (1995-01-01), pages 67-71, XP004057703, ISSN: 0264-410X, DOI: DOI:10.1016/0264-410X(95)80013-4
- HERNÁNDEZ M ET AL: "Chimeric synthetic peptide as antigen for immunodiagnosis of HIV-1 infection.", BIOCHEMICAL AND BIOPHYSICAL RESEARCH COMMUNICATIONS 27 MAY 2000 LNKD- PUBMED:10872836, vol. 272, no. 1, 27 May 2000 (2000-05-27), pages 259-262, XP002636483, ISSN: 0006-291X
- MARIN MILENEN HERNÁNDEZ ET AL: "Antigenic activity of three chimeric synthetic peptides of the transmembrane (gp41) and the envelope (gp120) glycoproteins of HIV-1 virus.", PREPARATIVE BIOCHEMISTRY & BIOTECHNOLOGY AUG 2004 LNKD- PUBMED:15461139, vol. 34, no. 3, August 2004 (2004-08), pages 227-237, XP009148200, ISSN: 1082-6068
- HINKULA, J. ET AL.: 'Recognition of prominent viral epitopes induced by immunization with human immunodeficiency virus type 1 regulatory genes' J. VIROL. vol. 71, no. 7, 1997, pages 5528 - 5539
- TAKEDA A ET AL: "TWO RECEPTORS ARE REQUIRED FOR ANTIBODY-DEPENDENT ENHANCEMENT OF HUMAN IMMUNODEFICIENCY VIRUS TYPE 1 INFECTION CD4 AND FC-GAMMA-R", JOURNAL OF VIROLOGY, vol. 64, no. 11, 1990, pages 5605-5610, ISSN: 0022-538X
- JAVIER GUENAGA ET AL: 'Heterologous Epitope-Scaffold Prime:Boosting Immuno-Focuses B Cell Responses to the HIV-1 gp41 2F5 Neutralization Determinant' PLOS ONE vol. 6, no. 1, 01 January 2011, pages E16074 - E16074, XP055016816 DOI: 10.1371/journal.pone.0016074 ISSN: 1932-6203
- GOEDERT J J ET AL: "A PROSPECTIVE STUDY OF HUMAN IMMUNODEFICIENCY VIRUS TYPE 1 INFECTION AND THE DEVELOPMENT OF AIDS IN SUBJECTS WITH HEMOPHILIA", NEW ENGLAND JOURNAL OF MEDICINE, vol. 321, no. 17, 1989, pages 1141-1148, ISSN: 0028-4793
- ALLAIN J-P ET AL: "LONG-TERM EVALUATION OF HIV ANTIGEN AND ANTIBODIES TO P24 AND GP41 IN PATIENTS WITH HEMOPHILIA POTENTIAL CLINICAL IMPORTANCE", NEW ENGLAND JOURNAL OF MEDICINE, vol. 317, no. 18, 1987, pages 1114-1121, ISSN: 0028-4793

### SEQUENCE LISTING

<110> HealthBanks.Co.,Ltd
<120> CHIMERIC HIV FUSION PRTOIENS As VACCINES
<130> 10003-100PCT
<150> US 61/025,094
   <151> 2008-01-31
<160> 252
<210> 1
   <211> 16
   <212> PRT
   <213> Human immunodeficiency virus
<400> 1
   Val Glu Lys Leu Trp Val Thr Val Tyr Tyr Gly Val Pro Val Trp Lys
   1               5                   10                  15
<210> 2
   <211> 25
   <212> PRT
   <213> Human immunodeficiency virus
<400> 2
   Lys Val Val Lys Ile Glu Pro Leu Gly Val Ala Pro Thr Lys Cys Lys
   1               5                   10                  15
   Arg Arg Val Val Gln Arg Glu Lys Arg
               20                  25
<210> 3
   <211> 35
   <212> PRT
   <213> Human immunodeficiency virus
<400> 3
   Cys Thr Arg Pro Ser Asn Asn Thr Arg Lys Gly Ile His Met Gly Pro
   1               5                   10                  15
   Gly Gly Ala Phe Tyr Thr Thr Gly Gln Ile Ile Arg Asn Ile Arg Gln
               20                  25                  30
   Ala His Cys
           35
<210> 4
   <211> 35
   <212> PRT
   <213> Human immunodeficiency virus
<400> 4
   Cys Thr Arg Pro Asn Asn Asn Thr Arg Arg Ser Ile His Ile Glu Pro
   1               5                   10                  15
   Glu Gly Ala Phe Tyr Thr Thr Gly Glu Ile Ile Gly Asp Ile Arg Gln
               20                  25                  30
   Ala His Cys
           35
<210> 5
   <211> 54
   <212> PRT
   <213> Human immunodeficiency virus
<400> 5
   Gln Ala Arg Val Ile Ala Val Glu Arg Tyr Leu Lys Asp Gln Gln Leu
   1               5                   10                  15
   Leu Gly Ile Trp Gly Gly Ser Gly Lys Leu Ile Cys Cys Thr Thr Ala
               20                  25                  30
   Val Pro Trp Asn Ser Ser Trp Ser Asn Lys Leu Asp Arg Ile Trp Asn
           35                  40                  45
   Asn Met Thr Trp Leu Glu
       50
<210> 6
   <211> 82
   <212> PRT
   <213> Artificial sequence
<220>
   <223> Chimera HIV gP120 V3-V3
<400> 6
   Ile Gly Cys Thr Arg Pro Ser Asn Asn Thr Arg Lys Gly Ile His Met
   1               5                   10                  15
   Gly Pro Gly Gly Ala Phe Tyr Thr Thr Gly Gln Ile Ile Arg Asn Ile
               20                  25                  30
   Arg Gln Ala His Cys Gly Leu Leu Gly Gly Cys Thr Arg Pro Asn Asn
           35                  40                  45
   Asn Thr Arg Arg Ser Ile His Ile Glu Pro Glu Gly Ala Phe Tyr Thr
       50                  55                  60
   Thr Gly Glu Ile Ile Gly Asp Ile Arg Gln Ala His Cys Gly Leu Gly
   65                  70                  75                  80
   Leu Glu
<210> 7
   <211> 100
   <212> PRT
   <213> Artificial sequence
<220>
   <223> Chimera HIV gP120-C1-C5-gp41
<400> 7
   Val Glu Lys Leu Trp Val Thr Val Tyr Tyr Gly Val Pro Val Trp Lys
   1               5                   10                  15
   Lys Val Val Lys Ile Glu Pro Leu Gly Val Ala Pro Thr Lys Cys Lys
               20                  25                  30
   Arg Arg Val Val Gln Arg Glu Lys Arg Gly Gly Gly Gly Gly Gln Ala
           35                  40                  45
   Arg Val Ile Ala Val Glu Arg Tyr Leu Lys Asp Gln Gln Leu Leu Gly
       50                  55                  60
   Ile Trp Gly Gly Ser Gly Lys Leu Ile Cys Cys Thr Thr Ala Val Pro
   65                  70                  75                  80
   Trp Asn Ser Ser Trp Ser Asn Lys Leu Asp Arg Ile Trp Asn Asn Met
                   85                  90                  95
   Thr Trp Leu Glu
               100
<210> 8
   <211> 264
   <212> DNA
   <213> Artificial sequence
<220>
   <223> chimeric gene HIV gP120 V3-V3
<400> 8
   gaattccata tggtcgacat cggttgcacc cgtccgagca acaacacccg taaaggtatc      60
   cacatgggcc cgggtggtgc tttctacacc accggtcaga tcatccgtaa catccgtcag     120
   gctcactgtg gtctgctggg tggttgtacc cgtccgaaca acaacacccg tcgtagcatc     180
   cacatcgaac cggaaggtgc tttctacacc accggtgaaa tcatcggtga catccgtcag     240
   gctcactgtg gcctgggtct cgag                                            264
<210> 9
   <211> 318
   <212> DNA
   <213> Artificial sequence
<220>
   <223> chimeric gene HIV gP120 C1-C5-gp41
<400> 9
   gaattccata tggtcgacgt tgaaaaactg tgggttaccg tttactacgg tgttccggtt     60
   tggaaaaaag ttgttaaaat cgaaccgctg ggtgttgctc cgaccaaatg caaacgtcgt    120
   gttgttcagc gtgaaaagcg tggtggcggt ggcggtcaag ctcgtgttat cgctgttgaa    180
   cgttacctga aagaccagca gctgctgggt atctggggtg gtagcggtaa actgatctgc    240
   tgcaccaccg ctgttccgtg gaacagcagc tggagcaaca aactggaccg tatctggaac    300
   aacatgacct ggctcgag                                                  318
<210> 10
   <211> 40
   <212> DNA
   <213> Artificial sequence
<220>
   <223> Forward primer F1 for chimeric gene HIV gP120 V3-V3
<400> 10
   catccgtaac atccgtcagg ctcactgtgg tctgctgggt 40
<210> 11
   <211> 39
   <212> DNA
   <213> Artificial sequence
<220>
   <223> Forward primer F2 for chimeric gene HIV gP120 V3-V3
<400> 11
   gctttctaca ccaccggtca gatcatccgt aacatccgt 39
<210> 12
   <211> 42
   <212> DNA
   <213> Artificial sequence
<220>
   <223> Forward primer F3 for chimeric gene HIV gP120 V3-V3
<400> 12
   aaaggtatcc acatgggccc gggtggtgct ttctacacca cc 42
<210> 13
   <211> 39
   <212> DNA
   <213> Artificial sequence
<220>
   <223> Forward primer F4 for chimeric gene HIV gP120 V3-V3
<400> 13
   acccgtccga gcaacaacac ccgtaaaggt atccacatg 39
<210> 14
   <211> 46
   <212> DNA
   <213> Artificial sequence
<220>
   <223> Forward primer F5 for chimeric gene HIV gP120 V3-V3
<400> 14
   cccgaattcc atatggtcga catcggttgc acccgtccga gcaaca 46
<210> 15
   <211> 39
   <212> DNA
   <213> Artificial sequence
<220>
   <223> Reverse primer R1 for chimeric gene HIV gP120 V3-V3
<400> 15
   gttgttgttc ggacgggtac aaccacccag cagaccaca 39
<210> 16
   <211> 39
   <212> DNA
   <213> Artificial sequence
<220>
   <223> Reverse primer R2 for chimeric gene HIV gP120 V3-V3
<400> 16
   ttcgatgtgg atgctacgac gggtgttgtt gttaggacg 39
<210> 17
   <211> 39
   <212> DNA
   <213> Artificial sequence
<220>
   <223> Reverse primer R3 for chimeric gene HIV gP120 V3-V3
<400> 17
   ggtggtgtag aaagcacctt ccggttcgat gtggatgct 39
<210> 18
   <211> 39
   <212> DNA
   <213> Artificial sequence
<220>
   <223> Reverse primer R4 for chimeric gene HIV gP120 V3-V3
<400> 18
   acggatatca ccgatgattt caccggtggt gtagaaagc 39
<210> 19
   <211> 64
   <212> DNA
   <213> Artificial sequence
<220>
   <223> Reverse primer R5 for chimeric gene HIV gP120 V3-V3
<400> 19
   aaaaaaattc ccattatttt tctcgagacc caggccacag tgagcctgac ggatgtcacc 60
   gatg 64
<210> 20
   <211> 44
   <212> DNA
   <213> Artificial sequence
<220>
   <223> Forward primer F1 for chimeric gene HIV gP120 C1-C4-gp41
<400> 20
   gttgctccga ccaaatgcaa acgtcgtgtt gttcagcgtg aaaa 44
<210> 21
   <211> 47
   <212> DNA
   <213> Artificial sequence
<220>
   <223> Forward primer F2 for chimeric gene HIV gP120 C1-C4-gp41
<400> 21
   aaaaaagttg ttaaaatcga accgctgggt gttgctccga ccaaatg 47
<210> 22
   <211> 49
   <212> DNA
   <213> Artificial sequence
<220>
   <223> Forward primer F3 for chimeric gene HIV gP120 C1-C4-gp41
<400> 22
   gtgggttacc gtttactacg gtgttccggt ttggaaaaaa gttgttaaa 49
<210> 23
   <211> 49
   <212> DNA
   <213> Artificial sequence
<220>
   <223> Forward primer F4 for chimeric gene HIV gP120 C1-C4-gp41
<400> 23
   cccgaattcc atatggtcga cgttgaaaaa ctgtgggtta ccgtttact 49
<210> 24
   <211> 54
   <212> DNA
   <213> Artificial sequence
<220>
   <223> Reverse primer R1 for chimeric gene HIV gP120 C1-C4-gp41
<400> 24
   aacagcgata acacgagctt gaccgccacc gccaccacgc ttttcacgct gaac 54
<210> 25
   <211> 46
   <212> DNA
   <213> Artificial sequence
<220>
   <223> Reverse primer R2 for chimeric gene HIV gP120 C1-C4-gp41
<400> 25
   cagcagctgc tggtctttca ggtaacgttc aacagcgata acacga 46
<210> 26
   <211> 44
   <212> DNA
   <213> Artificial sequence
<220>
   <223> Reverse primer R3 for chimeric gene HIV gP120 C1-C4-gp41
<400> 26
   gatcagttta ccgctaccac cccagatacc cagcagctgc tggt 44
<210> 27
   <211> 45
   <212> DNA
   <213> Artificial sequence
<220>
   <223> Reverse primer R4 for chimeric gene HIV gP120 C1-C4-gp41
<400> 27
   gctgttccac ggaacagcgg tggtgcagca gatcagttta ccgct 45
<210> 28
   <211> 44
   <212> DNA
   <213> Artificial sequence
<220>
   <223> Reverse primer R5 for chimeric gene HIV gP120 C1-C4-gp41
<400> 28
   ccagatacgg tccagtttgt tgctccagct gctgttccac ggaa 44
<210> 29
   <211> 39
   <212> DNA
   <213> Artificial sequence
<220>
   <223> Reverse primer R6 for chimeric gene HIV gP120 C1-C4-gp41
<400> 29
   aaactcgagc caggtcatgt tgttccagat acggtccag 39
<210> 30
   <211> 4
   <212> PRT
   <213> Artificial sequence
<220>
   <223> Carboxyl terminal moiety KDEL
<400> 30
   Lys Asp Glu Leu
   1
<210> 31
   <211> 98
   <212> PRT
   <213> Artificial sequence
<220>
   <223> Chimera for HIV subtype A gp120 C1-C5-gp41
<400> 31
   Ala Glu Asn Leu Trp Val Thr Val Tyr Tyr Gly Val Pro Ile Trp Lys
   1 5 10 15
   Lys Val Val Lys Ile Glu Pro Leu Gly Val Ala Pro Thr Lys Ala Arg
   20 25 30
   Arg Arg Val Val Glu Arg Glu Lys Arg Gly Gly Gly Gly Gly Gln Ala
   35 40 45
   Arg Val Leu Ala Val Glu Arg Tyr Leu Arg Asp Gln Gln Leu Leu Gly
   50 55 60
   Ile Trp Gly Cys Ser Gly Lys Leu Ile Cys Pro Thr Asn Val Pro Trp
   65 70 75 80
   Asn Ser Ser Trp Ser Asn Lys Ser Leu Asp Glu Ile Trp Glu Asn Met
   85 90 95
   Thr Trp
<210> 32
   <211> 98
   <212> PRT
   <213> Artificial sequence
<220>
   <223> Chimera for HIV subtype B gp120 C1-C5-gp41
<400> 32
   Thr Glu Lys Leu Trp Val Thr Val Tyr Tyr Gly Val Pro Val Trp Lys
   1 5 10 15
   Lys Val Val Lys Ile Glu Pro Leu Gly Ile Ala Pro Thr Lys Ala Lys
   20 25 30
   Arg Arg Val Val Gln Arg Glu Lys Arg Gly Gly Gly Gly Gly Gln Ala
   35 40 45
   Arg Val Leu Ala Leu Glu Arg Tyr Leu Arg Asp Gln Gln Leu Leu Gly
   50 55 60
   Ile Trp Gly Cys Ser Gly Lys Leu Ile Cys Thr Thr Thr Val Pro Trp
   65 70 75 80
   Asn Ala Ser Trp Ser Asn Arg Ser Leu Asp Glu Ile Trp Asp Asn Met
   85 90 95
   Thr Trp
<210> 33
   <211> 100
   <212> PRT
   <213> Artificial sequence
<220>
   <223> Chimera for HIV subtype C gp120 C1-C5-gp41
<400> 33
   Met Gly Asn Leu Trp Val Thr Val Tyr Tyr Gly Val Pro Val Trp Lys
   1 5 10 15
   Lys Tyr Lys Val Val Glu Ile Lys Pro Leu Gly Val Ala Pro Thr Lys
   20 25 30
   Pro Lys Arg Arg Val Val Glu Arg Glu Lys Arg Gly Gly Gly Gly Gly
   35 40 45
   Gln Thr Arg Val Leu Ala Ile Glu Arg His Leu Arg Asp Gln Gln Leu
   50 55 60
   Leu Gly Ile Trp Gly Cys Ser Gly Lys Leu Ile Cys Thr Thr Ala Val
   65 70 75 80
   Pro Trp Asn Ser Ser Trp Ser Asn Lys Ser Gln Glu Glu Ile Trp Asp
   85 90 95
   Asn Met Thr Trp
   100
<210> 34
   <211> 98
   <212> PRT
   <213> Artificial sequence
<220>
   <223> Chimera for HIV subtype D gp120 C1-C5-gp41
<400> 34
   Ala Asp Asn Leu Trp Val Thr Val Tyr Tyr Gly Val Pro Val Trp Lys
   1               5                   10                  15
   Lys Val Val Gln Ile Glu Pro Leu Gly Val Ala Pro Thr Arg Ala Lys
               20                  25                  30
   Arg Arg Val Val Glu Arg Glu Lys Arg Gly Gly Gly Gly Gly Gln Ala
           35                  40                  45
   Arg Ile Leu Ala Val Glu Arg Tyr Leu Lys Asp Gln Gln Leu Leu Gly
       50                  55                  60
   Ile Trp Gly Cys Ser Gly Lys His Ile Cys Thr Thr Asn Val Pro Trp
   65                  70                  75                  80
   Asn Ser Ser Trp Ser Asn Arg Ser Leu Asn Glu Ile Trp Gln Asn Met
                   85                  90                  95
   Thr Trp
<210> 35
   <211> 98
   <212> PRT
   <213> Artificial sequence
<220>
   <223> Chimera for HIV subtype E gp120 C1-C5-gp41
<400> 35
   Ser Xaa Asn Leu Trp Val Thr Val Tyr Tyr Gly Val Pro Val Trp Arg
   1               5                   10                  15
   Lys Val Val Gln Ile Glu Pro Leu Gly Ile Ala Pro Thr Arg Pro Lys
               20                  25                  30
   Arg Arg Val Val Glu Arg Glu Lys Arg Gly Gly Gly Gly Gly Gln Ala
           35                  40                  45
   Arg Val Leu Ala Val Glu Arg Tyr Leu Lys Asp Gln Lys Phe Leu Gly
       50                  55                  60
   Leu Trp Gly Cys Ser Gly Lys Ile Ile Cys Thr Thr Ala Val Pro Trp
   65                  70                  75                  80
   Asn Ser Ser Trp Ser Asn Arg Ser Phe Glu Glu Ile Trp Asn Asn Met
                   85                  90                  95
   Thr Trp
<210> 36
   <211> 98
   <212> PRT
   <213> Artificial sequence
<220>
   <223> Chimera for HIV subtype F gp120 C1-C5-gp41
<400> 36
   Ala Asp Asn Leu Trp Val Thr Val Tyr Tyr Gly Val Pro Val Trp Lys
   1               5                   10                  15
   Lys Val Val Glu Ile Glu Pro Leu Gly Val Ala Pro Thr Lys Ala Lys
               20                  25                  30
   Arg Gln Val Val Gln Arg Glu Lys Arg Gly Gly Gly Gly Gly Gln Ala
           35                  40                  45
   Arg Val Leu Ala Val Glu Arg Tyr Leu Lys Asp Gln Gln Leu Leu Gly
       50                  55                  60
   Ile Trp Gly Cys Ser Gly Lys Leu Ile Cys Thr Thr Asn Val Pro Trp
   65                  70                  75                  80
   Asn Ser Ser Trp Ser Asn Lys Ser Gln Glu Glu Ile Trp Asn Asn Met
                   85                  90                  95
   Thr Trp
<210> 37
   <211> 102
   <212> PRT
   <213> Artificial sequence
<220>
   <223> Chimera for HIV subtype G gp120 C1-C5-gp41
<400> 37
   Ala Ser Asn Asn Leu Trp Val Thr Val Tyr Tyr Gly Val Pro Val Trp
   1               5                   10                  15
   Glu Asp Ala Lys Lys Val Val Lys Ile Lys Pro Leu Gly Val Ala Pro
               20                  25                  30
   Thr Lys Ala Arg Arg Arg Val Val Gly Arg Glu Lys Arg Gly Gly Gly
           35                  40                  45
   Gly Gly Gln Ala Arg Val Leu Ala Leu Glu Arg Tyr Leu Arg Asp Gln
       50                  55                  60
   Gln Leu Leu Gly Ile Trp Gly Cys Ser Gly Lys Leu Ile Cys Thr Thr
   65                  70                  75                  80
   Asn Val Pro Trp Asn Ala Ser Trp Ser Asn Lys Thr Tyr Asn Asp Ile
                   85                  90                  95
   Trp Asp Asn Met Thr Trp
               100
<210> 38
   <211> 102
   <212> PRT
   <213> Artificial sequence
<220>
   <223> Chimera for HIV subtype H gp120 C1-C5-gp41
<400> 38
   Val Val Gly Asn Leu Trp Val Thr Val Tyr Tyr Gly Val Pro Val Trp
   1               5                   10                  15
   Lys Lys Val Val Lys Ile Glu Pro Leu Gly Val Ala Pro Thr Glu Ala
               20                  25                  30
   Arg Arg Arg Val Val Glu Arg Glu Lys Arg Gly Gly Gly Gly Gly Gln
           35                  40                  45
   Ala Arg Val Leu Ala Val Glu Arg Tyr Leu Lys Asp Gln Gln Leu Leu
       50                  55                  60
   Gly Ile Trp Gly Cys Ser Gly Lys Leu Ile Cys Thr Thr Asn Val Pro
   65                  70                  75                  80
   Trp Asn Ser Ser Trp Ser Asn Lys Ser Leu Asp Glu Ile Trp Asp Asn
                   85                  90                  95
   Met Thr Trp Met Glu Trp
               100
<210> 39
   <211> 99
   <212> PRT
   <213> Artificial sequence
<220>
   <223> Chimera for HIV subtype J gp120 C1-C5-gp41
<400> 39
   Ala Lys Glu Asp Leu Trp Val Thr Val Tyr Tyr Gly Val Pro Val Trp
   1               5                   10                  15
   Lys Lys Val Val Glu Ile Glu Pro Leu Gly Val Ala Pro Thr Lys Ala
               20                  25                  30
   Lys Arg Arg Val Val Glu Arg Glu Lys Arg Gly Gly Gly Gly Gly Gln
           35                  40                  45
   Ala Arg Val Leu Ala Val Glu Arg Tyr Leu Lys Asp Gln Gln Leu Leu
       50                  55                  60
   Gly Ile Trp Gly Cys Ser Gly Lys Leu Ile Cys Thr Thr Asn Val Pro
   65                  70                  75                  80
   Trp Asn Ala Ser Trp Ser Asn Lys Ser Tyr Glu Asp Ile Trp Glu Asn
                   85                  90                  95
   Met Thr Trp
<210> 40
   <211> 99
   <212> PRT
   <213> Artificial sequence
<220>
   <223> Chimera for HIV subtype K gp120 C1-C5-gp41
<400> 40
   Ala Ala Asn Asn Leu Trp Val Thr Val Tyr Tyr Gly Val Pro Val Trp
   1               5                   10                  15
   Lys Lys Val Val Gln Ile Glu Pro Leu Gly Ile Ala Pro Thr Arg Ala
               20                  25                  30
   Arg Arg Arg Val Val Gln Arg Glu Lys Arg Gly Gly Gly Gly Gly Arg
           35                  40                  45
   Ala Arg Val Leu Ala Val Glu Arg Tyr Leu Arg Asp Gln Gln Leu Leu
       50                  55                  60
   Gly Ile Trp Gly Cys Ser Gly Lys Leu Ile Cys Thr Thr Asn Val Pro
   65                  70                  75                  80
   Trp Asn Ser Ser Trp Ser Asn Lys Ser Gln Ser Glu Ile Trp Glu Asn
                   85                  90                  95
   Met Thr Trp
<210> 41
   <211> 294
   <212> DNA
   <213> Artificial sequence
<220>
   <223> Chimeric gene for HIV subtype A gp120 C1-C5-gp41
<400> 41
   gctgaaaacc tgtgggttac cgtttactac ggtgttccaa tctggaaaaa agttgttaaa      60
   atcgaaccac tgggtgttgc tccaaccaaa gctcgtcgtc gtgttgttga acgtgaaaaa     120
   cgtggtggtg gtggtggtca ggctcgtgtt ctggctgttg aacgttacct gcgtgaccag     180
   cagctgctgg gtatctgggg ttgttccggt aaactgatct gtccaaccaa cgttccatgg     240
   aactcctcct ggtccaacaa atccctggac gaaatctggg aaaacatgac ctgg           294
<210> 42
   <211> 294
   <212> DNA
   <213> Artificial sequence
<220>
   <223> Chimeric gene for HIV subtype B gp120 C1-C5-gp41
<400> 42
   accgaaaaac tgtgggttac cgtttactac ggtgttccag tgtggaaaaa agttgttaaa      60
   atcgaaccac tgggtatcgc tccaaccaaa gctaaacgtc gtgttgttca gcgtgaaaaa     120
   cgtggtggtg gtggtggtca ggctcgtgtt ctggctctgg aacgttacct gcgtgaccag     180
   cagctgctgg gtatctgggg ttgttccggt aaactgatct gtaccaccac cgttccatgg     240
   aacgcttcct ggtccaaccg ttccctggac gaaatctggg acaacatgac ctgg           294
<210> 43
   <211> 300
   <212> DNA
   <213> Artificial sequence
<220>
   <223> Chimeric gene for HIV subtype C gp120 C1-C5-gp41
<400> 43
   atgggtaacc tgtgggttac cgtttactac ggtgttccag tgtggaaaaa atacaaagtt      60
   gttgaaatca aaccactggg tgttgctcca accaaaccaa aacgtcgtgt tgttgaacgt     120
   gaaaaacgtg gtggtggtgg tggtcagacc cgtgttctgg ctatcgaacg tcacctgcgt     180
   gaccagcagc tgctgggtat ctggggttgt tccggtaaac tgatctgtac caccgctgtt     240
   ccatggaact cctcctggtc caacaaatcc caggaagaaa tctgggacaa catgacctgg     300
<210> 44
   <211> 294
   <212> DNA
   <213> Artificial sequence
<220>
   <223> Chimeric gene for HIV subtype D gp120 C1-C5-gp41
<400> 44
   gctgacaacc tgtgggttac cgtttactac ggtgttccag tgtggaaaaa agttgttcag      60
   atcgaaccac tgggtgttgc tccaacccgt gctaaacgtc gtgttgttga acgtgaaaaa     120
   cgtggtggtg gtggtggtca ggctcgtatt ctggctgttg aacgttacct gaaagaccag     180
   cagctgctgg gtatctgggg ttgttccggt aaacacatct gtaccaccaa cgttccatgg     240
   aactcctcct ggtccaaccg ttccctgaac gaaatctggc agaacatgac ctgg           294
<210> 45
   <211> 294
   <212> DNA
   <213> Artificial sequence
<220>
   <223> Chimeric gene for HIV subtype E gp120 C1-C5-gp41
<400> 45
   tccaacaacc tgtgggttac cgtttactac ggtgttccag tgtggcgtaa agttgttcag 60
   atcgaaccac tgggtatcgc tccaacccgt ccaaaacgtc gtgttgttga acgtgaaaaa 120
   cgtggtggtg gtggtggtca ggctcgtgtt ctggctgttg aacgttacct gaaagaccag 180
   aaattcctgg gtctgtgggg ttgttccggt aaaatcatct gtaccaccgc tgttccatgg 240
   aactcctcct ggtccaaccg ttccttcgaa gaaatctgga acaacatgac ctgg 294
<210> 46
   <211> 294
   <212> DNA
   <213> Artificial sequence
<220>
   <223> Chimeric gene for HIV subtype F gp120 C1-C5-gp41
<400> 46
   gctgacaacc tgtgggttac cgtttactac ggtgttccag tgtggaaaaa agttgttgaa 60
   atcgaaccac tgggtgttgc tccaaccaaa gctaaacgtc aggttgttca gcgtgaaaaa 120
   cgtggtggtg gtggtggtca ggctcgtgtt ctggctgttg aacgttacct gaaagaccag 180
   cagctgctgg gtatctgggg ttgttccggt aaactgatct gtaccaccaa cgttccatgg 240
   aactcctcct ggtccaacaa atcccaggaa gaaatctgga acaacatgac ctgg 294
<210> 47
   <211> 306
   <212> DNA
   <213> Artificial sequence
<220>
   <223> Chimeric gene for HIV subtype G gp120 C1-C5-gp41
<400> 47
   gcttccaaca acctgtgggt taccgtttac tacggtgttc cagtgtggga agacgctaaa 60
   aaagttgtta aaatcaaacc actgggtgtt gctccaacca aagctcgtcg tcgtgttgtt 120
   ggtcgtgaaa aacgtggtgg tggtggtggt caggctcgtg ttctggctct ggaacgttac 180
   ctgcgtgacc agcagctgct gggtatctgg ggttgttccg gtaaactgat ctgtaccacc 240
   aacgttccat ggaacgcttc ctggtccaac aaaacctaca acgacatctg ggacaacatg 300
   acctgg 306
<210> 48
   <211> 306
   <212> DNA
   <213> Artificial sequence
<220>
   <223> Chimeric gene for HIV subtype H gp120 C1-C5-gp41
<400> 48
   gttgttggta acctgtgggt taccgtttac tacggtgttc cagtgtggaa aaaagttgtt      60
   aaaatcgaac cactgggtgt tgctccaacc gaagctcgtc gtcgtgttgt tgaacgtgaa     120
   aaacgtggtg gtggtggtgg tcaggctcgt gttctggctg ttgaacgtta cctgaaagac     180
   cagcagctgc tgggtatctg gggttgttcc ggtaaactga tctgtaccac caacgttcca     240
   tggaactcct cctggtccaa caaatccctg gacgaaatct gggacaacat gacctggatg     300
   gaatgg                                                                306
<210> 49
   <211> 297
   <212> DNA
   <213> Artificial sequence
<220>
   <223> Chimeric gene for HIV subtype J gp120 C1-C5-gp41
<400> 49
   gctaaagaag acctgtgggt taccgtttac tacggtgttc cagtgtggaa aaaagttgtt      60
   gaaatcgaac cactgggtgt tgctccaacc aaagctaaac gtcgtgttgt tgaacgtgaa     120
   aaacgtggtg gtggtggtgg tcaggctcgt gttctggctg ttgaacgtta cctgaaagac     180
   cagcagctgc tgggtatctg gggttgttcc ggtaaactga tctgtaccac caacgttcca     240
   tggaacgctt cctggtccaa caaatcctac gaagacatct gggaaaacat gacctgg        297
<210> 50
   <211> 297
   <212> DNA
   <213> Artificial sequence
<220>
   <223> Chimeric gene for HIV subtype K gp120 C1-C5-gp41
<400> 50
   gctgctaaca acctgtgggt taccgtttac tacggtgttc cagtgtggaa aaaagttgtt 60
   cagatcgaac cactgggtat cgctccaacc cgtgctcgtc gtcgtgttgt tcagcgtgaa 120
   aaacgtggtg gtggtggtgg tcgtgctcgt gttctggctg ttgaacgtta cctgcgtgac 180
   cagcagctgc tgggtatctg gggttgttcc ggtaaactga tctgtaccac caacgttcca 240
   tggaactcct cctggtccaa caaatcccag tccgaaatct gggaaaacat gacctgg 297
<210> 51
   <211> 45
   <212> DNA
   <213> Artificial sequence
<220>
   <223> Forward primer F1 for chimeric gene HIV subtype A gp120 C1-C5-gp41
<400> 51
   gttgaacgtg aaaaacgtgg tggtggtggt ggtcaggctc gtgtt 45
<210> 52
   <211> 45
   <212> DNA
   <213> Artificial sequence
<220>
   <223> Forward primer F2 for chimeric gene HIV subtype A gp120 C1-C5-gp41
<400> 52
   gttgctccaa ccaaagctcg tcgtcgtgtt gttgaacgtg aaaaa 45
<210> 53
   <211> 49
   <212> DNA
   <213> Artificial sequence
<220>
   <223> Forward primer F3 for chimeric gene HIV subtype A gp120 C1-C5-gp41
<400> 53
   ctggaaaaaa gttgttaaaa tcgaaccact gggtgttgct ccaaccaaa 49
<210> 54
   <211> 46
   <212> DNA
   <213> Artificial sequence
<220>
   <223> Forward primer F4 for chimeric gene HIV subtype A gp120 C1-C5-gp41
<400> 54
   gtgggttacc gtttactacg gtgttccaat ctggaaaaaa gttgtt 46
<210> 55
   <211> 47
   <212> DNA
   <213> Artificial sequence
<220>
   <223> Forward primer F5 for chimeric gene HIV subtype A gp120 C1-C5-gp41
<400> 55
   cccgaattcc atatggtcga cgctgaaaac ctgtgggtta ccgttta 47
<210> 56
   <211> 47
   <212> DNA
   <213> Artificial sequence
<220>
   <223> Reverse primer R1 for chimeric gene HIV subtype A gp120 C1-C5-gp41
<400> 56
   tgctggtcac gcaggtaacg ttcaacagcc agaacacgag cctgacc 47
<210> 57
   <211> 46
   <212> DNA
   <213> Artificial sequence
<220>
   <223> Reverse primer R2 for chimeric gene HIV subtype A gp120 C1-C5-gp41
<400> 57
   tttaccggaa caaccccaga tacccagcag ctgctggtca cgcagg 46
<210> 58
   <211> 46
   <212> DNA
   <213> Artificial sequence
<220>
   <223> Reverse primer R3 for chimeric gene HIV subtype A gp120 C1-C5-gp41
<400> 58
   agttccatgg aacgttggtt ggacagatca gtttaccgga acaacc 46
<210> 59
   <211> 48
   <212> DNA
   <213> Artificial sequence
<220>
   <223> Reverse primer R4 for chimeric gene HIV subtype A gp120 C1-C5-gp41
<400> 59
   tttcgtccag ggatttgttg gaccaggagg agttccatgg aacgttgg 48
<210> 60
   <211> 49
   <212> DNA
   <213> Artificial sequence
<220>
   <223> Reverse primer R5 for chimeric gene HIV subtype A gp120 C1-C5-gp41
<400> 60
   atttttctcg agccaggtca tgttttccca gatttcgtcc agggatttg 49
<210> 61
   <211> 45
   <212> DNA
   <213> Artificial sequence
<220>
   <223> Forward primer F1 for chimeric gene HIV subtype B gp120 C1-C5-gp41
<400> 61
   gttcagcgtg aaaaacgtgg tggtggtggt ggtcaggctc gtgtt 45
<210> 62
   <211> 46
   <212> DNA
   <213> Artificial sequence
<220>
   <223> Forward primer F2 for chimeric gene HIV subtype B gp120 C1-C5-gp41
<400> 62
   atcgctccaa ccaaagctaa acgtcgtgtt gttcagcgtg aaaaac 46
<210> 63
   <211> 49
   <212> DNA
   <213> Artificial sequence
<220>
   <223> Forward primer F3 for chimeric gene HIV subtype B gp120 C1-C5-gp41
<400> 63
   gtggaaaaaa gttgttaaaa tcgaaccact gggtatcgct ccaaccaaa 49
<210> 64
   <211> 46
   <212> DNA
   <213> Artificial sequence
<220>
   <223> Forward primer F4 for chimeric gene HIV subtype B gp120 C1-C5-gp41
<400> 64
   gtgggttacc gtttactacg gtgttccagt gtggaaaaaa gttgtt 46
<210> 65
   <211> 47
   <212> DNA
   <213> Artificial sequence
<220>
   <223> Forward primer F5 for chimeric gene HIV subtype B gp120 C1-C5-gp41
<400> 65
   cccgaattcc atatggtcga caccgaaaaa ctgtgggtta ccgttta 47
<210> 66
   <211> 47
   <212> DNA
   <213> Artificial sequence
<220>
   <223> Reverse primer R1 for chimeric gene HIV subtype B gp120 C1-C5-gp41
<400> 66
   tgctggtcac gcaggtaacg ttccagagcc agaacacgag cctgacc 47
<210> 67
   <211> 46
   <212> DNA
   <213> Artificial sequence

<220>
   <223> Reverse primer R2 for chimeric gene HIV subtype B gp120 C1-C5-gp41
<400> 67
   tttaccggaa caaccccaga tacccagcag ctgctggtca cgcagg 46
<210> 68
   <211> 48
   <212> DNA
   <213> Artificial sequence
<220>
   <223> Reverse primer R3 for chimeric gene HIV subtype B gp120 C1-C5-gp41
<400> 68
   agcgttccat ggaacggtgg tggtacagat cagtttaccg gaacaacc 48
<210> 69
   <211> 44
   <212> DNA
   <213> Artificial sequence
<220>
   <223> Reverse primer R4 for chimeric gene HIV subtype B gp120 C1-C5-gp41
<400> 69
   atttcgtcca gggaacggtt ggaccaggaa gcgttccatg gaac 44
<210> 70
   <211> 47
   <212> DNA
   <213> Artificial sequence
<220>
   <223> Reverse primer R5 for chimeric gene HIV subtype B gp120 C1-C5-gp41
<400> 70
   atttttctcg agccaggtca tgttgtccca gatttcgtcc agggaac 47
<210> 71
   <211> 45
   <212> DNA
   <213> Artificial sequence
<220>
   <223> Forward primer F1 for chimeric gene HIV subtype C gp120 C1-C5-gp41
<400> 71
   gttgaacgtg aaaaacgtgg tggtggtggt ggtcagaccc gtgtt 45
<210> 72
   <211> 45
   <212> DNA
   <213> Artificial sequence
<220>
   <223> Forward primer F2 for chimeric gene HIV subtype C gp120 C1-C5-gp41
<400> 72
   gttgctccaa ccaaaccaaa acgtcgtgtt gttgaacgtg aaaaa 45
<210> 73
   <211> 52
   <212> DNA
   <213> Artificial sequence
<220>
   <223> Forward primer F3 for chimeric gene HIV subtype C gp120 C1-C5-gp41
<400> 73
   aaaaaataca aagttgttga aatcaaacca ctgggtgttg ctccaaccaa ac 52
<210> 74
   <211> 51
   <212> DNA
   <213> Artificial sequence
<220>
   <223> Forward primer F4 for chimeric gene HIV subtype C gp120 C1-C5-gp41
<400> 74
   ctgtgggtta ccgtttacta cggtgttcca gtgtggaaaa aatacaaagt t 51
<210> 75
   <211> 45
   <212> DNA
   <213> Artificial sequence
<220>
   <223> Forward primer F5 for chimeric gene HIV subtype C gp120 C1-C5-gp41
<400> 75
   cccgaattcc atatggtcga catgggtaac ctgtgggtta ccgtt 45
<210> 76
   <211> 47
   <212> DNA
   <213> Artificial sequence
<220>
   <223> Reverse primer R1 for chimeric gene HIV subtype C gp120 C1-C5-gp41
<400> 76
   tgctggtcac gcaggtgacg ttcgatagcc agaacacggg tctgacc 47
<210> 77
   <211> 46
   <212> DNA
   <213> Artificial sequence
<220>
   <223> Reverse primer R2 for chimeric gene HIV subtype C gp120 C1-C5-gp41
<400> 77
   tttaccggaa caaccccaga tacccagcag ctgctggtca cgcagg 46
<210> 78
   <211> 46
   <212> DNA
   <213> Artificial sequence
<220>
   <223> Reverse primer R3 for chimeric gene HIV subtype C gp120 C1-C5-gp41
<400> 78
   agttccatgg aacagcggtg gtacagatca gtttaccgga acaacc 46
<210> 79
   <211> 48
   <212> DNA
   <213> Artificial sequence
<220>
   <223> Reverse primer R4 for chimeric gene HIV subtype C gp120 C1-C5-gp41
<400> 79
   tttcttcctg ggatttgttg gaccaggagg agttccatgg aacagcgg 48
<210> 80
   <211> 49
   <212> DNA
   <213> Artificial sequence
<220>
   <223> Reverse primer R5 for chimeric gene HIV subtype C gp120 C1-C5-gp41
<400> 80
   atttttctcg agccaggtca tgttgtccca gatttcttcc tgggatttg 49
<210> 81
   <211> 45
   <212> DNA
   <213> Artificial sequence
<220>
   <223> Forward primer F1 for chimeric gene HIV subtype D gp120 C1-C5-gp41
<400> 81
   gttgaacgtg aaaaacgtgg tggtggtggt ggtcaggctc gtatt 45
<210> 82
   <211> 45
   <212> DNA
   <213> Artificial sequence
<220>
   <223> Forward primer F2 for chimeric gene HIV subtype D gp120 C1-C5-gp41
<400> 82
   gttgctccaa cccgtgctaa acgtcgtgtt gttgaacgtg aaaaa 45
<210> 83
   <211> 49
   <212> DNA
   <213> Artificial sequence
<220>
   <223> Forward primer F3 for chimeric gene HIV subtype D gp120 C1-C5-gp41
<400> 83
   gtggaaaaaa gttgttcaga tcgaaccact gggtgttgct ccaacccgt 49
<210> 84
   <211> 46
   <212> DNA
   <213> Artificial sequence
<220>
   <223> Forward primer F4 for chimeric gene HIV subtype D gp120 C1-C5-gp41
<400> 84
   gtgggttacc gtttactacg gtgttccagt gtggaaaaaa gttgtt 46
<210> 85
   <211> 47
   <212> DNA
   <213> Artificial sequence
<220>
   <223> Forward primer F5 for chimeric gene HIV subtype D gp120 C1-C5-gp41
<400> 85
   cccgaattcc atatggtcga cgctgacaac ctgtgggtta ccgttta 47
<210> 86
   <211> 47
   <212> DNA
   <213> Artificial sequence
<220>
   <223> Reverse primer R1 for chimeric gene HIV subtype D gp120 C1-C5-gp41
<400> 86
   tgctggtctt tcaggtaacg ttcaacagcc agaatacgag cctgacc 47
<210> 87
   <211> 46
   <212> DNA
   <213> Artificial sequence
<220>
   <223> Reverse primer R2 for chimeric gene HIV subtype D gp120 C1-C5-gp41
<400> 87
   tttaccggaa caaccccaga tacccagcag ctgctggtct ttcagg 46
<210> 88
   <211> 46
   <212> DNA
   <213> Artificial sequence
<220>
   <223> Reverse primer R3 for chimeric gene HIV subtype D gp120 C1-C5-gp41
<400> 88
   agttccatgg aacgttggtg gtacagatgt gtttaccgga acaacc 46
<210> 89
   <211> 48
   <212> DNA
   <213> Artificial sequence
<220>
   <223> Reverse primer R4 for chimeric gene HIV subtype D gp120 C1-C5-gp41
<400> 89
   tttcgttcag ggaacggttg gaccaggagg agttccatgg aacgttgg 48
<210> 90
   <211> 49
   <212> DNA
   <213> Artificial sequence
<220>
   <223> Reverse primer R5 for chimeric gene HIV subtype D gp120 C1-C5-gp41
<400> 90
   atttttctcg agccaggtca tgttctgcca gatttcgttc agggaacgg 49
<210> 91
   <211> 45
   <212> DNA
   <213> Artificial sequence
<220>
   <223> Forward primer F1 for chimeric gene HIV subtype E gp120 C1-C5-gp41
<400> 91
   gttgaacgtg aaaaacgtgg tggtggtggt ggtcaggctc gtgtt 45
<210> 92
   <211> 46
   <212> DNA
   <213> Artificial sequence
<220>
   <223> Forward primer F2 for chimeric gene HIV subtype E gp120 C1-C5-gp41
<400> 92
   atcgctccaa cccgtccaaa acgtcgtgtt gttgaacgtg aaaaac 46
<210> 93
   <211> 49
   <212> DNA
   <213> Artificial sequence
<220>
   <223> Forward primer F3 for chimeric gene HIV subtype E gp120 C1-C5-gp41
<400> 93
   gtggcgtaaa gttgttcaga tcgaaccact gggtatcgct ccaacccgt 49
<210> 94
   <211> 46
   <212> DNA
   <213> Artificial sequence
<220>
   <223> Forward primer F4 for chimeric gene HIV subtype E gp120 C1-C5-gp41
<400> 94
   gtgggttacc gtttactacg gtgttccagt gtggcgtaaa gttgtt 46
<210> 95
   <211> 44
   <212> DNA
   <213> Artificial sequence
<220>
   <223> Forward primer F5 for chimeric gene HIV subtype E gp120 C1-C5-gp41
<400> 95
   cccgaattcc atatggtcga ctccaacctg tgggttaccg ttta 44
<210> 96
   <211> 48
   <212> DNA
   <213> Artificial sequence
<220>
   <223> Reverse primer R1 for chimeric gene HIV subtype E gp120 C1-C5-gp41
<400> 96
   tttctggtct ttcaggtaac gttcaacagc cagaacacga gcctgacc 48
<210> 97
   <211> 46
   <212> DNA
   <213> Artificial sequence
<220>
   <223> Reverse primer R2 for chimeric gene HIV subtype E gp120 C1-C5-gp41
<400> 97
   tttaccggaa caaccccaca gacccaggaa tttctggtct ttcagg 46
<210> 98
   <211> 46
   <212> DNA
   <213> Artificial sequence
<220>
   <223> Reverse primer R3 for chimeric gene HIV subtype E gp120 C1-C5-gp41
<400> 98
   agttccatgg aacagcggtg gtacagatga ttttaccgga acaacc 46
<210> 99
   <211> 48
   <212> DNA
   <213> Artificial sequence
<220>
   <223> Reverse primer R4 for chimeric gene HIV subtype E gp120 C1-C5-gp41
<400> 99
   tttcttcgaa ggaacggttg gaccaggagg agttccatgg aacagcgg 48
<210> 100
   <211> 49
   <212> DNA
   <213> Artificial sequence
<220>
   <223> Reverse primer R5 for chimeric gene HIV subtype E gp120 C1-C5-gp41
<400> 100
   atttttctcg agccaggtca tgttgttcca gatttcttcg aaggaacgg 49
<210> 101
   <211> 45
   <212> DNA
   <213> Artificial sequence
<220>
   <223> Forward primer F1 for chimeric gene HIV subtype F gp120 C1-C5-gp41
<400> 101
   gttcagcgtg aaaaacgtgg tggtggtggt ggtcaggctc gtgtt 45
<210> 102
   <211> 45
   <212> DNA
   <213> Artificial sequence
<220>
   <223> Forward primer F2 for chimeric gene HIV subtype F gp120 C1-C5-gp41
<400> 102
   gttgctccaa ccaaagctaa acgtcaggtt gttcagcgtg aaaaa 45
<210> 103
   <211> 49
   <212> DNA
   <213> Artificial sequence
<220>
   <223> Forward primer F3 for chimeric gene HIV subtype F gp120 C1-C5-gp41
<400> 103
   gtggaaaaaa gttgttgaaa tcgaaccact gggtgttgct ccaaccaaa 49
<210> 104
   <211> 46
   <212> DNA
   <213> Artificial sequence
<220>
   <223> Forward primer F4 for chimeric gene HIV subtype F gp120 C1-C5-gp41
<400> 104
   gtgggttacc gtttactacg gtgttccagt gtggaaaaaa gttgtt 46
<210> 105
   <211> 47
   <212> DNA
   <213> Artificial sequence
<220>
   <223> Forward primer F5 for chimeric gene HIV subtype F gp120 C1-C5-gp41
<400> 105
   cccgaattcc atatggtcga cgctgacaac ctgtgggtta ccgttta 47
<210> 106
   <211> 47
   <212> DNA
   <213> Artificial sequence
<220>
   <223> Reverse primer R1 for chimeric gene HIV subtype F gp120 C1-C5-gp41
<400> 106
   tgctggtctt tcaggtaacg ttcaacagcc agaacacgag cctgacc 47
<210> 107
   <211> 46
   <212> DNA
   <213> Artificial sequence
<220>
   <223> Reverse primer R2 for chimeric gene HIV subtype F gp120 C1-C5-gp41
<400> 107
   tttaccggaa caaccccaga tacccagcag ctgctggtct ttcagg 46
<210> 108
   <211> 46
   <212> DNA
   <213> Artificial sequence
<220>
   <223> Reverse primer R3 for chimeric gene HIV subtype F gp120 C1-C5-gp41
<400> 108
   agttccatgg aacgttggtg gtacagatca gtttaccgga acaacc 46
<210> 109
   <211> 48
   <212> DNA
   <213> Artificial sequence
<220>
   <223> Reverse primer R4 for chimeric gene HIV subtype F gp120 C1-C5-gp41
<400> 109
   tttcttcctg ggatttgttg gaccaggagg agttccatgg aacgttgg 48
<210> 110
   <211> 49
   <212> DNA
   <213> Artificial sequence
<220>
   <223> Reverse primer R5 for chimeric gene HIV subtype F gp120 C1-C5-gp41
<400> 110
   atttttctcg agccaggtca tgttgttcca gatttcttcc tgggatttg 49
<210> 111
   <211> 45
   <212> DNA
   <213> Artificial sequence
<220>
   <223> Forward primer F1 for chimeric gene HIV subtype G gp120 C1-C5-gp41
<400> 111
   gttggtcgtg aaaaacgtgg tggtggtggt ggtcaggctc gtgtt 45
<210> 112
   <211> 45
   <212> DNA
   <213> Artificial sequence
<220>
   <223> Forward primer F2 for chimeric gene HIV subtype G gp120 C1-C5-gp41
<400> 112
   gttgctccaa ccaaagctcg tcgtcgtgtt gttggtcgtg aaaaa 45
<210> 113
   <211> 51
   <212> DNA
   <213> Artificial sequence
<220>
   <223> Forward primer F3 for chimeric gene HIV subtype G gp120 C1-C5-gp41
<400> 113
   gacgctaaaa aagttgttaa aatcaaacca ctgggtgttg ctccaaccaa a 51
<210> 114
   <211> 52
   <212> DNA
   <213> Artificial sequence
<220>
   <223> Forward primer F4 for chimeric gene HIV subtype G gp120 C1-C5-gp41
<400> 114
   gtgggttacc gtttactacg gtgttccagt gtgggaagac gctaaaaaag tt 52
<210> 115
   <211> 50
   <212> DNA
   <213> Artificial sequence
<220>
   <223> Forward primer F5 for chimeric gene HIV subtype G gp120 C1-C5-gp41
<400> 115
   cccgaattcc atatggtcga cgcttccaac aacctgtggg ttaccgttta 50
<210> 116
   <211> 47
   <212> DNA
   <213> Artificial sequence
<220>
   <223> Reverse primer R1 for chimeric gene HIV subtype G gp120 C1-C5-gp41
<400> 116
   tgctggtcac gcaggtaacg ttccagagcc agaacacgag cctgacc 47
<210> 117
   <211> 46
   <212> DNA
   <213> Artificial sequence
<220>
   <223> Reverse primer R2 for chimeric gene HIV subtype G gp120 C1-C5-gp41
<400> 117
   tttaccggaa caaccccaga tacccagcag ctgctggtca cgcagg 46
<210> 118
   <211> 48
   <212> DNA
   <213> Artificial sequence
<220>
   <223> Reverse primer R3 for chimeric gene HIV subtype G gp120 C1-C5-gp41
<400> 118
   agcgttccat ggaacgttgg tggtacagat cagtttaccg gaacaacc 48
<210> 119
   <211> 44
   <212> DNA
   <213> Artificial sequence
<220>
   <223> Reverse primer R4 for chimeric gene HIV subtype G gp120 C1-C5-gp41
<400> 119
   atgtcgttgt aggttttgtt ggaccaggaa gcgttccatg gaac 44
<210> 120
   <211> 46
   <212> DNA
   <213> Artificial sequence
<220>
   <223> Reverse primer R5 for chimeric gene HIV subtype G gp120 C1-C5-gp41
<400> 120
   tttctcgagc caggtcatgt tgtcccagat gtcgttgtag gttttg 46
<210> 121
   <211> 45
   <212> DNA
   <213> Artificial sequence
<220>
   <223> Forward primer F1 for chimeric gene HIV subtype H gp120 C1-C5-gp41
<400> 121
   gttgaacgtg aaaaacgtgg tggtggtggt ggtcaggctc gtgtt 45
<210> 122
   <211> 45
   <212> DNA
   <213> Artificial sequence
<220>
   <223> Forward primer F2 for chimeric gene HIV subtype H gp120 C1-C5-gp41
<400> 122
   gttgctccaa ccgaagctcg tcgtcgtgtt gttgaacgtg aaaaa 45
<210> 123
   <211> 49
   <212> DNA
   <213> Artificial sequence
<220>
   <223> Forward primer F3 for chimeric gene HIV subtype H gp120 C1-C5-gp41
<400> 123
   gtggaaaaaa gttgttaaaa tcgaaccact gggtgttgct ccaaccgaa 49
<210> 124
   <211> 48
   <212> DNA
   <213> Artificial sequence
<220>
   <223> Forward primer F4 for chimeric gene HIV subtype H gp120 C1-C5-gp41
<400> 124
   ctgtgggtta ccgtttacta cggtgttcca gtgtggaaaa aagttgtt 48
<210> 125
   <211> 48
   <212> DNA
   <213> Artificial sequence
<220>
   <223> Forward primer F5 for chimeric gene HIV subtype H gp120 C1-C5-gp41
<400> 125
   cccgaattcc atatggtcga cgttgttggt aacctgtggg ttaccgtt 48
<210> 126
   <211> 47
   <212> DNA
   <213> Artificial sequence
<220>
   <223> Reverse primer R1 for chimeric gene HIV subtype H gp120 C1-C5-gp41
<400> 126
   tgctggtctt tcaggtaacg ttcaacagcc agaacacgag cctgacc 47
<210> 127
   <211> 46
   <212> DNA
   <213> Artificial sequence
<220>
   <223> Reverse primer R2 for chimeric gene HIV subtype H gp120 C1-C5-gp41
<400> 127
   tttaccggaa caaccccaga tacccagcag ctgctggtct ttcagg 46
<210> 128
   <211> 46
   <212> DNA
   <213> Artificial sequence
<220>
   <223> Reverse primer R3 for chimeric gene HIV subtype H gp120 C1-C5-gp41
<400> 128
   agttccatgg aacgttggtg gtacagatca gtttaccgga acaacc 46
<210> 129
   <211> 48
   <212> DNA
   <213> Artificial sequence
<220>
   <223> Reverse primer R4 for chimeric gene HIV subtype H gp120 C1-C5-gp41
<400> 129
   tttcgtccag ggatttgttg gaccaggagg agttccatgg aacgttgg 48
<210> 130
   <211> 55
   <212> DNA
   <213> Artificial sequence
<220>
   <223> Reverse primer R5 for chimeric gene HIV subtype H gp120 C1-C5-gp41
<400> 130
   tttctcgagc cattccatcc aggtcatgtt gtcccagatt tcgtccaggg atttg 55
<210> 131
   <211> 45
   <212> DNA
   <213> Artificial sequence
<220>
   <223> Forward primer F1 for chimeric gene HIV subtype J gp120 C1-C5-gp41
<400> 131
   gttgaacgtg aaaaacgtgg tggtggtggt ggtcaggctc gtgtt 45
<210> 132
   <211> 45
   <212> DNA
   <213> Artificial sequence
<220>
   <223> Forward primer F2 for chimeric gene HIV subtype J gp120 C1-C5-gp41
<400> 132
   gttgctccaa ccaaagctaa acgtcgtgtt gttgaacgtg aaaaa 45
<210> 133
   <211> 49
   <212> DNA
   <213> Artificial sequence
<220>
   <223> Forward primer F3 for chimeric gene HIV subtype J gp120 C1-C5-gp41
<400> 133
   gtggaaaaaa gttgttgaaa tcgaaccact gggtgttgct ccaaccaaa 49
<210> 134
   <211> 48
   <212> DNA
   <213> Artificial sequence
<220>
   <223> Forward primer F4 for chimeric gene HIV subtype J gp120 C1-C5-gp41
<400> 134
   ctgtgggtta ccgtttacta cggtgttcca gtgtggaaaa aagttgtt 48
<210> 135
   <211> 48
   <212> DNA
   <213> Artificial sequence
<220>
   <223> Forward primer F5 for chimeric gene HIV subtype J gp120 C1-C5-gp41
<400> 135
   cccgaattcc atatggtcga cgctaaagaa gacctgtggg ttaccgtt 48
<210> 136
   <211> 47
   <212> DNA
   <213> Artificial sequence
<220>
   <223> Reverse primer R1 for chimeric gene HIV subtype J gp120 C1-C5-gp41
<400> 136
   tgctggtctt tcaggtaacg ttcaacagcc agaacacgag cctgacc 47
<210> 137
   <211> 46
   <212> DNA
   <213> Artificial sequence
<220>
   <223> Reverse primer R2 for chimeric gene HIV subtype J gp120 C1-C5-gp41
<400> 137
   tttaccggaa caaccccaga tacccagcag ctgctggtct ttcagg 46
<210> 138
   <211> 48
   <212> DNA
   <213> Artificial sequence
<220>
   <223> Reverse primer R3 for chimeric gene HIV subtype J gp120 C1-C5-gp41
<400> 138
   agcgttccat ggaacgttgg tggtacagat cagtttaccg gaacaacc 48
<210> 139
   <211> 44
   <212> DNA
   <213> Artificial sequence
<220>
   <223> Reverse primer R4 for chimeric gene HIV subtype J gp120 C1-C5-gp41
<400> 139
   atgtcttcgt aggatttgtt ggaccaggaa gcgttccatg gaac 44
<210> 140
   <211> 46
   <212> DNA
   <213> Artificial sequence
<220>
   <223> Reverse primer R5 for chimeric gene HIV subtype J gp120 C1-C5-gp41
<400> 140
   tttctcgagc caggtcatgt tttcccagat gtcttcgtag gatttg 46
<210> 141
   <211> 45
   <212> DNA
   <213> Artificial sequence
<220>
   <223> Forward primer F1 for chimeric gene HIV subtype K gp120 C1-C5-gp41
<400> 141
   gttcagcgtg aaaaacgtgg tggtggtggt ggtcgtgctc gtgtt 45
<210> 142
   <211> 46
   <212> DNA
   <213> Artificial sequence
<220>
   <223> Forward primer F2 for chimeric gene HIV subtype K gp120 C1-C5-gp41
<400> 142
   atcgctccaa cccgtgctcg tcgtcgtgtt gttcagcgtg aaaaac 46
<210> 143
   <211> 49
   <212> DNA
   <213> Artificial sequence
<220>
   <223> Forward primer F3 for chimeric gene HIV subtype K gp120 C1-C5-gp41
<400> 143
   gtggaaaaaa gttgttcaga tcgaaccact gggtatcgct ccaacccgt 49
<210> 144
   <211> 48
   <212> DNA
   <213> Artificial sequence
<220>
   <223> Forward primer F4 for chimeric gene HIV subtype K gp120 C1-C5-gp41
<400> 144
   ctgtgggtta ccgtttacta cggtgttcca gtgtggaaaa aagttgtt 48
<210> 145
   <211> 48
   <212> DNA
   <213> Artificial sequence
<220>
   <223> Forward primer F5 for chimeric gene HIV subtype K gp120 C1-C5-gp41
<400> 145
   cccgaattcc atatggtcga cgctgctaac aacctgtggg ttaccgtt 48
<210> 146
   <211> 47
   <212> DNA
   <213> Artificial sequence
<220>
   <223> Reverse primer R1 for chimeric gene HIV subtype K gp120 C1-C5-gp41
<400> 146
   tgctggtcac gcaggtaacg ttcaacagcc agaacacgag cacgacc 47
<210> 147
   <211> 46
   <212> DNA
   <213> Artificial sequence
<220>
   <223> Reverse primer R2 for chimeric gene HIV subtype K gp120 C1-C5-gp41
<400> 147
   tttaccggaa caaccccaga tacccagcag ctgctggtca cgcagg 46
<210> 148
   <211> 46
   <212> DNA
   <213> Artificial sequence
<220>
   <223> Reverse primer R3 for chimeric gene HIV subtype K gp120 C1-C5-gp41
<400> 148
   agttccatgg aacgttggtg gtacagatca gtttaccgga acaacc 46
<210> 149
   <211> 48
   <212> DNA
   <213> Artificial sequence
<220>
   <223> Reverse primer R4 for chimeric gene HIV subtype K gp120 C1-C5-gp41
<400> 149
   tttcggactg ggatttgttg gaccaggagg agttccatgg aacgttgg 48
<210> 150
   <211> 46
   <212> DNA
   <213> Artificial sequence
<220>
   <223> Reverse primer R5 for chimeric gene HIV subtype K gp120 C1-C5-gp41
<400> 150
   tttctcgagc caggtcatgt tttcccagat ttcggactgg gatttg 46
<210> 151
   <211> 71
   <212> PRT
   <213> Human immunodeficiency virus
<223> Full length Gag24
<400> 151
   Val Asp Arg Asp Glu Leu Lys Gly Ile Gly Met Thr Asn Asn Pro Pro
   1               5                   10                  15
   Ile Pro Val Gly Glu Ile Tyr Lys Arg Trp Ile Ile Leu Gly Leu Asn
               20                  25                  30
   Lys Ile Val Arg Met Tyr Ser Pro Thr Met Thr Asn Asn Pro Pro Ile
           35                  40                  45
   Pro Val Gly Glu Ile Tyr Arg Trp Ile Ile Leu Gly Leu Asn Lys Ile
       50                  55                  60
   Val Arg Met Tyr Ser Pro Thr
   65                  70
<210> 152
   <211> 132
   <212> PRT
   <213> Human immunodeficiency virus
<223> Nef- N terminus
<400> 152
   Pro Thr Val Arg Gln Arg Met Asp Arg Thr Glu Pro Ala Ala Glu Gly
   1 5 10 15
   Val Gly Ala Val Ser Arg Asp Leu Glu Lys His Gly Ala Ile Thr Ser
   20 25 30
   Ser Asn Thr Ala Ala Thr Asn Ala Asp Cys Ala Trp Leu Glu Ala Gln
   35 40 45
   Glu Glu Glu Glu Val Gly Phe Pro Val Arg Pro Gln Val Pro Leu Arg
   50 55 60
   Pro Met Thr Tyr Lys Ala Ala Val Asp Ile Ser His Phe Leu Lys Glu
   65 70 75 80
   Lys Gly Gly Leu Glu Gly Leu Ile Tyr Ser Gln Lys Arg Gln Glu Ile
   85 90 95
   Leu Asp Leu Trp Ile Tyr His Thr Gln Gly Tyr Phe Pro Asp Trp Gln
   100 105 110
   Asn Tyr Thr Pro Gly Pro Gly Ile Arg Tyr Pro Leu Thr Phe Gly Trp
   115 120 125
   Cys Phe Lys Leu
   130
<210> 153
   <211> 66
   <212> PRT
   <213> Human immunodeficiency virus
<223> Nef- C terminus
<400> 153
   Phe Leu Lys Val Pro Val Asp Pro Glu Gln Val Glu Lys Ala Asn Glu
   1 5 10 15
   Gly Asp Asn Asn Cys Leu Leu His Pro Ile Ser Gln His Gly Met Asp
   20 25 30
   Asp Pro Glu Lys Glu Val Leu Met Trp Lys Phe Asp Ser Arg Leu Ala
   35 40 45
   Phe Gln His Ile Ala Arg Glu Lys His Pro Glu Tyr Tyr Lys Asp Cys
   50 55 60
   Leu Gly
   65
<210> 154
   <211> 109
   <212> PRT
   <213> Human immunodeficiency virus
<223> Full length Tat
<400> 154
   Arg Asp Glu Leu Lys Gly Ile Gly Met Glu Pro Val Asp Pro Arg Leu
   1 5 10 15
   Glu Pro Trp Lys His Pro Gly Ser Gln Pro Arg Thr Ala Cys Asn Asn
   20 25 30
   Cys Tyr Cys Lys Lys Cys Cys Phe His Cys Pro Val Cys Phe Ile Ser
   35 40 45
   Lys Gly Leu Gly Ile Ser Tyr Gly Arg Lys Lys Arg Arg Gln Arg Arg
   50 55 60
   Arg Ala Pro Gln Asp Ser Glu Thr His Gln Val Ser Leu Ser Lys Gln
   65 70 75 80
   Pro Thr Ser Gln Leu Arg Gly Asp Pro Thr Gly Pro Lys Glu Ser Lys
   85 90 95
   Lys Lys Val Glu Arg Glu Thr Glu Thr Asp Pro Asn Val
   100 105
<210> 155
   <211> 88
   <212> PRT
   <213> Human immunodeficiency virus
<223> Full length Rev
<400> 155
   Leu Leu Ala Val Arg Ile Ile Lys Thr Leu Tyr Gln Ser Asn Pro Tyr
   1 5 10 15
   Pro Lys Pro Glu Gly Tyr Arg Arg Val Arg Arg Asn Arg Arg Arg Arg
   20 25 30
   Trp Arg Ala Arg Gln Arg Gln Ile His Ser Ile Ser Glu Arg Ile Leu
   35 40 45
   Ile Thr Cys Leu Gly Arg Pro Thr Glu Pro Val Pro Leu Gln Leu Pro
   50 55 60
   Pro Ile Glu Arg Leu Asn Ile Asn Cys Ser Glu Ser Gly Gly Thr Ser
   65 70 75 80
   Gly Thr Gln Arg Val Gly Asn Pro
   85

<210> 156
   <211> 207
   <212> DNA
   <213> Human immunodeficiency virus
<223> Full length gag24
<400> 156
   cgtgacgaac tgaaaggtat cggtatgacc aacaacccgc cgatcccggt tggtgaaatc 60
   tacaaacgtt ggatcatcct gggtctgaac aaaatcgttc gtatgtacag cccgaccatg 120
   accaacaacc cgccaattcc agtaggcgag atttaccgtt ggatcatcct gggtctgaac 180
   aaaatcgttc gcatgtacag cccgact 207
<210> 157
   <211> 396
   <212> DNA
   <213> Human immunodeficiency virus
<223> nef- N terminus
<400> 157
   ccgaccgttc gtcagcgtat ggaccgtacc gaaccggctg ctgagggtgt tggtgctgtt 60
   agccgtgacc tggaaaaaca cggtgctatc acctcttcta acaccgctgc taccaacgct 120
   gactgcgctt ggctggaagc tcaggaagaa gaagaagttg gttttccggt tcgtccgcag 180
   gttccgctgc gtccgatgac ctacaaagct gctgttgaca tcagccactt cctgaaagaa 240
   aaaggcggcc tggaaggtct gatctacagc cagaaacgtc aggaaatcct ggacctgtgg 300
   atctaccaca cccagggcta cttcccggac tggcagaact acaccccggg tccgggtatc 360
   cgttacccgc tgaccttcgg ctggtgtttc aaactg 396
<210> 158
   <211> 198
   <212> DNA
   <213> Human immunodeficiency virus
<223> nef- C terminus
<400> 158
   ttcctgaaag ttccggttga cccggaacag gttgaaaaag ctaacgaagg tgataacaac 60
   tgcctgctgc acccgatcag ccagcacggt atggacgacc cggaaaaaga agttctgatg 120
   tggaaattcg acagccgtct ggctttccag cacatcgctc gtgaaaaaca cccggaatac 180
   tacaaagact gcctgggt 198
<210> 159
   <211> 327
   <212> DNA
   <213> Human immunodeficiency virus
<223> Full length tat
<400> 159
   cgtgacgaac tgaaaggtat cggtatggaa ccggttgacc cgcgtctgga accgtggaaa 60
   cacccgggta gccagccgcg taccgcttgc aacaactgct actgcaaaaa atgttgtttc 120
   cactgcccgg tttgctttat ctctaaaggt ctgggtatca gctacggtcg taaaaagcgt 180
   cgccagcgcc gccgcgctcc gcaggactcc gaaacccacc aggttagcct gagcaagcaa 240
   ccgaccagcc agctgcgtgg tgacccgacc ggtccgaaag aaagcaaaaa aaaagttgaa 300
   cgtgaaaccg aaaccgaccc gaacgtt 327
<210> 160
   <211> 264
   <212> DNA
   <213> Human immunodeficiency virus
<223> Full length rev
<400> 160
   ctgctggctg ttcgtatcat caaaaccctg taccagtcta acccgtaccc gaaaccggaa 60
   ggttaccgtc gtgtccgtcg taaccgccgc cgccgctggc gtgctcgtca gcgtcagatc 120
   cactccatca gcgaacgtat cctgatcacc tgcctgggtc gtccgaccga accggttccg 180
   ctgcaactgc cgccgatcga acgtctgaac atcaactgta gcgaaagcgg tggtaccagc 240
   ggtacccagc gtgttggtaa cccg 264
<210> 161
   <211> 39
   <212> DNA
   <213> Artificial sequence
<220>
   <223> Forward Primer F1 for gag24
<400> 161
   tggatcatcc tgggtctgaa caaaatcgtt cgtatgtac 39
<210> 162
   <211> 39
   <212> DNA
   <213> Artificial sequence
<220>
   <223> Forward Primer F2 for gag24
<400> 162
   ccggttggtg aaatctacaa acgttggatc atcctgggt 39
<210> 163
   <211> 41
   <212> DNA
   <213> Artificial sequence
<220>
   <223> Forward Primer F3 for gag24
<400> 163
   tcggtatgac caacaacccg ccgatcccgg ttggtgaaat c 41
<210> 164
   <211> 56
   <212> DNA
   <213> Artificial sequence
<220>
   <223> Forward Primer F4 for gag24
<400> 164
   cccgaattcc atatggtcga ccgtgacgaa ctgaaaggta tcggtatgac caacaa 56
<210> 165
   <211> 39
   <212> DNA
   <213> Artificial sequence
<220>
   <223> Reverse Primer R1 for gag24
<400> 165
   cgggttgttg gtcatggtcg ggctgtacat acgaacgat 39
<210> 166
   <211> 39
   <212> DNA
   <213> Artificial sequence
<220>
   <223> Reverse Primer R2 for gag24
<400> 166
   gtaaatctcg cctactggaa ttggcgggtt gttgttcat 39
<210> 167
   <211> 40
   <212> DNA
   <213> Artificial sequence
<220>
   <223> Reverse Primer R3 for gag24
<400> 167
   tgttcagacc caggatgatc caacggtaaa tctcgcctac 40
<210> 168
   <211> 69
   <212> DNA
   <213> Artificial sequence
<220>
   <223> Reverse Primer R4 for gag24
<400> 168
   aaaaaaattc ccattatttt tctcgagagt cgggctgtac atgcgaacga ttttgttcag 60
   acccaggat 69
<210> 169
   <211> 39
   <212> DNA
   <213> Artificial sequence
<220>
   <223> Forward Primer F1 for nef-N terminus
<400> 169
   gcttggctgg aagctcagga agaagaagaa gttggtttt 39
<210> 170
   <211> 40
   <212> DNA
   <213> Artificial sequence
<220>
   <223> Forward Primer F2 for nef-N terminus
<400> 170
   caccgctgct accaacgctg actgcgcttg gctggaagct 40
<210> 171
   <211> 42
   <212> DNA
   <213> Artificial sequence
<220>
   <223> Forward Primer F3 for nef-N terminus
<400> 171
   aaacacggtg ctatcacctc ttctaacacc gctgctacca ac 42
<210> 172
   <211> 40
   <212> DNA
   <213> Artificial sequence
<220>
   <223> Forward Primer F4 for nef-N terminus
<400> 172
   tggtgctgtt agccgtgacc tggaaaaaca cggtgctatc 40
<210> 173
   <211> 42
   <212> DNA
   <213> Artificial sequence
<220>
   <223> Forward Primer F5 for nef-N terminus
<400> 173
   cgtaccgaac cggctgctga gggtgttggt gctgttagcc gt 42
<210> 174
   <211> 40
   <212> DNA
   <213> Artificial sequence
<220>
   <223> Forward Primer F6 for nef-N terminus
<400> 174
   gaccgttcgt cagcgtatgg accgtaccga accggctgct 40
<210> 175
   <211> 44
   <212> DNA
   <213> Artificial sequence
<220>
   <223> Forward Primer F7 for nef-N terminus
<400> 175
   cccgaattcc atatggtcga cccgaccgtt cgtcagcgta tgga 44
<210> 176
   <211> 42
   <212> DNA
   <213> Artificial sequence
<220>
   <223> Reverser Primer R1 for nef-N terminus
<400> 176
   acgcagcgga acctgcggac gaaccggaaa accaacttct tc 42
<210> 177
   <211> 39
   <212> DNA
   <213> Artificial sequence
<220>
   <223> Reverser Primer R2 for nef-N terminus
<400> 177
   aacagcagct ttgtaggtca tcggacgcag cggaacccg 39
<210> 178
   <211> 43
   <212> DNA
   <213> Artificial sequence
<220>
   <223> Reverser Primer R3 for nef-N terminus
<400> 178
   ctttttcttt caggaagtgg ctgatgtcaa cagcagcttt gta 43
<210> 179
   <211> 39
   <212> DNA
   <213> Artificial sequence
<220>
   <223> Reverser Primer R4 for nef-N terminus
<400> 179
   gtagatcaga ccttccaggc cgcctttttc tttcaggaa 39
<210> 180
   <211> 41
   <212> DNA
   <213> Artificial sequence
<220>
   <223> Reverser Primer R5 for nef-N terminus
<400> 180
   tccaggattt cctgacgttt ctggctgtag atcagacctt c 41
<210> 181
   <211> 40
   <212> DNA
   <213> Artificial sequence
<220>
   <223> Reverser Primer R6 for nef-N terminus
<400> 181
   agccctgggt gtggtagatc cacaggtcca ggatttcctg 40
<210> 182
   <211> 39
   <212> DNA
   <213> Artificial sequence
<220>
   <223> Reverser Primer R7 for nef-N terminus
<400> 182
   gtagttctgc cagtccggga agtagccctg ggtgtggta 39
<210> 183
   <211> 44
   <212> DNA
   <213> Artificial sequence
<220>
   <223> Reverser Primer R8 for nef-N terminus
<400> 183
   agcgggtaac ggatacccgg acccggggtg tagttctgcc agtc 44
<210> 184
   <211> 66
   <212> DNA
   <213> Artificial sequence
<220>
   <223> Reverser Primer 9 for nef-N terminus
<400> 184
   aaaaaaattc ccattatttt tctcgagcag tttgaaacac cagccgaagg tcagcgggta 60
   acggat 66
<210> 185
   <211> 39
   <212> DNA
   <213> Artificial sequence
<220>
   <223> Forward Primer F1 for nef-C terminus
<400> 185
   ctgcacccga tcagccagca cggtatggac gacccggaa 39
<210> 186
   <211> 39
   <212> DNA
   <213> Artificial sequence
<220>
   <223> Forward Primer F2 for nef-C terminus
<400> 186
   aacgaaggtg ataacaactg cctgctgcac ccgatcagc 39
<210> 187
   <211> 40
   <212> DNA
   <213> Artificial sequence
<220>
   <223> Forward Primer F3 for nef-C terminus
<400> 187
   tgacccggaa caggttgaaa aagctaacga aggtgataac 40
<210> 188
   <211> 54
   <212> DNA
   <213> Artificial sequence
<220>
   <223> Forward Primer F4 for nef-C terminus
<400> 188
   cccgaattcc atatggtcga cttcctgaaa gttccggttg acccggaaca ggtt 54
<210> 189
   <211> 39
   <212> DNA
   <213> Artificial sequence
<220>
   <223> Reverse Primer R1 for nef-C terminus
<400> 189
   gaatttccac atcagaactt ctttttccgg gtcgtccat 39
<210> 190
   <211> 40
   <212> DNA
   <213> Artificial sequence
<220>
   <223> Reverse Primer R2 for nef-C terminus
<400> 190
   tgtgctggaa agccagacgg ctgtcgaatt tccacatcag 40
<210> 191
   <211> 39
   <212> DNA
   <213> Artificial sequence
<220>
   <223> Reverse Primer R3 for nef-C terminus
<400> 191
   ttccgggtgt ttttcacgag cgatgtgctg gaaagccag 39
<210> 192
   <211> 63
   <212> DNA
   <213> Artificial sequence
<220>
   <223> Reverse Primer R4 for nef-C terminus
<400> 192
   aaaaaaattc ccattatttt tctcgagacc caggcagtct ttgtagtatt ccgggtgttt 60
   ttc 63
<210> 193
   <211> 40
   <212> DNA
   <213> Artificial sequence
<220>
   <223> Forward Primer F1 for tat
<400> 193
   ctttatctct aaaggtctgg gtatcagcta cggtcgtaaa 40
<210> 194
   <211> 46
   <212> DNA
   <213> Artificial sequence
<220>
   <223> Forward Primer 2 for tat
<400> 194
   caaaaaatgt tgtttccact gcccggtttg ctttatctct aaaggt 46
<210> 195
   <211> 39
   <212> DNA
   <213> Artificial sequence
<220>
   <223> Forward Primer F3 for tat
<400> 195
   accgcttgca acaactgcta ctgcaaaaaa tgttgtttc 39
<210> 196
   <211> 39
   <212> DNA
   <213> Artificial sequence
<220>
   <223> Forward Primer F4 for tat
<400> 196
   aaacacccgg gtagccagcc gcgtaccgct tgcaacaac 39
<210> 197
   <211> 43
   <212> DNA
   <213> Artificial sequence
<220>
   <223> Forward Primer F5 for tat
<400> 197
   accggttgac ccgcgtctgg aaccgtggaa acacccgggt agc 43
<210> 198
   <211> 39
   <212> DNA
   <213> Artificial sequence
<220>
   <223> Forward Primer F6 for tat
<400> 198
   gaactgaaag gtatcggtat ggaaccggtt gacccgcgt 39
<210> 199
   <211> 45
   <212> DNA
   <213> Artificial sequence
<220>
   <223> Forward Primer F7 for tat
<400> 199
   cccgaattcc atatggtcga ccgtgacgaa ctgaaaggta tcggt 45
<210> 200
   <211> 39
   <212> DNA
   <213> Artificial sequence
<220>
   <223> Reverse Primer R1 for tat
<400> 200
   gagcgcggcg gcgctggcga cgctttttac gccgtagct 39
<210> 201
   <211> 42
   <212> DNA
   <213> Artificial sequence
<220>
   <223> Reverse Primer R2 for tat
<400> 201
   aacctggtgg gtttcggagt cctgcggagc gcggcggcgc tg 42
<210> 202
   <211> 41
   <212> DNA
   <213> Artificial sequence
<220>
   <223> Reverse Primer R3 for tat
<400> 202
   tggctggtcg gttgcttgct caggctaacc tggtgggttt c 41
<210> 203
   <211> 40
   <212> DNA
   <213> Artificial sequence
<220>
   <223> Reverse Primer R4 for tat
<400> 203
   gaccggtcgg gtcaccacgc agctggctgg tcggttgctt 40
<210> 204
   <211> 39
   <212> DNA
   <213> Artificial sequence
<220>
   <223> Reverse Primer 5 for tat
<400> 204
   aacttttttt ttgctttctt tcggaccggt cgggtcacc 39
<210> 205
   <211> 39
   <212> DNA
   <213> Artificial sequence
<220>
   <223> Reverse Primer R6 for tat
<400> 205
   cgggtcggtt tcggtttcac gttcaacttt ttttttgct 39
<210> 206
   <211> 51
   <212> DNA
   <213> Artificial sequence
<220>
   <223> Reverse Primer R7 for tat
<400> 206
   aaaaaaattc tcattatttt tctcgagaac gttcgggtcg gtttcggttt c 51
<210> 207
   <211> 40
   <212> DNA
   <213> Artificial sequence
<220>
   <223> Forward Primer F1 for rev
<400> 207
   tcagatccac tccatcagcg aacgtatcct gatcacctgc 40
<210> 208
   <211> 48
   <212> DNA
   <213> Artificial sequence
<220>
   <223> Forward Primer F2 for rev
<400> 208
   aaccgccgcc gccgctggcg tgctcgtcag cgtcagatcc actccatc 48
<210> 209
   <211> 45
   <212> DNA
   <213> Artificial sequence
<220>
   <223> Forward Primer F3 for rev
<400> 209
   aaaccggaag gttaccgtcg tgtccgtcgt aaccgccgcc gccgc 45
<210> 210
   <211> 42
   <212> DNA
   <213> Artificial sequence
<220>
   <223> Forward Primer F4 for rev
<400> 210
   accctgtacc agtctaaccc gtacccgaaa ccggaaggtt ac 42
<210> 211
   <211> 39
   <212> DNA
   <213> Artificial sequence
<220>
   <223> Forward Primer F5 for rev
<400> 211
   ctgctggctg ttcgtatcat caaaaccctg taccagtct 39
<210> 212
   <211> 48
   <212> DNA
   <213> Artificial sequence
<220>
   <223> Forward Primer F6 for rev
<400> 212
   ccccccgaat tccatatggt cgacctgctg gctgttcgta tcatcaaa 48
<210> 213
   <211> 40
   <212> DNA
   <213> Artificial sequence
<220>
   <223> Reverse Primer R1 for rev
<400> 213
   gaaccggttc ggtcggacga cccaggcagg tgatcaggat 40
<210> 214
   <211> 39
   <212> DNA
   <213> Artificial sequence
<220>
   <223> Reverse Primer R2 for rev
<400> 214
   ttcgatcggc ggcagttgca gcggaaccgg ttcggtcgg 39
<210> 215
   <211> 39
   <212> DNA
   <213> Artificial sequence
<220>
   <223> Reverse Primer R3 for rev
<400> 215
   ttcgctacag ttgatgttca gacgttcgat cggcggcag 39
<210> 216
   <211> 39
   <212> DNA
   <213> Artificial sequence
<220>
   <223> Reverse Primer R4 for rev
<400> 216
   ctgggtaccg ctggtaccac cgctttcgct acagttgat 39
<210> 217
   <211> 59
   <212> DNA
   <213> Artificial sequence
<220>
   <223> Reverse Primer R5 for rev
<400> 217
   aaaaaaattc ccattatttt tctcgagcgg gttaccaaca cgctgggtac cgctggtac 59
<210> 218
   <211> 16
   <212> PRT
   <213> Artificial sequence
<220>
   <223> Fusion protein gp120-41-N1
<400> 218
<210> 219
   <211> 16
   <212> PRT
   <213> Artificial sequence
<220>
   <223> Fusion protein gp120-41-N2
<400> 219
   Lys Val Val Lys Ile Glu Pro Leu Gly Val Ala Pro Thr Lys Cys Lys
   1               5                   10                  15
<210> 220
   <211> 15
   <212> PRT
   <213> Artificial sequence
<220>
   <223> Fusion protein gp120-41-N3
<400> 220
   Ala Pro Thr Lys Cys Lys Arg Arg Val Val Gln Arg Glu Lys Arg
   1               5                   10                  15
<210> 221
   <211> 14
   <212> PRT
   <213> Artificial sequence
<220>
   <223> Fusion protein gp120-41-C1
<400> 221
   Gln Ala Arg Val Trp Arg Tyr Leu Lys Asp Gln Gln Leu Leu
   1               5                   10
<210> 222
   <211> 17
   <212> PRT
   <213> Artificial sequence
<220>
   <223> Fusion protein gp120-41-C2
<400> 222
   Gly Ile Trp Gly Cys Ser Gly Lys Leu Ile Cys Cys Thr Thr Ala Val
   1               5                   10                  15
   Pro
<210> 223
   <211> 15
   <212> PRT
   <213> Artificial sequence
<220>
   <223> Fusion protein gp120-41-C3
<400> 223
   Ala Val Pro Trp Asn Ala Ser Ser Trp Ser Asn Lys Leu Asp Arg
   1               5                   10                  15
<210> 224
   <211> 20
   <212> PRT
   <213> Human immunodeficiency virus
<223> HIV-Tat-N
<400> 224
   Pro Val Asp Pro Arg Leu Glu Pro Trp Lys His Pro Gly Ser Gln Pro
   1               5                   10                  15
   Arg Thr Ala Cys
               20
<210> 225
   <211> 20
   <212> PRT
   <213> Human immunodeficiency virus
<223> HIV-Tat-C
<400> 225
   Gln Leu Arg Gly Asp Pro Thr Gly Pro Lys Glu Ser Lys Lys Lys Val
   1               5                   10                  15
   Glu Arg Glu Thr
               20
<210> 226
   <211> 20
   <212> PRT
   <213> Human immunodeficiency virus
<223> HIV-Tat-M
<400> 226
   Ser Tyr Gly Arg Lys Lys Arg Arg Gln Arg Arg Arg Ala Pro Gln Asp
   1               5                   10                  15
   Ser Glu Thr His
               20
<210> 227
   <211> 20
   <212> PRT
   <213> Human immunodeficiency virus
<223> HIV-Rev-N
<400> 227
   Gln Ser Asn Pro Tyr Pro Lys Pro Glu Gly Tyr Arg Arg Val Arg Arg
   1               5                   10                  15
   Asn Arg Arg Arg
               20
<210> 228
   <211> 20
   <212> PRT
   <213> Human immunodeficiency virus
<223> HIV-Rev-C
<400> 228
   Asn Cys Ser Glu Ser Gly Gly Thr Ser Gly Thr Gln Arg Val Gly Asn
   1               5                   10                  15
   Pro Leu Glu Lys
               20
<210> 229
   <211> 18
   <212> PRT
   <213> Human immunodeficiency virus
<223> HIV-Nef-nl-N
<400> 229
   Ser Lys Leu Lys Lys Gly Trp Pro Thr Val Arg Gln Arg Met Asp Arg
   1               5                   10                  15
   Thr Glu
<210> 230
   <211> 18
   <212> PRT
   <213> Human immunodeficiency virus
<223> HIV-Nef-nl-C
<400> 230
   Thr Gln Gly Tyr Phe Pro Asp Trp Gln Asn Tyr Thr Pro Gly Pro Gly
   1               5                   10                  15
   Ile Arg
<210> 231
   <211> 15
   <212> PRT
   <213> Human immunodeficiency virus
<223> HIV-Nef-cl-N
<400> 231
   Val Asp Pro Glu Gln Val Glu Lys Ala Asn Glu Gly Asp Asn Asn
   1               5                   10                  15
<210> 232
   <211> 15
   <212> PRT
   <213> Human immunodeficiency virus
<223> HIV-Nef-cl-M
<400> 232
   Ile Ser Gln His Gly Met Asp Asp Pro Glu Lys Glu Val Leu Met
   1               5                   10                  15
<210> 233
   <211> 20
   <212> PRT
   <213> Human immunodeficiency virus
<223> HIV-Nef-c1-C
<400> 233
   Gln His Ile Ala Arg Glu Lys His Pro Glu Tyr Tyr Lys Asp Cys Leu
   1               5                   10                  15
   Gly Leu Glu Lys
               20
<210> 234
   <211> 18
   <212> PRT
   <213> Human immunodeficiency virus
<223> HIV-Gag24-N
<400> 234
   Pro Glu Phe His Met Val Asp Arg Asp Glu Leu Lys Gly Ile Gly Met
   1               5                   10                  15
   Thr Asn
<210> 235
   <211> 15
   <212> PRT
   <213> Human immunodeficiency virus
<223> HIV Gag24-C
<400> 235
   Arg Met Tyr Ser Pro Thr Met Thr Asn Asn Pro Pro Ile Pro Val
   1               5                   10                  15
<210> 236
   <211> 1563
   <212> DNA
   <213> Artificial sequence
<220>
   <223> Fusion gene PE(delta III)-HIV-Gag24-K3
<400> 236
   atgggcagca gccatcatca tcatcatcac agcagcggcc tggtgccgcg cggcagccat     60
   atggccgaag aagctttcga cctctggaac gaatgcgcca aagcctgcgt gctcgacctc    120
   aaggacggcg tgcgttccag ccgcatgagc gtcgacccgg ccatcgccga caccaacggc    180
   cagggcgtgc tgcactactc catggtcctg gagggcggca acgacgcgct caagctggcc    240
   atcgacaacg ccctcagcat caccagcgac ggcctgacca tccgcctcga aggcggcgtc    300
   gagccgaaca agccggtgcg ctacagctac acgcgccagg cgcgcggcag ttggtcgctg    360
   aactggctgg taccgatcgg ccacgagaag ccctcgaaca tcaaggtgtt catccacgaa    420
   ctgaacgccg gcaaccagct cagccacatg tcgccgatct acaccatcga gatgggcgac    480
   gagttgctgg cgaagctggc gcgcgatgcc accttcttcg tcagggcgca cgagagcaac    540
   gagatgcagc cgacgctcgc catcagccat gccggggtca gcgtggtcat ggcccagacc    600
   cagccgcgcc gggaaaagcg ctggagcgaa tgggccagcg gcaaggtgtt gtgcctgctc    660
   gacccgctgg acggggtcta caactacctc gcccagcaac gctgcaacct cgacgatacc    720
   tgggaaggca agatctaccg ggtgctcgcc ggcaacccgg cgaagcatga cctggacatc    780
   aaacccacgg tcatcagtca tcgcctgcac tttcccgagg gcggcagcct ggccgcgctg    840
   accgcgcacc aggcttgcca cctgccgctg gagactttca cccgtcatcg ccagccgcgc    900
   ggctgggaac aactggagca gtgcggctat ccggtgcagc ggctggtcgc cctctacctg    960
   gcggcgcggc tgtcgtggaa ccaggtcgac caggtgatcc gcaacgccct ggccagcccc   1020
   ggcagcggcg gcgacctggg cgaagcgatc cgcgagcagc cggagcaggc ccgtctggcc   1080
   ctgaccctgg ccgccgccga gagcgagcgc ttcgtccggc agggcaccgg caacgacgag   1140
   gccggcgcgg ccaacgccga cgtggtgagc ctgacctgcc cggtcgccgc cggtgaatgc   1200
   gcgggcccgg cggacagcgg cgacgccctg ctggagcgca actatcccac tggcgcggag   1260
   ttcctcggcg acggcggcga cgtcgaattc catatggtcg accgtgacga actgaaaggt   1320
   atcggtatga ccaacaaccc gccgatcccg gttggtgaaa tctacaaacg ttggatcatc   1380
   ctgggtctga acaaaatcgt tcgtatgtac agcccgacca tgaccaacaa cccgccaatt   1440
   ccagtaggcg agatttaccg ttggatcatc ctgggtctga acaaaatcgt tcgcatgtac   1500
   agcccgactc tcgagtacct caaaaaagac gaactgcgtg tagaactgaa agacgaactg   1560
   taa                                                                 1563
<210> 237
   <211> 520
   <212> PRT
   <213> Artificial sequence
<220>
   <223> Fusion protein PE(delta III)-HIV Gag24-K3
<400> 237
   Met Gly Ser Ser His His His His His His Ser Ser Gly Leu Val Pro
   1               5                   10                  15
   Arg Gly Ser His Met Ala Glu Glu Ala Phe Asp Leu Trp Asn Glu Cys
               20                  25                  30
   Ala Lys Ala Cys Val Leu Asp Leu Lys Asp Gly Val Arg Ser Ser Arg
           35                  40                  45
   Met Ser Val Asp Pro Ala Ile Ala Asp Thr Asn Gly Gln Gly Val Leu
       50                  55                  60
   His Tyr Ser Met Val Leu Glu Gly Gly Asn Asp Ala Leu Lys Leu Ala
   65                  70                  75                  80
   Ile Asp Asn Ala Leu Ser Ile Thr Ser Asp Gly Leu Thr Ile Arg Leu
                   85                  90                  95
   Glu Gly Gly Val Glu Pro Asn Lys Pro Val Arg Tyr Ser Tyr Thr Arg
               100                 105                 110
   Gln Ala Arg Gly Ser Trp Ser Leu Asn Trp Leu Val Pro Ile Gly His
           115                 120                 125
   Glu Lys Pro Ser Asn Ile Lys Val Phe Ile His Glu Leu Asn Ala Gly
       130                 135                 140
   Asn Gln Leu Ser His Met Ser Pro Ile Tyr Thr Ile Glu Met Gly Asp
   145                 150                 155                 160
   Glu Leu Leu Ala Lys Leu Ala Arg Asp Ala Thr Phe Phe Val Arg Ala
                   165                 170                 175
   His Glu Ser Asn Glu Met Gln Pro Thr Leu Ala Ile Ser His Ala Gly
               180                 185                 190
   Val Ser Val Val Met Ala Gln Thr Gln Pro Arg Arg Glu Lys Arg Trp
           195                 200                 205
   Ser Glu Trp Ala Ser Gly Lys Val Leu Cys Leu Leu Asp Pro Leu Asp
       210                 215                 220
   Gly Val Tyr Asn Tyr Leu Ala Gln Gln Arg Cys Asn Leu Asp Asp Thr
   225                 230                 235                 240
   Trp Glu Gly Lys Ile Tyr Arg Val Leu Ala Gly Asn Pro Ala Lys His
                   245                 250                 255
   Asp Leu Asp Ile Lys Pro Thr Val Ile Ser His Arg Leu His Phe Pro
               260                 265                 270
   Glu Gly Gly Ser Leu Ala Ala Leu Thr Ala His Gln Ala Cys His Leu
           275                 280                 285
   Pro Leu Glu Thr Phe Thr Arg His Arg Gln Pro Arg Gly Trp Glu Gln
       290                 295                 300
   Leu Glu Gln Cys Gly Tyr Pro Val Gln Arg Leu Val Ala Leu Tyr Leu
   305                 310                 315                 320
   Ala Ala Arg Leu Ser Trp Asn Gln Val Asp Gln Val Ile Arg Asn Ala
                   325                 330                 335
   Leu Ala Ser Pro Gly Ser Gly Gly Asp Leu Gly Glu Ala Ile Arg Glu
               340                 345                 350
   Gln Pro Glu Gln Ala Arg Leu Ala Leu Thr Leu Ala Ala Ala Glu Ser
           355                 360                 365
   Glu Arg Phe Val Arg Gln Gly Thr Gly Asn Asp Glu Ala Gly Ala Ala
       370                 375                 380
   Asn Ala Asp Val Val Ser Leu Thr Cys Pro Val Ala Ala Gly Glu Cys
   385                 390                 395                 400
   Ala Gly Pro Ala Asp Ser Gly Asp Ala Leu Leu Glu Arg Asn Tyr Pro
                   405                 410                 415
   Thr Gly Ala Glu Phe Leu Gly Asp Gly Gly Asp Val Glu Phe His Met
               420                 425                 430
   Val Asp Arg Asp Glu Leu Lys Gly Ile Gly Met Thr Asn Asn Pro Pro
           435                 440                 445
   Ile Pro Val Gly Glu Ile Tyr Lys Arg Trp Ile Ile Leu Gly Leu Asn
       450                 455                 460
   Lys Ile Val Arg Met Tyr Ser Pro Thr Met Thr Asn Asn Pro Pro Ile
   465                 470                 475                 480
   Pro Val Gly Glu Ile Tyr Arg Trp Ile Ile Leu Gly Leu Asn Lys Ile
                   485                 490                 495
   Val Arg Met Tyr Ser Pro Thr Leu Glu Tyr Leu Lys Lys Asp Glu Leu
               500                 505                 510
   Arg Val Glu Leu Lys Asp Glu Leu
           515                 520

<210> 238
   <211> 1863
   <212> DNA
   <213> Artificial sequence
<220>
   <223> Fusion gene PE(delta III)-HIV Gag24-gp120-41-K3
<400> 238
   atgggcagca gccatcatca tcatcatcac agcagcggcc tggtgccgcg cggcagccat    60
   atggccgaag aagctttcga cctctggaac gaatgcgcca aagcctgcgt gctcgacctc   120
   aaggacggcg tgcgttccag ccgcatgagc gtcgacccgg ccatcgccga caccaacggc   180
   cagggcgtgc tgcactactc catggtcctg gagggcggca acgacgcgct caagctggcc   240
   atcgacaacg ccctcagcat caccagcgac ggcctgacca tccgcctcga aggcggcgtc   300
   gagccgaaca agccggtgcg ctacagctac acgcgccagg cgcgcggcag ttggtcgctg   360
   aactggctgg taccgatcgg ccacgagaag ccctcgaaca tcaaggtgtt catccacgaa   420
   ctgaacgccg gcaaccagct cagccacatg tcgccgatct acaccatcga gatgggcgac   480
   gagttgctgg cgaagctggc gcgcgatgcc accttcttcg tcagggcgca cgagagcaac   540
   gagatgcagc cgacgctcgc catcagccat gccggggtca gcgtggtcat ggcccagacc   600
   cagccgcgcc gggaaaagcg ctggagcgaa tgggccagcg gcaaggtgtt gtgcctgctc   660
   gacccgctgg acggggtcta caactacctc gcccagcaac gctgcaacct cgacgatacc   720
   tgggaaggca agatctaccg ggtgctcgcc ggcaacccgg cgaagcatga cctggacatc   780
   aaacccacgg tcatcagtca tcgcctgcac tttcccgagg gcggcagcct ggccgcgctg   840
   accgcgcacc aggcttgcca cctgccgctg gagactttca cccgtcatcg ccagccgcgc    900
   ggctgggaac aactggagca gtgcggctat ccggtgcagc ggctggtcgc cctctacctg    960
   gcggcgcggc tgtcgtggaa ccaggtcgac caggtgatcc gcaacgccct ggccagcccc   1020
   ggcagcggcg gcgacctggg cgaagcgatc cgcgagcagc cggagcaggc ccgtctggcc   1080
   ctgaccctgg ccgccgccga gagcgagcgc ttcgtccggc agggcaccgg caacgacgag   1140
   gccggcgcgg ccaacgccga cgtggtgagc ctgacctgcc cggtcgccgc cggtgaatgc   1200
   gcgggcccgg cggacagcgg cgacgccctg ctggagcgca actatcccac tggcgcggag   1260
   ttcctcggcg acggcggcga cgtcgaattc catatggtcg accgtgacga actgaaaggt   1320
   atcggtatga ccaacaaccc gccgatcccg gttggtgaaa tctacaaacg ttggatcatc   1380
   ctgggtctga acaaaatcgt tcgtatgtac agcccgacca tgaccaacaa cccgccaatt   1440
   ccagtaggcg agatttaccg ttggatcatc ctgggtctga acaaaatcgt tcgcatgtac   1500
   agcccgactc tcgacgttga aaaactgtgg gttaccgttt actacggtgt tccggtttgg   1560
   aaaaaagttg ttaaaatcga accgctgggt gttgctccga ccaaatgcaa acgtcgtgtt   1620
   gttcagcgtg aaaagcgtgg tggcggtggc ggtcaagctc gtgttatcgc tgttgaacgt   1680
   tacctgaaag accagcagct gctgggtatc tggggtggta gcggtaaact gatctgctgc   1740
   accaccgctg ttccgtggaa cagcagctgg agcaacaaac tggaccgtat ctggaacaac   1800
   atgacctggc tcgagtacct caaaaaagac gaactgcgtg tagaactgaa agacgaactg   1860
   taa                                                                 1863
<210> 239
   <211> 620
   <212> PRT
   <213> Artificial sequence
<220>
   <223> Fusion protein PE(delta III)-HIV Gag24-gp120-41-K3
<400> 239
   Met Gly Ser Ser His His His His His His Ser Ser Gly Leu Val Pro
   1               5                   10                  15
   Arg Gly Ser His Met Ala Glu Glu Ala Phe Asp Leu Trp Asn Glu Cys
               20                  25                  30
   Ala Lys Ala Cys Val Leu Asp Leu Lys Asp Gly Val Arg Ser Ser Arg
           35                  40                  45
   Met Ser Val Asp Pro Ala Ile Ala Asp Thr Asn Gly Gln Gly Val Leu
       50                  55                  60
   His Tyr Ser Met Val Leu Glu Gly Gly Asn Asp Ala Leu Lys Leu Ala
   65                  70                  75                  80
   Ile Asp Asn Ala Leu Ser Ile Thr Ser Asp Gly Leu Thr Ile Arg Leu
                   85                  90                  95
   Glu Gly Gly Val Glu Pro Asn Lys Pro Val Arg Tyr Ser Tyr Thr Arg
               100                 105                 110
   Gln Ala Arg Gly Ser Trp Ser Leu Asn Trp Leu Val Pro Ile Gly His
           115                 120                 125
   Glu Lys Pro Ser Asn Ile Lys Val Phe Ile His Glu Leu Asn Ala Gly
       130                 135                 140
   Asn Gln Leu Ser His Met Ser Pro Ile Tyr Thr Ile Glu Met Gly Asp
   145                 150                 155                 160
   Glu Leu Leu Ala Lys Leu Ala Arg Asp Ala Thr Phe Phe Val Arg Ala
                   165                 170                 175
   His Glu Ser Asn Glu Met Gln Pro Thr Leu Ala Ile Ser His Ala Gly
               180                 185                 190
   Val Ser Val Val Met Ala Gln Thr Gln Pro Arg Arg Glu Lys Arg Trp
           195                 200                 205
   Ser Glu Trp Ala Ser Gly Lys Val Leu Cys Leu Leu Asp Pro Leu Asp
       210                 215                 220
   Gly Val Tyr Asn Tyr Leu Ala Gln Gln Arg Cys Asn Leu Asp Asp Thr
   225                 230                 235                 240
   Trp Glu Gly Lys Ile Tyr Arg Val Leu Ala Gly Asn Pro Ala Lys His
                   245                 250                 255
   Asp Leu Asp Ile Lys Pro Thr Val Ile Ser His Arg Leu His Phe Pro
               260                 265                 270
   Glu Gly Gly Ser Leu Ala Ala Leu Thr Ala His Gln Ala Cys His Leu
           275                 280                 285
   Pro Leu Glu Thr Phe Thr Arg His Arg Gln Pro Arg Gly Trp Glu Gln
       290                 295                 300
   Leu Glu Gln Cys Gly Tyr Pro Val Gln Arg Leu Val Ala Leu Tyr Leu
   305                 310                 315                 320
   Ala Ala Arg Leu Ser Trp Asn Gln Val Asp Gln Val Ile Arg Asn Ala
                   325                 330                 335
   Leu Ala Ser Pro Gly Ser Gly Gly Asp Leu Gly Glu Ala Ile Arg Glu
               340                 345                 350
   Gln Pro Glu Gln Ala Arg Leu Ala Leu Thr Leu Ala Ala Ala Glu Ser
           355                 360                 365
   Glu Arg Phe Val Arg Gln Gly Thr Gly Asn Asp Glu Ala Gly Ala Ala
       370                 375                 380
   Asn Ala Asp Val Val Ser Leu Thr Cys Pro Val Ala Ala Gly Glu Cys
   385                 390                 395                 400
   Ala Gly Pro Ala Asp Ser Gly Asp Ala Leu Leu Glu Arg Asn Tyr Pro
                   405                 410                 415
   Thr Gly Ala Glu Phe Leu Gly Asp Gly Gly Asp Val Glu Phe His Met
               420                 425                 430
   Val Asp Arg Asp Glu Leu Lys Gly Ile Gly Met Thr Asn Asn Pro Pro
           435                 440                 445
   Ile Pro Val Gly Glu Ile Tyr Lys Arg Trp Ile Ile LEu Gly Leu Asn
       450                 455                 460
   Lys Ile Val Arg Met Tyr Ser Pro Thr Met Thr Asn Asn Pro Pro Ile
   465                 470                 475                 480
   Pro Val Gly Glu Ile Tyr Arg Trp Ile Ile Leu Gly Leu Asn Lys Ile
                   485                 490                 495
   Val Arg Met Tyr Ser Pro Thr Leu Asp Val Glu Lys Leu Trp Val Thr
               500                 505                 510
   Val Tyr Tyr Gly Val Pro Val Trp Lys Lys Val Val Lys Ile Glu Pro
           515                 520                 525
   Leu Gly Val Ala Pro Thr Lys Cys Lys Arg Arg Val Val Gln Arg Glu
       530                 535                 540
   Lys Arg Gly Gly Gly Gly Gly Gln Ala Arg Val Ile Ala Val Glu Arg
   545                 550                 555                 560
   Tyr Leu Lys Asp Gln Gln Leu Leu Gly Ile Trp Gly Gly Ser Gly Lys
                   565                 570                 575
   Leu Ile Cys Cys Thr Thr Ala Val Pro Trp Asn Ser Ser Trp Ser Asn
               580                 585                 590
   Lys Leu Asp Arg Ile Trp Asn Asn Met Thr Trp Leu Glu Tyr Leu Lys
           595                 600                 605
   Lys Asp Glu Leu Arg Val Glu Leu Lys Asp Glu Leu
       610                 615                 620
<210> 240
   <211> 1749
   <212> DNA
   <213> Artificial sequence
<220>
   <223> Fusion gene PE(delta III)-HIV nef-N-K3
<400> 240
   atgggcagca gccatcatca tcatcatcac agcagcggcc tggtgccgcg cggcagccat    60
   atggccgaag aagctttcga cctctggaac gaatgcgcca aagcctgcgt gctcgacctc   120
   aaggacggcg tgcgttccag ccgcatgagc gtcgacccgg ccatcgccga caccaacggc   180
   cagggcgtgc tgcactactc catggtcctg gagggcggca acgacgcgct caagctggcc   240
   atcgacaacg ccctcagcat caccagcgac ggcctgacca tccgcctcga aggcggcgtc 300
   gagccgaaca agccggtgcg ctacagctac acgcgccagg cgcgcggcag ttggtcgctg 360
   aactggctgg taccgatcgg ccacgagaag ccctcgaaca tcaaggtgtt catccacgaa 420
   ctgaacgccg gcaaccagct cagccacatg tcgccgatct acaccatcga gatgggcgac 480
   gagttgctgg cgaagctggc gcgcgatgcc accttcttcg tcagggcgca cgagagcaac 540
   gagatgcagc cgacgctcgc catcagccat gccggggtca gcgtggtcat ggcccagacc 600
   cagccgcgcc gggaaaagcg ctggagcgaa tgggccagcg gcaaggtgtt gtgcctgctc 660
   gacccgctgg acggggtcta caactacctc gcccagcaac gctgcaacct cgacgatacc 720
   tgggaaggca agatctaccg ggtgctcgcc ggcaacccgg cgaagcatga cctggacatc 780
   aaacccacgg tcatcagtca tcgcctgcac tttcccgagg gcggcagcct ggccgcgctg 840
   accgcgcacc aggcttgcca cctgccgctg gagactttca cccgtcatcg ccagccgcgc 900
   ggctgggaac aactggagca gtgcggctat ccggtgcagc ggctggtcgc cctctacctg 960
   gcggcgcggc tgtcgtggaa ccaggtcgac caggtgatcc gcaacgccct ggccagcccc 1020
   ggcagcggcg gcgacctggg cgaagcgatc cgcgagcagc cggagcaggc ccgtctggcc 1080
   ctgaccctgg ccgccgccga gagcgagcgc ttcgtccggc agggcaccgg caacgacgag 1140
   gccggcgcgg ccaacgccga cgtggtgagc ctgacctgcc cggtcgccgc cggtgaatgc 1200
   gcgggcccgg cggacagcgg cgacgccctg ctggagcgca actatcccac tggcgcggag 1260
   ttcctcggcg acggcggcga cgtcgaattc catatggtcg acccgaccgt tcgtcagcgt 1320
   atggaccgta ccgaaccggc tgctgagggt gttggtgctg ttagccgtga cctggaaaaa 1380
   cacggtgcta tcacctcttc taacaccgct gctaccaacg ctgactgcgc ttggctggaa 1440
   gctcaggaag aagaagaagt tggttttccg gttcgtccgc aggttccgct gcgtccgatg 1500
   acctacaaag ctgctgttga catcagccac ttcctgaaag aaaaaggcgg cctggaaggt 1560
   ctgatctaca gccagaaacg tcaggaaatc ctggacctgt ggatctacca cacccagggc 1620
   tacttcccgg actggcagaa ctacaccccg ggtccgggta tccgttaccc gctgaccttc 1680
   ggctggtgtt tcaaactgct cgagtacctc aaaaaagacg aactgcgtgt agaactgaaa 1740
   gacgaactg 1749
<210> 241
   <211> 583
   <212> PRT
   <213> Artificial sequence
<220>
   <223> fusion protein PE(delta III)-HIV Nef-N-K3
<400> 241
   Met Gly Ser Ser His His His His His His Ser Ser Gly Leu Val Pro
   1               5                   10                  15
   Arg Gly Ser His Met Ala Glu Glu Ala Phe Asp Leu Trp Asn Glu Cys
               20                  25                  30
   Ala Lys Ala Cys Val Leu Asp Leu Lys Asp Gly Val Arg Ser Ser Arg
           35                  40                  45
   Met Ser Val Asp Pro Ala Ile Ala Asp Thr Asn Gly Gln Gly Val Leu
       50                  55                  60
   His Tyr Ser Met Val Leu Glu Gly Gly Asn Asp Ala Leu Lys Leu Ala
   65                  70                  75                  80
   Ile Asp Asn Ala Leu Ser Ile Thr Ser Asp Gly Leu Thr Ile Arg Leu
                   85                  90                  95
   Glu Gly Gly Val Glu Pro Asn Lys Pro Val Arg Tyr Ser Tyr Thr Arg
               100                 105                 110
   Gln Ala Arg Gly Ser Trp Ser Leu Asn Trp Leu Val Pro Ile Gly His
           115                 120                 125
   Glu Lys Pro Ser Asn Ile Lys Val Phe Ile His Glu Leu Asn Ala Gly
       130                 135                 140
   Asn Gln Leu Ser His Met Ser Pro Ile Tyr Thr Ile Glu Met Gly Asp
   145                 150                 155                 160
   Glu Leu Leu Ala Lys Leu Ala Arg Asp Ala Thr Phe Phe Val Arg Ala
                   165                 170                 175
   His Glu Ser Asn Glu Met Gln Pro Thr Leu Ala Ile Ser His Ala Gly
               180                 185                 190
   Val Ser Val Val Met Ala Gln Thr Gln Pro Arg Arg Glu Lys Arg Trp
           195                 200                 205
   Ser Glu Trp Ala Ser Gly Lys Val Leu Cys Leu Leu Asp Pro Leu Asp
       210                 215                 220
   Gly Val Tyr Asn Tyr Leu Ala Gln Gln Arg Cys Asn Leu Asp Asp Thr
   225                 230                 235                 240
   Trp Glu Gly Lys Ile Tyr Arg Val Leu Ala Gly Asn Pro Ala Lys His
                   245                 250                 255
   Asp Leu Asp Ile Lys Pro Thr Val Ile Ser His Arg Leu His Phe Pro
               260                 265                 270
   Glu Gly Gly Ser Leu Ala Ala Leu Thr Ala His Gln Ala Cys His Leu
           275                 280                 285
   Pro Leu Glu Thr Phe Thr Arg His Arg Gln Pro Arg Gly Trp Glu Gln
       290                 295                 300
   Leu Glu Gln Cys Gly Tyr Pro Val Gln Arg Leu Val Ala Leu Tyr Leu
   305                 310                 315                 320
   Ala Ala Arg Leu Ser Trp Asn Gln Val Asp Gln Val Ile Arg Asn Ala
                   325                 330                 335
   Leu Ala Ser Pro Gly Ser Gly Gly Asp Leu Gly Glu Ala Ile Arg Glu
               340                 345                 350
   Gln Pro Glu Gln Ala Arg Leu Ala Leu Thr Leu Ala Ala Ala Glu Ser
           355                 360                 365
   Glu Arg Phe Val Arg Gln Gly Thr Gly Asn Asp Glu Ala Gly Ala Ala
       370                 375                 380
   Asn Ala Asp Val Val Ser Leu Thr Cys Pro Val Ala Ala Gly Glu Cys
   385                 390                 395                 400
   Ala Gly Pro Ala Asp Ser Gly Asp Ala Leu Leu Glu Arg Asn Tyr Pro
                   405                 410                 415
   Thr Gly Ala Glu Phe Leu Gly Asp Gly Gly Asp Val Glu Phe His Met
               420                 425                 430
   Val Asp Pro Thr Val Arg Gln Arg Met Asp Arg Thr Glu Pro Ala Ala
           435                 440                 445
   Glu Gly Val Gly Ala Val Ser Arg Asp Leu Glu Lys His Gly Ala Ile
       450                 455                 460
   Thr Ser Ser Asn Thr Ala Ala Thr Asn Ala Asp Cys Ala Trp Leu Glu
   465                 470                 475                 480
   Ala Gln Glu Glu Glu Glu Val Gly Phe Pro Val Arg Pro Gln Val Pro
                   485                 490                 495
   Leu Arg Pro Met Thr Tyr Lys Ala Ala Val Asp Ile Ser His Phe Leu
               500                 505                 510
   Lys Glu Lys Gly Gly Leu Glu Gly Leu Ile Tyr Ser Gln Lys Arg Gln
           515                 520                 525
   Glu Ile Leu Asp Leu Trp Ile Tyr His Thr Gln Gly Tyr Phe Pro Asp
       530                 535                 540
   Trp Gln Asn Tyr Thr Pro Gly Pro Gly Ile Arg Tyr Pro Leu Thr Phe
   545                 550                 555                 560
   Gly Trp Cys Phe Lys Leu Leu Glu Tyr Leu Lys Lys Asp Glu Leu Arg
                   565                 570                 575
   Val Glu Leu Lys Asp Glu Leu
               580
<210> 242
   <211> 1881
   <212> DNA
   <213> Artificial sequence
<220>
   <223> Fusion gene PE(delta III)-HIV nef-C-K3
<400> 242
   atgggcagca gccatcatca tcatcatcac agcagcggcc tggtgccgcg cggcagccat     60
   atggccgaag aagctttcga cctctggaac gaatgcgcca aagcctgcgt gctcgacctc    120
   aaggacggcg tgcgttccag ccgcatgagc gtcgacccgg ccatcgccga caccaacggc    180
   cagggcgtgc tgcactactc catggtcctg gagggcggca acgacgcgct caagctggcc    240
   atcgacaacg ccctcagcat caccagcgac ggcctgacca tccgcctcga aggcggcgtc    300
   gagccgaaca agccggtgcg ctacagctac acgcgccagg cgcgcggcag ttggtcgctg    360
   aactggctgg taccgatcgg ccacgagaag ccctcgaaca tcaaggtgtt catccacgaa    420
   ctgaacgccg gcaaccagct cagccacatg tcgccgatct acaccatcga gatgggcgac    480
   gagttgctgg cgaagctggc gcgcgatgcc accttcttcg tcagggcgca cgagagcaac    540
   gagatgcagc cgacgctcgc catcagccat gccggggtca gcgtggtcat ggcccagacc    600
   cagccgcgcc gggaaaagcg ctggagcgaa tgggccagcg gcaaggtgtt gtgcctgctc    660
   gacccgctgg acggggtcta caactacctc gcccagcaac gctgcaacct cgacgatacc    720
   tgggaaggca agatctaccg ggtgctcgcc ggcaacccgg cgaagcatga cctggacatc    780
   aaacccacgg tcatcagtca tcgcctgcac tttcccgagg gcggcagcct ggccgcgctg    840
   accgcgcacc aggcttgcca cctgccgctg gagactttca cccgtcatcg ccagccgcgc    900
   ggctgggaac aactggagca gtgcggctat ccggtgcagc ggctggtcgc cctctacctg    960
   gcggcgcggc tgtcgtggaa ccaggtcgac caggtgatcc gcaacgccct ggccagcccc   1020
   ggcagcggcg gcgacctggg cgaagcgatc cgcgagcagc cggagcaggc ccgtctggcc   1080
   ctgaccctgg ccgccgccga gagcgagcgc ttcgtccggc agggcaccgg caacgacgag   1140
   gccggcgcgg ccaacgccga cgtggtgagc ctgacctgcc cggtcgccgc cggtgaatgc   1200
   gcgggcccgg cggacagcgg cgacgccctg ctggagcgca actatcccac tggcgcggag   1260
   ttcctcggcg acggcggcga cgtccgtcac cactttaccc cgagtgagcg tcaattgtgt   1320
   ttgtcgtcaa tccagactgc ctttaatcaa ggcgctggta cttgcatcct gtcagattct   1380
   gggcgtatca gttacactgt ggagtttagt ttgcctacgc atcatactgt gcgcctgatc   1440
   cgcgttacag caccaccgtc agcactcgac gcgaccgtct acaacggtag cagtaagtac   1500
   ggtgacacca gcactagcaa cgtgcgtggt gaccttcaag tgttagctca gaaggcagaa   1560
   cgtactctgc ctacctcctt caacttcggt gccatcaagg caactcgtgt tactgaattc   1620
   catatggtcg acttcctgaa agttccggtt gacccggaac aggttgaaaa agctaacgaa   1680
   ggtgataaca actgcctgct gcacccgatc agccagcacg gtatggacga cccggaaaaa   1740
   gaagttctga tgtggaaatt cgacagccgt ctggctttcc agcacatcgc tcgtgaaaaa   1800
   cacccggaat actacaaaga ctgcctgggt ctcgagtacc tcaaaaaaga cgaactgcgt   1860
   gtagaactga aagacgaact g                                             1881
<210> 243
   <211> 627
   <212> PRT
   <213> Artificial sequence
<220>
   <223> Fusion protein PE(delta III)-HIV Nef-C-K3
<400> 243
   Met Gly Ser Ser His His His His His His Ser Ser Gly Leu Val Pro
   1               5                   10                  15
   Arg Gly Ser His Met Ala Glu Glu Ala Phe Asp Leu Trp Asn Glu Cys
               20                  25                  30
   Ala Lys Ala Cys Val Leu Asp Leu Lys Asp Gly Val Arg Ser Ser Arg
           35                  40                  45
   Met Ser Val Asp Pro Ala Ile Ala Asp Thr Asn Gly Gln Gly Val Leu
       50                  55                  60
   His Tyr Ser Met Val Leu Glu Gly Gly Asn Asp Ala Leu Lys Leu Ala
   65                  70                  75                  80
   Ile Asp Asn Ala Leu Ser Ile Thr Ser Asp Gly Leu Thr Ile Arg Leu
                   85                  90                  95
   Glu Gly Gly Val Glu Pro Asn Lys Pro Val Arg Tyr Ser Tyr Thr Arg
               100                 105                 110
   Gln Ala Arg Gly Ser Trp Ser Leu Asn Trp Leu Val Pro Ile Gly His
           115                 120                 125
   Glu Lys Pro Ser Asn Ile Lys Val Phe Ile His Glu Leu Asn Ala Gly
       130                 135                 140
   Asn Gln Leu Ser His Met Ser Pro Ile Tyr Thr Ile Glu Met Gly Asp
   145                 150                 155                 160
   Glu Leu Leu Ala Lys Leu Ala Arg Asp Ala Thr Phe Phe Val Arg Ala
                   165                 170                 175
   His Glu Ser Asn Glu Met Gln Pro Thr Leu Ala Ile Ser His Ala Gly
               180                 185                 190
   Val Ser Val Val Met Ala Gln Thr Gln Pro Arg Arg Glu Lys Arg Trp
           195                 200                 205
   Ser Glu Trp Ala Ser Gly Lys Val Leu Cys Leu Leu Asp Pro Leu Asp
       210                 215                 220
   Gly Val Tyr Asn Tyr Leu Ala Gln Gln Arg Cys Asn Leu Asp Asp Thr
   225                 230                 235                 240
   Trp Glu Gly Lys Ile Tyr Arg Val Leu Ala Gly Asn Pro Ala Lys His
                   245                 250                 255
   Asp Leu Asp Ile Lys Pro Thr Val Ile Ser His Arg Leu His Phe Pro
               260                 265                 270
   Glu Gly Gly Ser Leu Ala Ala Leu Thr Ala His Gln Ala Cys His Leu
           275                 280                 285
   Pro Leu Glu Thr Phe Thr Arg His Arg Gln Pro Arg Gly Trp Glu Gln
       290                 295                 300
   Leu Glu Gln Cys Gly Tyr Pro Val Gln Arg Leu Val Ala Leu Tyr Leu
   305                 310                 315                 320
   Ala Ala Arg Leu Ser Trp Asn Gln Val Asp Gln Val Ile Arg Asn Ala
                   325                 330                 335
   Leu Ala Ser Pro Gly Ser Gly Gly Asp Leu Gly Glu Ala Ile Arg Glu
               340                 345                 350
   Gln Pro Glu Gln Ala Arg Leu Ala Leu Thr Leu Ala Ala Ala Glu Ser
           355                 360                 365
   Glu Arg Phe Val Arg Gln Gly Thr Gly Asn Asp Glu Ala Gly Ala Ala
       370                 375                 380
   Asn Ala Asp Val Val Ser Leu Thr Cys Pro Val Ala Ala Gly Glu Cys
   385                 390                 395                 400
   Ala Gly Pro Ala Asp Ser Gly Asp Ala Leu Leu Glu Arg Asn Tyr Pro
                   405                 410                 415
   Thr Gly Ala Glu Phe Leu Gly Asp Gly Gly Asp Val Arg His His Phe
               420                 425                 430
   Thr Pro Ser Glu Arg Gln Leu Cys Leu Ser Ser Ile Gln Thr Ala Phe
           435                 440                 445
   Asn Gln Gly Ala Gly Thr Cys Ile Leu Ser Asp Ser Gly Arg Ile Ser
       450                 455                 460
   Tyr Thr Val Glu Phe Ser Leu Pro Thr His His Thr Val Arg Leu Ile
   465                 470                 475                 480
   Arg Val Thr Ala Pro Pro Ser Ala Leu Asp Ala Thr Val Tyr Asn Gly
                   485                 490                 495
   Ser Ser Lys Tyr Gly Asp Thr Ser Thr Ser Asn Val Arg Gly Asp Leu
               500                 505                 510
   Gln Val Leu Ala Gln Lys Ala Glu Arg Thr Leu Pro Thr Ser Phe Asn
           515                 520                 525
   Phe Gly Ala Ile Lys Ala Thr Arg Val Thr Glu Phe His Met Val Asp
       530                 535                 540
   Phe Leu Lys Val Pro Val Asp Pro Glu Gln Val Glu Lys Ala Asn Glu
   545                 550                 555                 560
   Gly Asp Asn Asn Cys Leu Leu His Pro Ile Ser Gln His Gly Met Asp
                   565                 570                 575
   Asp Pro Glu Lys Glu Val Leu Met Trp Lys Phe Asp Ser Arg Leu Ala
               580                 585                 590
   Phe Gln His Ile Ala Arg Glu Lys His Pro Glu Tyr Tyr Lys Asp Cys
           595                 600                 605
   Leu Gly Leu Glu Tyr Leu Lys Lys Asp Glu Leu Arg Val Glu Leu Lys
       610                 615                 620
   Asp Glu Leu
   625
<210> 244
   <211> 1953
   <212> DNA
   <213> Artificial sequence
<220>
   <223> Fusion gene PE(delta III)-HIV nef-NC-K3
<400> 244
   atgggcagca gccatcatca tcatcatcac agcagcggcc tggtgccgcg cggcagccat    60
   atggccgaag aagctttcga cctctggaac gaatgcgcca aagcctgcgt gctcgacctc   120
   aaggacggcg tgcgttccag ccgcatgagc gtcgacccgg ccatcgccga caccaacggc   180
   cagggcgtgc tgcactactc catggtcctg gagggcggca acgacgcgct caagctggcc   240
   atcgacaacg ccctcagcat caccagcgac ggcctgacca tccgcctcga aggcggcgtc   300
   gagccgaaca agccggtgcg ctacagctac acgcgccagg cgcgcggcag ttggtcgctg   360
   aactggctgg taccgatcgg ccacgagaag ccctcgaaca tcaaggtgtt catccacgaa   420
   ctgaacgccg gcaaccagct cagccacatg tcgccgatct acaccatcga gatgggcgac   480
   gagttgctgg cgaagctggc gcgcgatgcc accttcttcg tcagggcgca cgagagcaac   540
   gagatgcagc cgacgctcgc catcagccat gccggggtca gcgtggtcat ggcccagacc   600
   cagccgcgcc gggaaaagcg ctggagcgaa tgggccagcg gcaaggtgtt gtgcctgctc   660
   gacccgctgg acggggtcta caactacctc gcccagcaac gctgcaacct cgacgatacc   720
   tgggaaggca agatctaccg ggtgctcgcc ggcaacccgg cgaagcatga cctggacatc   780
   aaacccacgg tcatcagtca tcgcctgcac tttcccgagg gcggcagcct ggccgcgctg   840
   accgcgcacc aggcttgcca cctgccgctg gagactttca cccgtcatcg ccagccgcgc   900
   ggctgggaac aactggagca gtgcggctat ccggtgcagc ggctggtcgc cctctacctg   960
   gcggcgcggc tgtcgtggaa ccaggtcgac caggtgatcc gcaacgccct ggccagcccc  1020
   ggcagcggcg gcgacctggg cgaagcgatc cgcgagcagc cggagcaggc ccgtctggcc 1080
   ctgaccctgg ccgccgccga gagcgagcgc ttcgtccggc agggcaccgg caacgacgag 1140
   gccggcgcgg ccaacgccga cgtggtgagc ctgacctgcc cggtcgccgc cggtgaatgc 1200
   gcgggcccgg cggacagcgg cgacgccctg ctggagcgca actatcccac tggcgcggag 1260
   ttcctcggcg acggcggcga cgtcgaattc catatggtcg acccgaccgt tcgtcagcgt 1320
   atggaccgta ccgaaccggc tgctgagggt gttggtgctg ttagccgtga cctggaaaaa 1380
   cacggtgcta tcacctcttc taacaccgct gctaccaacg ctgactgcgc ttggctggaa 1440
   gctcaggaag aagaagaagt tggttttccg gttcgtccgc aggttccgct gcgtccgatg 1500
   acctacaaag ctgctgttga catcagccac ttcctgaaag aaaaaggcgg cctggaaggt 1560
   ctgatctaca gccagaaacg tcaggaaatc ctggacctgt ggatctacca cacccagggc 1620
   tacttcccgg actggcagaa ctacaccccg ggtccgggta tccgttaccc gctgaccttc 1680
   ggctggtgtt tcaaactgct cgacttcctg aaagttccgg ttgacccgga acaggttgaa 1740
   aaagctaacg aaggtgataa caactgcctg ctgcacccga tcagccagca cggtatggac 1800
   gacccggaaa aagaagttct gatgtggaaa ttcgacagcc gtctggcttt ccagcacatc 1860
   gctcgtgaaa aacacccgga atactacaaa gactgcctgg gtctcgagta cctcaaaaaa 1920
   gacgaactgc gtgtagaact gaaagacgaa ctg 1953
<210> 245
   <211> 651
   <212> PRT
   <213> Artificial sequence
<220>
   <223> Fusion protein PE(delta III)-HIV Nef-NC-K3
<400> 245
   Met Gly Ser Ser His His His His His His Ser Ser Gly Leu Val Pro
   1               5                   10                  15
   Arg Gly Ser His Met Ala Glu Glu Ala Phe Asp Leu Trp Asn Glu Cys
               20                  25                  30
   Ala Lys Ala Cys Val Leu Asp Leu Lys Asp Gly Val Arg Ser Ser Arg
           35                  40                  45
   Met Ser Val Asp Pro Ala Ile Ala Asp Thr Asn Gly Gln Gly Val Leu
       50                  55                  60
   His Tyr Ser Met Val Leu Glu Gly Gly Asn Asp Ala Leu Lys Leu Ala
   65                  70                  75                  80
   Ile Asp Asn Ala Leu Ser Ile Thr Ser Asp Gly Leu Thr Ile Arg Leu
                   85                  90                  95
   Glu Gly Gly Val Glu Pro Asn Lys Pro Val Arg Tyr Ser Tyr Thr Arg
               100                 105                 110
   Gln Ala Arg Gly Ser Trp Ser Leu Asn Trp Leu Val Pro Ile Gly His
           115                 120                 125
   Glu Lys Pro Ser Asn Ile Lys Val Phe Ile His Glu Leu Asn Ala Gly
       130                 135                 140
   Asn Gln Leu Ser His Met Ser Pro Ile Tyr Thr Ile Glu Met Gly Asp
   145                 150                 155                 160
   Glu Leu Leu Ala Lys Leu Ala Arg Asp Ala Thr Phe Phe Val Arg Ala
                   165                 170                 175
   His Glu Ser Asn Glu Met Gln Pro Thr Leu Ala Ile Ser His Ala Gly
               180                 185                 190
   Val Ser Val Val Met Ala Gln Thr Gln Pro Arg Arg Glu Lys Arg Trp
           195                 200                 205
   Ser Glu Trp Ala Ser Gly Lys Val Leu Cys Leu Leu Asp Pro Leu Asp
       210                 215                 220
   Gly Val Tyr Asn Tyr Leu Ala Gln Gln Arg Cys Asn Leu Asp Asp Thr
   225                 230                 235                 240
   Trp Glu Gly Lys Ile Tyr Arg Val Leu Ala Gly Asn Pro Ala Lys His
                   245                 250                 255
   Asp Leu Asp Ile Lys Pro Thr Val Ile Ser His Arg Leu His Phe Pro
               260                 265                 270
   Glu Gly Gly Ser Leu Ala Ala Leu Thr Ala His Gln Ala Cys His Leu
           275                 280                 285
   Pro Leu Glu Thr Phe Thr Arg His Arg Gln Pro Arg Gly Trp Glu Gln
       290                 295                 300
   Leu Glu Gln Cys Gly Tyr Pro Val Gln Arg Leu Val Ala Leu Tyr Leu
   305                 310                 315                 320
   Ala Ala Arg Leu Ser Trp Asn Gln Val Asp Gln Val Ile Arg Asn Ala
                   325                 330                 335
   Leu Ala Ser Pro Gly Ser Gly Gly Asp Leu Gly Glu Ala Ile Arg Glu
               340                 345                 350
   Gln Pro Glu Gln Ala Arg Leu Ala Leu Thr Leu Ala Ala Ala Glu Ser
           355                 360                 365
   Glu Arg Phe Val Arg Gln Gly Thr Gly Asn Asp Glu Ala Gly Ala Ala
       370                 375                 380
   Asn Ala Asp Val Val Ser Leu Thr Cys Pro Val Ala Ala Gly Glu Cys
   385                 390                 395                 400
   Ala Gly Pro Ala Asp Ser Gly Asp Ala Leu Leu Glu Arg Asn Tyr Pro
                   405                 410                 415
   Thr Gly Ala Glu Phe Leu Gly Asp Gly Gly Asp Val Glu Phe His Met
               420                 425                 430
   Val Asp Pro Thr Val Arg Gln Arg Met Asp Arg Thr Glu Pro Ala Ala
           435                 440                 445
   Glu Gly Val Gly Ala Val Ser Arg Asp Leu Glu Lys His Gly Ala Ile
       450                 455                 460
   Thr Ser Ser Asn Thr Ala Ala Thr Asn Ala Asp Cys Ala Trp Leu Glu
   465                 470                 475                 480
   Ala Gln Glu Glu Glu Glu Val Gly Phe Pro Val Arg Pro Gln Val Pro
                   485                 490                 495
   Leu Arg Pro Met Thr Tyr Lys Ala Ala Val Asp Ile Ser His Phe Leu
               500                 505                 510
   Lys Glu Lys Gly Gly Leu Glu Gly Leu Ile Tyr Ser Gln Lys Arg Gln
           515                 520                 525
   Glu Ile Leu Asp Leu Trp Ile Tyr His Thr Gln Gly Tyr Phe Pro Asp
       530                 535                 540
   Trp Gln Asn Tyr Thr Pro Gly Pro Gly Ile Arg Tyr Pro Leu Thr Phe
   545                 550                 555                 560
   Gly Trp Cys Phe Lys Leu Leu Asp Phe Leu Lys Val Pro Val Asp Pro
                   565                 570                 575
   Glu Gln Val Glu Lys Ala Asn Glu Gly Asp Asn Asn Cys Leu Leu His
               580                 585                 590
   Pro Ile Ser Gln His Gly Met Asp Asp Pro Glu Lys Glu Val Leu Met
           595                 600                 605
   Trp Lys Phe Asp Ser Arg Leu Ala Phe Gln His Ile Ala Arg Glu Lys
       610                 615                 620
   His Pro Glu Tyr Tyr Lys Asp Cys Leu Gly Leu Glu Tyr Leu Lys Lys
   625                 630                 635                 640
   Asp Glu Leu Arg Val Glu Leu Lys Asp Glu Leu
                   645                 650
<210> 246
   <211> 1947
   <212> DNA
   <213> Artificial sequence
<220>
   <223> Fusion gene PE(delta III)-HIV rev-K3
<400> 246
   atgggcagca gccatcatca tcatcatcac agcagcggcc tggtgccgcg cggcagccat     60
   atggccgaag aagctttcga cctctggaac gaatgcgcca aagcctgcgt gctcgacctc    120
   aaggacggcg tgcgttccag ccgcatgagc gtcgacccgg ccatcgccga caccaacggc 180
   cagggcgtgc tgcactactc catggtcctg gagggcggca acgacgcgct caagctggcc 240
   atcgacaacg ccctcagcat caccagcgac ggcctgacca tccgcctcga aggcggcgtc 300
   gagccgaaca agccggtgcg ctacagctac acgcgccagg cgcgcggcag ttggtcgctg 360
   aactggctgg taccgatcgg ccacgagaag ccctcgaaca tcaaggtgtt catccacgaa 420
   ctgaacgccg gcaaccagct cagccacatg tcgccgatct acaccatcga gatgggcgac 480
   gagttgctgg cgaagctggc gcgcgatgcc accttcttcg tcagggcgca cgagagcaac 540
   gagatgcagc cgacgctcgc catcagccat gccggggtca gcgtggtcat ggcccagacc 600
   cagccgcgcc gggaaaagcg ctggagcgaa tgggccagcg gcaaggtgtt gtgcctgctc 660
   gacccgctgg acggggtcta caactacctc gcccagcaac gctgcaacct cgacgatacc 720
   tgggaaggca agatctaccg ggtgctcgcc ggcaacccgg cgaagcatga cctggacatc 780
   aaacccacgg tcatcagtca tcgcctgcac tttcccgagg gcggcagcct ggccgcgctg 840
   accgcgcacc aggcttgcca cctgccgctg gagactttca cccgtcatcg ccagccgcgc 900
   ggctgggaac aactggagca gtgcggctat ccggtgcagc ggctggtcgc cctctacctg 960
   gcggcgcggc tgtcgtggaa ccaggtcgac caggtgatcc gcaacgccct ggccagcccc 1020
   ggcagcggcg gcgacctggg cgaagcgatc cgcgagcagc cggagcaggc ccgtctggcc 1080
   ctgaccctgg ccgccgccga gagcgagcgc ttcgtccggc agggcaccgg caacgacgag 1140
   gccggcgcgg ccaacgccga cgtggtgagc ctgacctgcc cggtcgccgc cggtgaatgc 1200
   gcgggcccgg cggacagcgg cgacgccctg ctggagcgca actatcccac tggcgcggag 1260
   ttcctcggcg acggcggcga cgtccgtcac cactttaccc cgagtgagcg tcaattgtgt 1320
   ttgtcgtcaa tccagactgc ctttaatcaa ggcgctggta cttgcatcct gtcagattct 1380
   gggcgtatca gttacactgt ggagtttagt ttgcctacgc atcatactgt gcgcctgatc 1440
   cgcgttacag caccaccgtc agcactcgac gcgaccgtct acaacggtag cagtaagtac 1500
   ggtgacacca gcactagcaa cgtgcgtggt gaccttcaag tgttagctca gaaggcagaa 1560
   cgtactctgc ctacctcctt caacttcggt gccatcaagg caactcgtgt tactgaattc 1620
   catatggtcg acctgctggc tgttcgtatc atcaaaaccc tgtaccagtc taacccgtac 1680
   ccgaaaccgg aaggttaccg tcgtgtccgt cgtaaccgcc gccgccgctg gcgtgctcgt 1740
   cagcgtcaga tccactccat cagcgaacgt atcctgatca cctgcctggg tcgtccgacc 1800
   gaaccggttc cgctgcaact gccgccgatc gaacgtctga acatcaactg tagcgaaagc 1860
   ggtggtacca gcggtaccca gcgtgttggt aacccgctcg agtacctcaa aaaagacgaa 1920
   ctgcgtgtag aactgaaaga cgaactg 1947
<210> 247
   <211> 649
   <212> PRT
   <213> Artificial sequence
<220>
   <223> Fusion protein PE(delta III)-HIV Rev-K3
<400> 247
   Met Gly Ser Ser His His His His His His Ser Ser Gly Leu Val Pro
   1               5                   10                  15
   Arg Gly Ser His Met Ala Glu Glu Ala Phe Asp Leu Trp Asn Glu Cys
               20                  25                  30
   Ala Lys Ala Cys Val Leu Asp Leu Lys Asp Gly Val Arg Ser Ser Arg
           35                  40                  45
   Met Ser Val Asp Pro Ala Ile Ala Asp Thr Asn Gly Gln Gly Val Leu
       50                  55                  60
   His Tyr Ser Met Val Leu Glu Gly Gly Asn Asp Ala Leu Lys Leu Ala
   65                  70                  75                  80
   Ile Asp Asn Ala Leu Ser Ile Thr Ser Asp Gly Leu Thr Ile Arg Leu
                   85                  90                  95
   Glu Gly Gly Val Glu Pro Asn Lys Pro Val Arg Tyr Ser Tyr Thr Arg
               100                 105                 110
   Gln Ala Arg Gly Ser Trp Ser Leu Asn Trp Leu Val Pro Ile Gly His
           115                 120                 125
   Glu Lys Pro Ser Asn Ile Lys Val Phe Ile His Glu Leu Asn Ala Gly
       130                 135                 140
   Asn Gln Leu Ser His Met Ser Pro Ile Tyr Thr Ile Glu Met Gly Asp
   145                 150                 155                 160
   Glu Leu Leu Ala Lys Leu Ala Arg Asp Ala Thr Phe Phe Val Arg Ala
                   165                 170                 175
   His Glu Ser Asn Glu Met Gln Pro Thr Leu Ala Ile Ser His Ala Gly
               180                 185                 190
   Val Ser Val Val Met Ala Gln Thr Gln Pro Arg Arg Glu Lys Arg Trp
           195                 200                 205
   Ser Glu Trp Ala Ser Gly Lys Val Leu Cys Leu Leu Asp Pro Leu Asp
       210                 215                 220
   Gly Val Tyr Asn Tyr Leu Ala Gln Gln Arg Cys Asn Leu Asp Asp Thr
   225                 230                 235                 240
   Trp Glu Gly Lys Ile Tyr Arg Val Leu Ala Gly Asn Pro Ala Lys His
                   245                 250                 255
   Asp Leu Asp Ile Lys Pro Thr Val Ile Ser His Arg Leu His Phe Pro
               260                 265                 270
   Glu Gly Gly Ser Leu Ala Ala Leu Thr Ala His Gln Ala Cys His Leu
           275                 280                 285
   Pro Leu Glu Thr Phe Thr Arg His Arg Gln Pro Arg Gly Trp Glu Gln
       290                 295                 300
   Leu Glu Gln Cys Gly Tyr Pro Val Gln Arg Leu Val Ala Leu Tyr Leu
   305                 310                 315                 320
   Ala Ala Arg Leu Ser Trp Asn Gln Val Asp Gln Val Ile Arg Asn Ala
                   325                 330                 335
   Leu Ala Ser Pro Gly Ser Gly Gly Asp Leu Gly Glu Ala Ile Arg Glu
               340                 345                 350
   Gln Pro Glu Gln Ala Arg Leu Ala Leu Thr Leu Ala Ala Ala Glu Ser
           355                 360                 365
   Glu Arg Phe Val Arg Gln Gly Thr Gly Asn Asp Glu Ala Gly Ala Ala
       370                 375                 380
   Asn Ala Asp Val Val Ser Leu Thr Cys Pro Val Ala Ala Gly Glu Cys
   385                 390                 395                 400
   Ala Gly Pro Ala Asp Ser Gly Asp Ala Leu Leu Glu Arg Asn Tyr Pro
                   405                 410                 415
   Thr Gly Ala Glu Phe Leu Gly Asp Gly Gly Asp Val Arg His His Phe
               420                 425                 430
   Thr Pro Ser Glu Arg Gln Leu Cys Leu Ser Ser Ile Gln Thr Ala Phe
           435                 440                 445
   Asn Gln Gly Ala Gly Thr Cys Ile Leu Ser Asp Ser Gly Arg Ile Ser
       450                 455                 460
   Tyr Thr Val Glu Phe Ser Leu Pro Thr His His Thr Val Arg Leu Ile
   465                 470                 475                 480
   Arg Val Thr Ala Pro Pro Ser Ala Leu Asp Ala Thr Val Tyr Asn Gly
                   485                 490                 495
   Ser Ser Lys Tyr Gly Asp Thr Ser Thr Ser Asn Val Arg Gly Asp Leu
               500                 505                 510
   Gln Val Leu Ala Gln Lys Ala Glu Arg Thr Leu Pro Thr Ser Phe Asn
           515                 520                 525
   Phe Gly Ala Ile Lys Ala Thr Arg Val Thr Glu Phe His Met Val Asp
       530                 535                 540
   Leu Leu Ala Val Arg Ile Ile Lys Thr Leu Tyr Gln Ser Asn Pro Tyr
   545                 550                 555                 560
   Pro Lys Pro Glu Gly Tyr Arg Arg Val Arg Arg Asn Arg Arg Arg Arg
                   565                 570                 575
   Trp Arg Ala Arg Gln Arg Gln Ile His Ser Ile Ser Glu Arg Ile Leu
                   580                   585                   590
   Ile Thr Cys Leu Gly Arg Pro Thr Glu Pro Val Pro Leu Gln Leu Pro
           595                 600                 605
   Pro Ile Glu Arg Leu AsN Ile AsN Cys Ser Glu Ser Gly Gly Thr Ser
       610                 615                 620
   Gly Thr Gln Arg Val Gly Asn Pro Leu Glu Tyr Leu Lys Lys Asp Glu
   625                 630                 635                 640
   Leu Arg Val Glu Leu Lys Asp Glu Leu
                   645
<210> 248
   <211> 1680
   <212> DNA
   <213> Artificial sequence
<220>
   <223> Fusion gene PE(delta III)-HIV tat-K3
<400> 248
   atgggcagca gccatcatca tcatcatcac agcagcggcc tggtgccgcg cggcagccat     60
   atggccgaag aagctttcga cctctggaac gaatgcgcca aagcctgcgt gctcgacctc    120
   aaggacggcg tgcgttccag ccgcatgagc gtcgacccgg ccatcgccga caccaacggc    180
   cagggcgtgc tgcactactc catggtcctg gagggcggca acgacgcgct caagctggcc    240
   atcgacaacg ccctcagcat caccagcgac ggcctgacca tccgcctcga aggcggcgtc    300
   gagccgaaca agccggtgcg ctacagctac acgcgccagg cgcgcggcag ttggtcgctg    360
   aactggctgg taccgatcgg ccacgagaag ccctcgaaca tcaaggtgtt catccacgaa    420
   ctgaacgccg gcaaccagct cagccacatg tcgccgatct acaccatcga gatgggcgac    480
   gagttgctgg cgaagctggc gcgcgatgcc accttcttcg tcagggcgca cgagagcaac    540
   gagatgcagc cgacgctcgc catcagccat gccggggtca gcgtggtcat ggcccagacc    600
   cagccgcgcc gggaaaagcg ctggagcgaa tgggccagcg gcaaggtgtt gtgcctgctc    660
   gacccgctgg acggggtcta caactacctc gcccagcaac gctgcaacct cgacgatacc    720
   tgggaaggca agatctaccg ggtgctcgcc ggcaacccgg cgaagcatga cctggacatc    780
   aaacccacgg tcatcagtca tcgcctgcac tttcccgagg gcggcagcct ggccgcgctg    840
   accgcgcacc aggcttgcca cctgccgctg gagactttca cccgtcatcg ccagccgcgc    900
   ggctgggaac aactggagca gtgcggctat ccggtgcagc ggctggtcgc cctctacctg    960
   gcggcgcggc tgtcgtggaa ccaggtcgac caggtgatcc gcaacgccct ggccagcccc   1020
   ggcagcggcg gcgacctggg cgaagcgatc cgcgagcagc cggagcaggc ccgtctggcc   1080
   ctgaccctgg ccgccgccga gagcgagcgc ttcgtccggc agggcaccgg caacgacgag   1140
   gccggcgcgg ccaacgccga cgtggtgagc ctgacctgcc cggtcgccgc cggtgaatgc   1200
   gcgggcccgg cggacagcgg cgacgccctg ctggagcgca actatcccac tggcgcggag   1260
   ttcctcggcg acggcggcga cgtcgaattc catatggtcg accgtgacga actgaaaggt   1320
   atcggtatgg aaccggttga cccgcgtctg gaaccgtgga aacacccggg tagccagccg   1380
   cgtaccgctt gcaacaactg ctactgcaaa aaatgttgtt tccactgccc ggtttgcttt   1440
   atctctaaag gtctgggtat cagctacggt cgtaaaaagc gtcgccagcg ccgccgcgct   1500
   ccgcaggact ccgaaaccca ccaggttagc ctgagcaagc aaccgaccag ccagctgcgt   1560
   ggtgacccga ccggtccgaa agaaagcaaa aaaaaagttg aacgtgaaac cgaaaccgac   1620
   ccgaacgttc tcgagtacct caaaaaagac gaactgcgtg tagaactgaa agacgaactg   1680
<210> 249
   <211> 560
   <212> PRT
   <213> Artificial sequence
<220>
   <223> Fusion protein PE(delta III)-HIV Tat-K3
<400> 249
   Met Gly Ser Ser His His His His His His Ser Ser Gly Leu Val Pro
   1               5                   10                  15
   Arg Gly Ser His Met Ala Glu Glu Ala Phe Asp Leu Trp Asn Glu Cys
               20                  25                  30
   Ala Lys Ala Cys Val Leu Asp Leu Lys Asp Gly Val Arg Ser Ser Arg
           35                  40                  45
   Met Ser Val Asp Pro Ala Ile Ala Asp Thr Asn Gly Gln Gly Val Leu
       50                  55                  60
   His Tyr Ser Met Val Leu Glu Gly Gly Asn Asp Ala Leu Lys Leu Ala
   65                  70                  75                  80
   Ile Asp Asn Ala Leu Ser Ile Thr Ser Asp Gly Leu Thr Ile Arg Leu
                   85                  90                  95
   Glu Gly Gly Val Glu Pro Asn Lys Pro Val Arg Tyr Ser Tyr Thr Arg
               100                 105                 110
   Gln Ala Arg Gly Ser Trp Ser Leu Asn Trp Leu Val Pro Ile Gly His
           115                 120                 125
   Glu Lys Pro Ser Asn Ile Lys Val Phe Ile His Glu Leu Asn Ala Gly
       130                 135                 140
   Asn Gln Leu Ser His Met Ser Pro Ile Tyr Thr Ile Glu Met Gly Asp
   145                 150                 155                 160
   Glu Leu Leu Ala Lys Leu Ala Arg Asp Ala Thr Phe Phe Val Arg Ala
                   165                 170                 175
   His Glu Ser Asn Glu Met Gln Pro Thr Leu Ala Ile Ser His Ala Gly
               180                 185                 190
   Val Ser Val Val Met Ala Gln Thr Gln Pro Arg Arg Glu Lys Arg Trp
           195                 200                 205
   Ser Glu Trp Ala Ser Gly Lys Val Leu Cys Leu Leu Asp Pro Leu Asp
       210                 215                 220
   Gly Val Tyr Asn Tyr Leu Ala Gln Gln Arg Cys Asn Leu Asp Asp Thr
   225                 230                 235                 240
   Trp Glu Gly Lys Ile Tyr Arg Val Leu Ala Gly Asn Pro Ala Lys His
                   245                 250                 255
   Asp Leu Asp Ile Lys Pro Thr Val Ile Ser His Arg Leu His Phe Pro
               260                 265                 270
   Glu Gly Gly Ser Leu Ala Ala Leu Thr Ala His Gln Ala Cys His Leu
           275                 280                 285
   Pro Leu Glu Thr Phe Thr Arg His Arg Gln Pro Arg Gly Trp Glu Gln
       290                 295                 300
   Leu Glu Gln Cys Gly Tyr Pro Val Gln Arg Leu Val Ala Leu Tyr Leu
   305                 310                 315                 320
   Ala Ala Arg Leu Ser Trp Asn Gln Val Asp Gln Val Ile Arg Asn Ala
                   325                 330                 335
   Leu Ala Ser Pro Gly Ser Gly Gly Asp Leu Gly Glu Ala Ile Arg Glu
               340                 345                 350
   Gln Pro Glu Gln Ala Arg Leu Ala Leu Thr Leu Ala Ala Ala Glu Ser
           355                 360                 365
   Glu Arg Phe Val Arg Gln Gly Thr Gly Asn Asp Glu Ala Gly Ala Ala
       370                 375                 380
   Asn Ala Asp Val Val Ser Leu Thr Cys Pro Val Ala Ala Gly Glu Cys
   385                 390                 395                 400
   Ala Gly Pro Ala Asp Ser Gly Asp Ala Leu Leu Glu Arg Asn Tyr Pro
                   405                 410                 415
   Thr Gly Ala Glu Phe Leu Gly Asp Gly Gly Asp Val Glu Phe His Met
               420                 425                 430
   Val Asp Arg Asp Glu Leu Lys Gly Ile Gly Met Glu Pro Val Asp Pro
           435                 440                 445
   Arg Leu Glu Pro Trp Lys His Pro Gly Ser Gln Pro Arg Thr Ala Cys
       450                 455                 460
   Asn Asn Cys Tyr Cys Lys Lys Cys Cys Phe His Cys Pro Val Cys Phe
   465                 470                 475                 480
   Ile Ser Lys Gly Leu Gly Ile Ser Tyr Gly Arg Lys Lys Arg Arg Gln
                   485                 490                 495
   Arg Arg Arg Ala Pro Gln Asp Ser Glu Thr His Gln Val Ser Leu Ser
               500                 505                 510
   Lys Gln Pro Thr Ser Gln Leu Arg Gly Asp Pro Thr Gly Pro Lys Glu
           515                 520                 525
   Ser Lys Lys Lys Val Glu Arg Glu Thr Glu Thr Asp Pro Asn Val Leu
       530                 535                 540
   Glu Tyr Leu Lys Lys Asp Glu Leu Arg Val Glu Leu Lys Asp Glu Leu
   545                 550                 555                 560

<210> 250
   <211> 12
   <212> PRT
   <213> Artificial sequence
<220>
   <223> Carboxyl terminal moiety
<400> 250
   Lys Lys Asp Leu Arg Asp Glu Leu Lys Asp Glu Leu
   1               5                   10
<210> 251
   <211> 13
   <212> PRT
   <213> Artificial sequence
<220>
   <223> Carboxyl terminal moiety
<400> 251
   Lys Lys Asp Glu Leu Arg Asp Glu Leu Lys Asp Glu Leu
   1               5                   10
<210> 252
   <211> 13
   <212> PRT
   <213> Artificial sequence
<220>
   <223> Carboxyl terminal moiety
<400> 252
   Lys Lys Asp Glu Leu Arg Val Glu Leu Lys Asp Glu Leu
   1               5                   10

## Description

### FIELD OF THE INVENTION

The present invention relates generally to HIV vaccines, and more specifically to chimeric HIV fusion proteins useful for inducing humoral and cell-mediated immune responses.

### BACKGROUND OF THE INVENTION

The global epidemic of AIDS has created an urgent need for a vaccine against Human immunodeficiency virus type I (HIV-1). It is likely that effective AIDS vaccines will need to generate efficient humoral and cellular immune responses. Virus-neutralizing antibodies and anti-HIV cytotoxic (CD8+) T lymphocytes (CTLs) mediated immunity are major requirements for protective immune responses elicited by HIV vaccines.

HIV has several major genes coding for viral proteins. The *gag* gene codes for p24, the viral capsid; p6 and p7, the nucleocapsid proteins; and p 17, a matrix protein. The *pol* gene codes for reverse transcriptase, integrase, and protease which cleaves the proteins derived from gag and *pol* into functional proteins. The *env* gene codes for the precursor to gp120 and gyp41, envelope proteins embedded in the viral envelope that enable the virus to attach to and fuse with target cells. The *tal, rev, nef, vif, vpr, vpu* genes each codes for a single protein with the same names, Tat, Rev, Nef, Vif, Vpr, Vpu, respectively.

Neutralizing antibodies have been shown to contribute to protection from virus infection in animal models of HIV-1 infection. The virus-specific targets on HIV-1 accessible to neutralizing antibodies are the envelope glycoproteins (Yang, X. et al. (2005) "Stoichiometry of Antibody Neutralization of Human Immunodeficiency Virus Type I" Journal of Virology 79: 3500-3508). During the normal course of HIV-1 infections, virus-neutralizing antibodies are often generated but the titer of neutralizing is often low. Most neutralizing antibodies bind the gp120 envelope glycoprotein, which is the major exposed protein of the viral envelope glycoprotein trimer. The more conserved receptor-binding surfaces of the HIV-1 gp120 glycoprotein are also the targets for neutralizing antibodies. The CD4-binding site (CD4BS) antibodies recognize a conformational epitope composed of several segments of gp120 region that overlaps the binding site for CD4. CD4-induced (CD4i) antibodies bind a highly conserved gp120 element that is critical for the gp120-chemokine receptor interaction. The ability of CDBS and CD4i antibodies to interfere with receptor binding contributes to their neutralizing capability.

GP120 contains ten domains: conserved domains 1-5 (C1-C5) and variable domains 1-5 (VI-V5). The C 1 and C5 domains are located at N- and C-terminals of gp 120, respectively. Antibodies directed against the V3 loop, which determines chemokine receptor choice, can block the binding of gp120 to the receptors CCR5 and/or CXCR4. Neutralization by anti-V3 antibodies, although potent, is often limited to a small number of HIV-1 strains.

Gp120 is non-covalently associated with gp41. The gp41 subunit is anchored in the membrane and has a non-polar fusion peptide at its N-terminus. The gp120-gp41 complex forms oligomers on the surface of infected cells and on virions. The binding of gp120 to CD4 is thought to result in activation of the membrane fusion activity of gyp41, leading to entry of the viral nucleocapsid into a cell. Antibodies to gp41 epitopes in the serum of HIV-infected individuals may play an important role in virus neutralization. Gp120-41 complex sequences of different HIV subtypes show a remarkably conserved N-terminal coiled-coil structures of gp41 as well as the C-terminal residues that interact with the N-terminal core structure of gp120.

Multiple immune effectors participate in prevention, containment and clearance of HIV infection. To prevent infection of host target cells, antibodies are required. After the first target cells have been infected with virus, it is important to have cytotoxic T lymphocytes (CTLs) as well as antibodies to reduce cell-to-cell spread and kill infected cells. An effective HIV vaccine should evoke antibodies that can bind to virus and prevent attachment of virus to target cells, as well as CTLs that can eliminate any cells that become infected.

It remains a difficult goal for vaccinologists to construct live-attenuated viruses that are both effective and safe, or to mimic the presentation of viral proteins observed in infection with recombinant antigens or with replicating or non-replicating vectors carrying appropriate genes or antigens. The large number of mutations in the V3 domain of gp120 has limited its usefulness as a target for HIV vaccine. It is still unclear how the trend of hypervariability in the variable domains is developing and how many domains are absolutely invariant in the evolving strains of HIV.

A previously unaddressed need exists in the art to address the deficiencies and inadequacies in HIV vaccine antigen production, especially in connection with the provision of efficacious, antigenic determinant peptides.

US 7, 314, 632 (Fitzgerald, D. J. et al.) discloses pseudomonas exotoxin A-like chimeric immunogens that include a non-native epitope in the Ib domain of Pseudomonas exotoxin, and methods of eliciting an immune response using these immunogens.

Cryz S. et al ("Human immunodeficiency virus-1 principal neutralizing domain peptide-toxin A conjugate vaccine" Vaccine, Elsevier Ltd, GB, vol. 13, no. 1, 1 January 1995, pages 67-71) describe coupling of a principal neutralizing domain (PND) peptide (KRIHIGPGRAFYT) which is a fragment of the HIV gp 120-V3 protein to *Pseudomonas aeruginosa* toxin A (TA).

### SUMMARY OF THE INVENTION

The importance of interaction between gp120 and gp41 for determination of the neutralization phenotype has been studied. One aspect of the invention relates to a chimeric fusion protein useful as an immunogen for inducing HIV antigen specific immune responses. The chimeric fusion protein comprises: (a) a first polypeptidyl region comprising a *Pseudomonas Exotoxin* A (PE) binding domain and a PE translocation domain, located at the N-terminus of the fusion protein; and (b) a second polypeptidyl region with a fusion peptide of HIV gp120-C1-C5-gp41 with the amino acid sequence of SEQ ID NO: 7.

In one embodiment of the invention, the fusion protein further includes an endoplasmic reticulum retention sequence, e.g., the amino acid sequence KDEL, at the C- terminus. In another embodiment of the invention, the chimeric fusion protein further includes an intermediate polypeptidyl region between the first and the second polypeptidyl regions, in which the intermediate polypeptidyl region contains a non-Env, HIV antigenic determinant. In one embodiment of the invention, the intermediate polypeptidyl region has the amino acid sequence of HIV Gag24. In another embodiment of the invention, the intermediate polypeptidyl region includes Gag24 amino acid sequence or a fragment thereof. Further in another embodiment of the invention, the intermediate polypeptidyl region contains an N- or C- terminus of Gag24 amino acid sequence. In one embodiment of the invention, the intermediate polypeptidyl region contains the amino acid sequence set forth by SEQ ID NO: 151.

Another aspect of the invention relates to a chimeric HIV fusion protein as aforementioned for use as an immunogen for inducing HIV antigen-specific immune responses.

Yet another aspect of the invention relates to the use of a chimeric HIV fusion protein as aforementioned in the manufacture of a medicament for inducing an HIV antigen-specific immune response. The medicament may comprise an effective amount of the chimeric fusion protein as described above in a biocompatible carrier fluid suitable for carrying and delivering a predetermined aliquot of the fusion protein to a pre-chosen site in a living subject.

These and other aspects will become apparent from the following description of the preferred embodiment taken in conjunction with the following drawings, although variations and modifications therein may be affected without departing from the spirit and scope of the novel concepts of the disclosure.

The accompanying drawings illustrate one or more embodiments of the invention and, together with the written description, serve to explain the principles of the invention. Wherever possible, the same reference numbers are used throughout the drawings to refer to the same or like elements of an embodiment.

### BRIEF DESCRIPTION OF THE DRAWINGS

FIGs. 1A-1F are the maps of plasmids encoding chimeric HIV fusion proteins.
FIG. 2A is a graph showing ELISA titers in the sera samples from animals immunized with various chimeric HIV envelope fusion proteins. Sera sample 1:2,500 dilution.
FIG. 2B is a graph showing ELISA titers in the sera samples from animals immunized with various chimeric HIV envelope fusion proteins. Sera sample 1:12,500 dilution.
FIG. 3 is a graph showing neutralizing antibodies against live viruses were induced in the mice immunized with HIV Env fusion protein vaccines.
FIG. 4 is a flow chart illustrating the construction of pPE(ΔIII)-HIV gag24-K3.
FIG. 5 is a flow chart illustrating the construction of pPE(ΔIII)-HIV gag24-gp 120-41-K3.
FIG. 6 is a schematic drawing illustrating the fusion of an HIV antigenic determinant peptide to gp120 C1-C5-gp41 creates a chimeric that enhances the antigenic determinant peptide's cell-mediated immune response.
FIG. 7 is a flow chart illustrating the construction of pPE(ΔIII)-HIV nef NC-K3.

### DETAILED DESCRIPTION OF THE INVENTION

### DEFINITIONS

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term, the scope and meaning of a term is the same, in the same context, whether or not it is highlighted. It will be appreciated that same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

Unless otherwise defined, all technical and scientific terms used herein have the same meaning as commonly understood by one of ordinary skill in the art to which this invention pertains. In the case of conflict, the present document, including definitions will control.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

As used herein, the term "carboxyl terminal moiety which permits retention of the fusion antigen to the endoplasmic reticulum (ER) membrane of a target cell" refers to a peptide fragment that enables the fusion antigen to bind to the ER membrane and to retain it in the ER lumen for glycosylation and make it appears to be more like foreign protein. In one embodiment of the invention, the carboxyl terminal moiety comprises, in a direction from the amino terminus to the carboxyl terminus, the following amino acid residues:

R¹-R²-R³-R⁴-(R⁵)ₙ

Wherein,
R¹ is a positively charged amino acid residue;
R² is a negatively charged amino acid residue;
R³ is a negatively charged amino acid residue;
R⁴ is L;
R⁵ is a positively charged amino acid residue; and
n is 0 or 1.

Preferably, the carboxyl terminal moiety is a member of the KDEL family protein. As used herein, the term "KDEL family protein" refers to a group of proteins, which has a similar carboxyl end binding to the ER membrane of a cell and further has an ability for retention of such protein in the ER lumen. Generally, the length of the carboxyl end ranges from 4 to 16 residues. As discussed in U.S. Pat. No, 5,705,163, the amino residues at the carboxyl end of a KDEL family protein, particularly those in the last five amino acids, are important. As shown in the studies on the similar sequences present in different molecules and performing a specific biological function, a sequence that retains a newly formed protein within the endoplasmic reticulum is Lys Asp Glu Leu (KDEL). These findings suggest that the sequence at the carboxyl end of the fusion antigen according to the invention acts as some type of recognition sequence to assist translocation of the fusion antigen from an endocytic compartment into the ER and retains it in the lumen. The carboxyl terminal moiety comprises the sequence of KDEL. For example, the carboxyl terminal moiety may comprise the sequence of KKDLRDELKDEL (SEQ ID NO: 250), KKDELRDELKDEL (SEQ ID NO 251), KKDELRVELKDEL (SEQ ID NO 252), or KKDELRXELKDEL, in which R is D or V.

The terms "PE(ΔIII)-HIV gp120" and "PE(ΔIII)-HIV gp120 V3-V3" are interchangeable

The terms "PE(ΔIII)-HIV gp120-41" and "PE(ΔIII)-HIV gp120 C1-C5-gp41" are interchangeable.

The terms "HIV subtype A gp120 C1-C5-gp41" and "chimera A" are interchangeable; the terms "HIV subtype B gp120 CI-C5-gp41" and "chimera B" are interchangeable; the terms "HIV subtype C gp120 C1-C5-gp41" and "chimera C" are interchangeable, and so on.

Immunogens. To be an immunogen, the formulation need only be a mixture of a fusion protein construct as described herein and a biocompatible carrier fluid suitable for carrying and delivering a predetermined aliquot of the fusion protein construct to a prechosen site in the body of a living subject. Immunogens embodying the invention can be administered in any appropriate carrier for intradermal, subcutaneous, intramuscular, parenteral, intranasal, intravaginal, intrarectal, oral or intragastric administration. They can be introduced by any means that effect antigenicity in humans. The dosage administered will vary and be dependent upon the age, health, and weight of the recipient; the kind of concurrent treatment, ifany; the frequency of treatment; and the nature of the Immoral antibody response desired. If the immunogens are to be given intradermally, subcutaneously, intramuscularly, intravenously or parenterally, they will be prepared in sterile form; in multiple or single dose formats; and dispersed in a fluid carrier such as sterile physiological saline or 5% dextrose solutions commonly used with injectables. In addition, other methods of administration can be advantageously employed as well.

Vaccines. To be a prepared vaccine, the minimal formulation comprises a predetermined quantity of a fusion protein construct as described herein; a biocompatible carrier suitable for carrying and delivering a predetermined aliquot of a fusion protein construct to a prechosen site in the body of a living subject; and at least one adjuvant composition dispersed in the carrier fluid or coupled to the fusion protein construct. The vaccine, by definition, incorporates an immunogen and includes one or more adjuvants to facilitate or stimulate the immune response and to prolong the antigenic effect in-vivo over time. Among the useful adjuvant substances conventionally known are those compositions approved by the FDA (currently or pending for systemic and/or mucosal immunizations). Some are preferred for mucosally-administered vaccines and others are preferred for intragastric administered vaccines.

**Modes of administration.** Multiple modes of inoculation, the manner of introducing an immunogen or vaccine, are conventionally known and used. The systemic or parenteral forms of administration (introduction by injection orperfusion) typically include intraperitoneal, intravenous, intramuscular, subcutaneous, and subdermal inoculations. In contrast, mucosal modes of administration may include not only the intranasal and intragastric forms of introduction, but also oral, intravaginal, and intrarectal introductions.

### EXAMPLES

Without intent to limit the scope of the invention, exemplary instruments, apparatus, methods and their related results according to the embodiments of the present invention are given below. Note that titles or subtitles may be used in the examples for convenience of a reader, which in no way should limit the scope of the invention. Moreover, certain theories are proposed and disclosed herein; however, in no way they, whether they are right or wrong, should limit the scope of the invention so long as the invention is practiced according to the invention without regard for any particular theory or scheme of action.

### HIV-1 gp120 and gp41 Fusion Proteins

### EXAMPLE 1

### Selection of truncated segments from HIV Env proteins, gp120 and gp41

The amino acid sequences of HIV gp120 and gp41 were retrieved from the National Center of Biotechnology Information (NCBI, USA) database and entered into software for evaluation of antigenic determinant (epitopes) of the target proteins, and candidate segments for synthesis displayed on an evaluation plot. Antigenic determinant regions of the target protein were chosen for synthesis by a reverse genetic engineering technique. Several peptide segments were selected as target peptides based on the results of the evaluation software. The software DNA strider v1.0 was used to analyze whether the nucleotide sequences of the target peptides contained restriction enzyme sites. If present in the DNA sequence in disadvantageous places, changes were made within the appropriate codons without altering the amino acid sequence. The software checked the newly created sequence, and designed restriction sites at both termini of the DNA sequence to facilitate clowning. Codons for some amino acid residues, such as Arg, Ile, Gln, Pro, were modified to increase the expression of proteins in *E*. *coli* expression systems. Table 1 lists the selected peptide segments and their corresponding amino acid sequences.

### EXAMPLE 2

### Construction of chimeric target polypeptides gp120 V3-V3 and gp120 C1-C5-gp41

Two target peptides gp 120 V3-V3 and gp120 C1-C5-gp41 were constructed using the selected peptide segments as follows: Three truncated peptide segments having the amino acid sequences of SEQ ID NO. 1 (from gp120 C1 domain), SEQ ID NO. 2 (from gp120 C5 domain) and SEQ ID NO. 5 (from gp41 region associated with gp120), respectively, were ligated to form a chimeric target peptide gp 120 C1-C5-gp41 (referred to as gp 120-41). Two truncated segments having amino acid sequences of SEQ ID NOs: 3 and 4 (both from gyp120 V3 domains), respectively, were fused to form polypeptide gp120 V3-V3 (referred to as gp120). One or more residues might be inserted in-between to link two peptide segments. The number of residues inserted in-between was about 1 to 15 amino acids, which might be selected from amino acid residues that would not alter the secondary structure of proteins, such as glycine, alanine, valine, and leucine. The amino acid residue cysteine in SEQ ID NO: 2 and in SEQ ID NO: 5 could form a disulfide bond so that the chimeric target peptide generated could possess a three-dimensional structure.

**Table**

| HIV Env Proteins | Target peptide segments | Sequence of selected peptide segments | SEQ ID No. |
|---|---|---|---|
| | C I domain | VEKLWVTVYYGVPVWK | 1 |
| | C5 domain | KVVKIEPLGVAPTKCKRRVVQREKR | 2 |
| gp120 | V3 domain | CTRPSNNTRKGIHMGPGGAFYTTGQIIRNIRQAHC | 3 |
| | V3 domain | CTRPNNNTRRSIHIEPEGAFYTTGEIIGDIRQAHC | 4 |
| gp41 | gyp 41 region in association with gp120 | | 5 |

Table 2 lists the amino acid sequences of the chimeric target peptides gp120 V3-V3 and gp120 C1-C5-gp41. For chimeric peptide gp120 V3-V3 in table 2: the underlined letters denote the restriction sites; bold letters denote the first V3 domain segment, bold and italic letters denote the second V3 domain segment. For chimeric peptide gp120 C1-C5-gp41 in table 2: the bold letters denote the C I domain segment; non-bold letters denote the C5 domain segment; non-bold and italic letters denote linkers; bold and italic letters denote the gp41 domain; and the underlined letters denote a restriction site.

**Table 2**

| Chimeric target peptide | Amino acid sequence | SEQ ID No. |
|---|---|---|
| gp120 V3-V3 | | 6 |
| gp120 C1-C5-gp41 | | 7 |

The chimeric target peptide gp120 V3-V3 (SEQ ID NO: 6) was designed based on the construction of repeats in the V3 domain of gp 120, and the chimera gp120 C1-CS-gp41 (SEQ I D NO: 7) was based on simulation of the gp41 region in association with gp120. The chimera gp120 V3-V3 (SEQ ID NO: 6) comprises amino acid sequences of SEX ID NOs: 3 and 4 (both from gp120 V3 domains). The chimera gp120 C1-C5-gp41 comprises amino acid sequences of SEQ ID NOs:1 (from gp120 C1 domain), 2 (from gp120 C5 domain), and 5 (gp41 region in j unction with gp120) (Table 1). Disulfide bonds were formed due to cysteines in SEQ ID NO: 2 (from the C5 domain of gp120) and SEQ ID No. 5 (from gp41 région in junction with gp120).

Using the similar method as described above, the following chimeric target peptides were constructed: HIV subtype A gpl20 C1-C5-gp41; HIV subtype B gp120 C1-C5-gp41; H I V subtype C gp120 C1-C5-gp41; HIV subtype D gp120 C1-C5-gp41; HIV subtype E gp 120 C1-C5-gp41 HIV subtype F gp120 C1-C5-gp41; HIV subtype G gp120 C1-C5-gp41; HIV subtype H gp120 C1-C5-gp41 ; HIV subtype J gp120 C1-C5-gp41; and HIV subtype K gp120 C1-C5-gp41 (abbreviated as chimeric target peptides A, B, C, D, E, F, G, H, J, and K, respectively).

These chimeras A, B, C, D, E, F, G, H, J and K were each constructed from a combination of 3 peptide segments selected from each corresponding HIV-1 subtypes, A, B, C, D, F, G, H, J and K, respectively. The basic scheme of the construction was to link two peptide segments, each selected from the C1 and C5 domains of gp120, to another segment selected from the gp41 region in association with gp120 for each HIV-1 subrype. Thus, like the SEQ ID NO: 7, all these chimera target peptides have gp120-C1-C5-gp41 -like structures.

Table 3 lists the amino acid sequences of these chimeras, in which the non-bold, italic letters denote a segment from the C1 domain, the bold letters denote a segment from the C5 domain, of HIV subtype A gp120 protein; the non-bold, non-italic letters GGGGG denote a linker, and the bold, italic letters denote a segment from HIV subtype A gp41 region in junction with gp120.

**Table 3**

| Chimera for HIV subtype | Amino Acid Sequence | SEQ ID NO. |
|---|---|---|
| A | | 31 |
| B | | 32 |
| C | | 33 |
| D | | 34 |
| E | | 35 |
| F | | 36 |
| G | | 37 |
| H | | 38 |
| J | | 39 |
| K | | 40 |

### EXAMPLE 3

### Synthesis of DNA fragments encoding chimeric target polypeptides gp120 V3-V3 and gp120 C1-C5-gp41

The nucleotide sequences of the DNA fragments encoding chimeric target peptides gp120 V3-V3 and gp120 C1-C5-gp41 were modified to increase translation efficiency without changing the amino acid sequences of the encoded proteins, using the method disclosed in Taiwan patent application No. 092126644. The sequence modification allowed the encoded peptides or proteins to be efficiently expressed in *E*. *coli* pET plasmid expression system. Table 4 lists the modified sequences of the DNA flagments encoding the chimeric target peptides gp120 V3-V3 and gp120 C1-C5-gp41, in which non-italic, capital letters denote restriction enzyme linkers for EcoR1, Nde1 and SalI cutting sites, and italic, capital letters denote a XhoI restriction enzyme site.

**Table 4**

| Chimeric target peptide | Nucleotide sequence | SEQ ID No. |
|---|---|---|
| gp120 V3-V3 | | 8 |
| gp120 C1-C5-gp41 | | 9 |

Table 5 lists SEQ ID NOs. of respective primer pairs used for PCR synthesis of the DNA fragments encoding chimeric target peptides gp120 V3-V3 (SEQ ID NO: 8) and gp 120 C1-C5-gp41 (SEQ ID NO. 9). Non-DNA-template PCR reactions were performed by continuously using forward and reverse primer pairs to PCR synthesize DNA fragments. In the first-round PCR, the 3' end of the first forward primer (FI) had about 10-15 bases that were complementary to those in the pairing reversed primer (R1). The PCR profile was as follows: 5 min at 95°C, 1 min at 94°C, 0.5 min at 55°C, 1 min at 72°C for 20 cycles, and 1 min at 72°C. Following the first-round PCR, the 3' ends of the primer pairs (such as F2 and R2, F3 and R3, F4 and R4, or F5 and R5) had about 10-15 bases that were complementary to the previous round PCR product as a DNA template. After the first-round PCR, 001 - 1µl of the product was used as the DNA template for the second-round PCR. The second primer pair, F2 and R2, were added in a suitable amount together with dNTPs, reagents and Pfu polymerase, and the second round PCR was performed. Other primers were subsequently added in this manner so that the final extended DNA fragments were synthesized. All the DNA fragments synthesized from each primer pair and each round of PCR were analyzed for the size by gel electrophoresis. The DNA fragment encoding each individual chimeric target peptide was synthesized in this manner until the final PCR product was extended to the expected size, e.g., 264 bp in the case of gp120 V3-V3.

The DNA fragments (Table 6) encoding each individual chimeric target peptides gp120 C1-C5-gp41 for H1V subtype A, B, C, D, E, F, G, H, J and K were synthesized in a manner similar to the method described above using primer pairs listed in Table 6.

**Table 5**

| Chimeric target peptide | primer pairs | Forward Primer | SEQ ID NO. | Reverse Primer | SEQ ID NO. |
|---|---|---|---|---|---|
| gp120 V3-V3 | P1 | F1 | 10 | R1 | 15 |
| | P2 | F2 | 11 | R2 | 16 |
| | P3 | F3 | 12 | R3 | 17 |
| | P4 | F4 | 13 | R4 | 18 |
| | P5 | F5 | 14 | R5 | 19 |
| gp120 C1-C5-gp4 1 | P1 | F1 | 20 | R1 | 24 |
| | P2 | F2 | 21 | R2 | 25 |
| | P3 | F3 | 22 | R3 | 26 |
| | P4 | F4 | 23 | R4 | 27 |
| | P5 | F4 | 23 | R5 | 28 |
| | P6 | F4 | 23 | R6 | 29 |

### EXAMPLE 4

### Construction of plasmids for expression of HIV fusion proteins PE(ΔIII)-gp120 V3-V3 nnd PE(ΔIII)-gp120 C1-C5-gp41

Various chimeric target polypeptides of HIV-1 Env proteins as described above were cloned and expressed as PE fusion proteins. Briefly, a polypeptide from *Pseudomonas* Exotoxin A, i.e., PE(ΔIII), which was devoid of cytotoxic domain III, was fused to respective chimeric target polypeptides. Plasmid pPE(ΔIII) was constructed by inserting a DNA fragment encoding the binding domain I and translocation domain II of Pseudomonas exotoxin A into vector pET15a. A DNA sequence coding for a carboxyl terminal peptide that comprises the amino acid sequence KDEL (SEQ ID NO. 30) was ligated to the carboxyl terminal portion of the PE(ΔIII) gene, and generated plasmid pPE(ΔIII)-KDEL3 (referred to as pPE(ΔIII)-K3). The isolated DNA fragments generated by the PCR reaction were respectively digested by restriction enzymes *Eco*R I and *Xho* I, and then ligated to the *EcoR I, Xho I* sites of the plasmid pPE(ΔIII)-KDEL3, resulting in chimeric genes that would expressed target peptides as fusion proteins (Hung, C.F. et al (2001) "Cancer Immunotherapy Using a DNA Vaccine Encoding the Translocation Domain of a Bacterial Toxin Linked to a Tumor Antigen" Cancer Research 61:3698-3703).

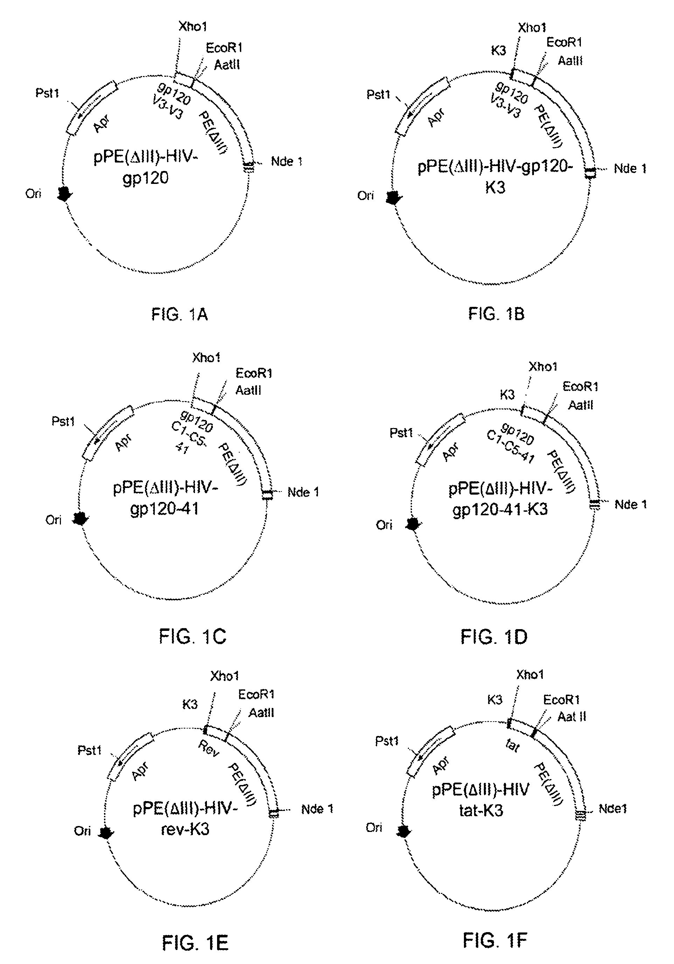

The plasmid pPE(ΔIII)-HIV gp120 (FIG. 1A) encodes a fusion protein PE(ΔIII)-HIV gp120 V3-V3 (referred to as PE(ΔIII)-HIV gp120). The plasmid pPE(ΔIII)-HIV gp120-K3 (FIG. 1B) encodes a fusion protein PE(ΔIII)-HIV gp120 V3-V3-K3 (referred to as PE(ΔIII)-HIV gp120-K3). The plasmid pPE(ΔIII)-HIV gp120-41 (FIG. 1C) encodes fusion protein PE(ΔIII)-HIV gp120 C1-C5-gp41 (referred to as PE(ΔIII)-HIV gp120 -41). The plasmid pPE(ΔIII)-HIV gp120-41-K3 (FIG. 1D) encodes the fusion protein PE(ΔIII)-HIV gp120 C1-C5-gp41-K3 (referred to as PE(ΔIII)-HIV gp120 -41-K3). The resulting plasmids were respectively transformed into *E. coli* Jam 109 to obtain clones and maintain the clones therein. The Jam109 strain could stably maintain the plasmid inside bacteria cells without protein expression.

**Table 6**

| Chimeric target peptide | Nucleotide Sequence ID NO. | primer pairs | Forward Primer | SEQ ID NO. | Reverse Primer | SEQ ID NO. |
|---|---|---|---|---|---|---|
| A | 41 | P1 | F1 | 51 | R1 | 56 |
| | | P2 | F2 | 52 | R2 | 57 |
| | | P3 | F3 | 53 | R3 | 58 |
| | | P4 | F4 | 54 | R4 | 59 |
| | | P5 | F5 | 55 | R5 | 60 |
| B | 42 | P1 | F1 | 61 | R1 | 66 |
| | | P2 | F2 | 62 | R2 | 67 |
| | | P3 | F3 | 63 | R3 | 68 |
| | | P4 | F4 | 64 | R4 | 69 |
| | | P5 | F5 | 65 | R5 | 70 |
| C | 43 | P1 | F1 | 71 | R1 | 76 |
| | | P2 | F2 | 72 | R2 | 77 |
| | | P3 | F3 | 73 | R3 | 78 |
| | | P4 | F4 | 74 | R4 | 79 |
| | | P5 | F5 | 75 | R5 | 80 |
| D | 44 | P1 | F1 | 81 | R1 | 86 |
| | | P2 | F2 | 82 | R2 | 87 |
| | | P3 | F3 | 83 | R3 | 88 |
| | | P4 | F4 | 84 | R4 | 89 |
| | | P5 | F5 | 85 | R5 | 90 |
| E | 45 | P1 | F1 | 91 | R1 | 96 |
| | | P2 | F2 | 92 | R2 | 97 |
| | | P3 | F3 | 93 | R3 | 98 |
| | | P4 | F4 | 94 | R4 | 99 |
| | | P5 | F5 | 95 | R5 | 100 |
| F | 46 | P1 | F1 | 101 | R1 | 106 |
| | | P2 | F2 | 102 | R2 | 107 |
| | | P3 | F3 | 103 | R3 | 108 |
| | | P4 | F4 | 104 | R4 | 109 |
| | | P5 | F5 | 105 | R5 | 110 |
| G | 47 | P1 | F1 | 111 | R1 | 116 |
| | | P 2 | F2 | 112 | R2 | 117 |
| | | P 3 | F3 | 113 | R3 | 118 |
| | | P4 | F4 | 114 | R4 | 119 |
| | | P5 | F5 | 115 | R5 | 120 |
| H | 48 | P1 | F1 | 121 | R1 | 126 |
| | | P 2 | F2 | 122 | R2 | 127 |
| | | P3 | F3 | 123 | R3 | 128 |
| | | P 4 | F4 | 124 | R4 | 129 |
| | | P5 | F5 | 125 | R5 | 130 |
| J | 49 | P1 | F1 | 131 | R1 | 136 |
| | | P 2 | F2 | 132 | R2 | 137 |
| | | P 3 | F3 | 133 | R3 | 138 |
| | | P 4 | F4 | 134 | R4 | 139 |
| | | P5 | F5 | 135 | R5 | 140 |
| K | 50 | P1 | F1 | 141 | R1 | 146 |
| | | P 2 | F2 | 142 | R2 | 147 |
| | | P 3 | F3 | 143 | R3 | 148 |
| | | P 4 | F4 | 144 | R4 | 149 |
| | | P5 | F5 | 145 | R5 | 150 |

### EXAMPLE 5

### Expression and analysis of target proteins

HIV-1 PE-fusion proteins were expressed in *E. coli* BL21(DE3) plys cultures containing corresponding expression plasmids. Briefly. 5 ml of bacterial seeds (A600 of 1.0±0.3 O.D.) were inoculated into 250 ml of liquid broth (LB) supplemented with 500 µg/ml ampicillin and 50 ml of 10% glucose at 37° C in a rotating incubator shaken at 150 rpm for 2-3 hours. Once O.D.₆₀₀ₙₘ reached 0.3±0.1, the bacterial culture was induced with isopropylthio-β-D-galactoside (IPTG; Promega, USA) at a final concentration of 0.1 to 2 mM at 37° C in a rotating incubator shaken at 150 rpm for 2 hours for protein expression.

Bacterial cells were pelleted after the protein induction was completed. After freezing and thawing of the pellet, bacterial cells were lysed with a solution containing in 10 ml: 0.3 mg/ml lysozyme, 1 mM phenylmethylsulfonyl fluoride (PMSF) and 0.06 mg/ml DNase I at room temperature for 20 minutes, followed by addition of 1 ml of 10% Triton X-100 and incubation at room temperature for 10 minutes. The lysed cells were centrifuged at 12,000 g for 10 minutes, and pellets were washed by 1 M and 2M urea solutions. Insoluble inclusion bodies containing recombinant proteins were collected and dissolved in 8 ml of 8M. urea solution or in an alkaline solution (pH 10 to 12) containing 1 to 3 M urea, and purified using a commercial pET His-Tag purification column system. The protein inclusion bodies dissolved in the urea solution were loaded onto a 4 ml Ni²⁺-nitrilotriacetic acid (Ni-NTA) resin affinity column, and the bound material eluted by different pH buffers (e.g. pH= 8.0, 7.0, 6.5, 6.0, 5.4, and 3.5) containing 1 to 6 M urea with 0.1 to 0.3 M NaCl, 5 to 50 mM phosphate buffer, and 5 to 50 mM Tris.

The eluted proteins were analyzed by sodium dodecyl sulfate-polyacrylamide gel electrophoresis (SDS-PAGE), and stained with coomassie blue. The optical densities of the bands in the gels were measured with a densitometer for evaluation of protein quantities. The concentration of fusion proteins, e.g., PE(ΔIII)-HIV gp120 C1-C5-gp41-K3 (referred to as gp120-41-K3) and PE(ΔIII)-HIV gp120 V3-V3-K3 (referred to as gp120-K3), in the eluted samples were about 0.8 mg/ml.

### EXAMPLE 6

### Antibody Assay

Materials and methods. PE-HIV envelope peptides used were: (1) PE(ΔIII)-HIV gp120; (2) PE(ΔIII)-HIV gp120-KDEL; (3) PE(V)-HIV gp120-41; and (4) PE(ΔIII)-HIV gp120-41-KDEL. An oil adjuvant, ISA 206, was used with each of the peptide immunogens in emulsified preparations for injection into mice.

Animals. BALB/c mice were purchased form Harlan laboratories and housed in the Laboratory animal Resources Facility of KUMC. All mice were used in accordance with AAALAC and the KUMC Institutional Animal Care and Use Committee guidelines.

**Immunization of animals with PE-HIV-Env fusion protein vaccines**. Four- to six- week old BALB/c mice were divided into 5 groups, with 6 mice per group. The animals in groups 1 to 5 received (1) PE(ΔIII)-HIV gp120, (2) PE(ΔIII)-HIV gp120-K3, (3) PE(ΔIII)-HIV gp120-41, (4) PE(ΔIII)-HIV gp120-41-K3, and (5) PBS (control group), in adjuvant, respectively, following the immunization schedule shown in Table 7. Immunized mice were then exsanguinated following deep anesthesia and blood and spleens were collected for immunological assays.

**Table 7**

| Group | No. of mice | Immunogen | Immunization Schedule | | | | |
|---|---|---|---|---|---|---|---|
| | | | 1^{st} time *(IM) 0 week | 2^{nd} time (IM) 2 weeks | 3^{rd} time (IM) 4 weeks | 4^{th} time (I/M) 6 weeks | 5^{th} time **(IP) 8 weeks |
| 1 | 6 | PE(ΔIII)-HIV gp120 | 50 µg | 25 µg | 25 µg | 25 µg | 50 µg |
| | | Adjuvant | 50 µl | 50 µl | 50 µl | 50 µl | 0 |
| 2 | 6 | PE(ΔIII)-HIV gp120-K3 | 50 µg | 25 µg | 25 µg | 25 µg | 50 µg |
| | | Adjuvant | 50 µl | 50 µl | 50 µl | 50 µl | 0 |
| 3 | 6 | PE(ΔIII)-HIV gp120-41 | 50 µg | 25 µg | 25 µg | 25 µg | 50 µg |
| | | Adjuvant | 50 µl | 50 µl | 50 µl | 50 µl | 0 |
| 4 | 6 | PE(ΔIII)-HIV gp120-41-K3 | 50 µg | 25 µg | 25 µg | 25 µg | 50 µg |
| | | Adjuvant | 50 µl | 50 µl | 50 µl | 50 µl | 0 |
| 5 | 6 | PBS | 0 | 0 | 0 | 0 | 50 µl |
| | | Adjuvant | 50 µl | 50 µl | 50 µl | 50 µl | 0 |

| | | | | | | | |
|---|---|---|---|---|---|---|---|
| *: IM denotes intramuscular injection **IP denotes intraperitoneal injection | | | | | | | |

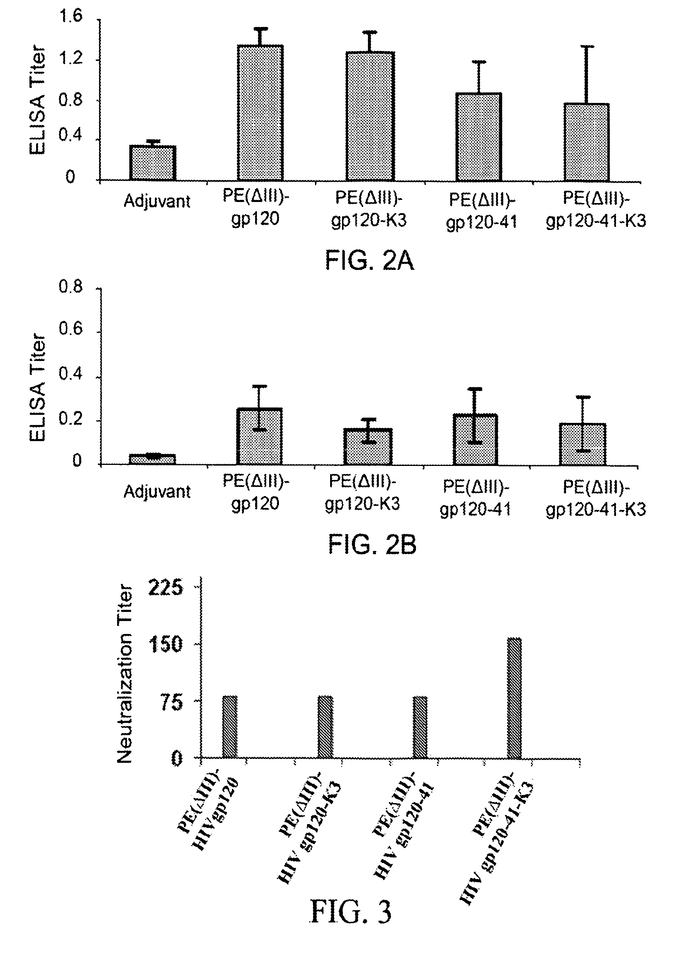

**Serum antibody binding assay**. An ELISA test using a commercial kit was used to determine binding antibody titers. Briefly, two weeks after the last immunization, mice were anesthetized, sacrificed, and spleens and blood samples were collected. Sera prepared from the blood samples of the animals immunized with fusion proteins in groups 1 to 4 and animals in the placebo group were serially diluted 500 x, 2500 x, 12500 x, and 62500 x, respectively. Binding antibody titers induced by the four peptide groups were analyzed using ELISA kit (BioChain) for detection of anti-HIV antibodies. Plates were coated with HIV antigen. The manufacturer's protocol was modified for using mouse sera by substituting the anti-human antibody with a goat anti-mouse serum conjugated with Horseradish peroxidase (HRP). Results were expressed as OD absorbance at 450 nm against the blank. FIGs. 2A-2B illustrate the test results of sera from animals immunized with respective fusion proteins. The absorbance data reflects the amount of antibody titers in serum samples. The result indicates that each fusion protein vaccine after being injected into animals was able to induce antibodies against HIV with good titers in the dilution of 1 : 2,500 (FIG. 2A) and 12,500 (FIG. 2B).

### EXAMPLE 7

### Immunized mouse sera neutralize HIV- attenuated live vaccine virus

Neutralization assays were performed using mouse sera against an attenuated live SHIV vaccine virus that was developed in the MMD Lab. The assays were performed in X4 GHOST cells using a plaque reduction assay. X4 GHOST cells were capable of harboring HIV virus plaques after being infected by an HIV-attenuated live vaccine virus. Briefly, serum samples collected from immunized mice were tested for their contents of HIV specific neutralizing antibodies. Briefly, quadruplicates of serial twofold dilutions of sera in RPMI 1640 medium were prepared in 96-well plates. Twenty plaque-forming units of virus were incubated with the two fold dilutions of serum samples from each immunized mouse and a normal serum for two hours at 37 C, respectively. The suspensions were then inoculated onto monolayers of X 4 GHOST cells that constitutively expressed the HIV LTR linked to GFP. These cultures were incubated for three days and then examined by immunofluorescence for determination of the numbers of fluorescence spots that represented successful virus hits. Neutralization titers of the serum samples were scored as the highest dilution of the immune serum sample that prevented development of 50% of the plaques induced with the control nonimmune serum. Mice developed neutralizing antibodies against the live vaccine virus (FIG. 3). The highest serum neutralization titers were obtained with sera from mice immunized with PE(ΔIII)- HIV gp120-41-KDEL.

### EXAMPLE 8

### Immunized mouse sera neutralize simian-human immunodeficiency virus (SHIV_{KU2})

**Immunization of animals**. Mice were immunized with higher dosages of PE-fusion protein vaccines using a similar protocol described above. Briefly, mice were divided into 5 groups (groups 6-10), with 6 mice per group, and received PE(ΔIII)-HIV gp120, PE(ΔIII)-HIV gp120-K3, PE(ΔIII)-HIV gp120-41, PE(ΔIII)-HIV gp120-41-K3, and PBS (control group), in adjuvant, respectively. The immunization schedule is shown in Table 8.

Two weeks after the third immunization, blood samples were collected from each group of animals and processed to obtain serum samples for assay of antibody titers. ELISA antibody assays showed similar titers in this experiment as those in Example 6.

To perform neutralization assays using SHIV_{KU2}, mice from each group were challenged by pathogenic SHIV_{KU2} vaccine (constructed by Dr. Narayan, University of Kansas Medical Center, US Patent No. 5, 849, 994). All four groups of the immunized animals developed neutralizing antibody titers of approximate I:20 against SHIV_{KU2}. Table 9 shows the survival data. PE(III) gp120-41 was the best antigen preparation for induction of neutralizing antibodies against SHIV_{KU2} since 5 out of 6 mice developed these antibodies.

**Table 8**

| Group | No. of mice | Immunogen | Immunization | | |
|---|---|---|---|---|---|
| | | | First time *(IM) 0 week | Second time (IM) 2 weeks | Third time **(Ip) 6 weeks |
| 6 | 6 | PE(ΔIII)HIVgp120 | 100 µg | 50 µg | 50 µg |
| | | Adjuvant | 100 µl | 50 µl | 0 |
| 7 | 6 | PE(ΔIII)-HIVgp120-K3 | 100 µg | 50 µg | 50 µg |
| | | Adjuvant | 100 µl | 50 µl | 0 |
| 8 | 6 | PE(ΔIII)-HIVgp120-4) | 100 µg | 50 µg | 50 µg |
| | | Adjuvant | 100 µl | 50 µl | 0 |
| 9 | 6 | PE(ΔIII)-HIVgp120-41-K3 | 100 µg | 50 µg | 50 µg |
| | | Adjuvant | 100 µl | 50 µl | 0 |
| 10 | 6 | PBS | 100 µl | 50 µl | 50 µl |
| | | Adjuvant | 100 µl | 50 µl | 0 |

| | | | | | |
|---|---|---|---|---|---|
| *IM: intramuscular injection with adjuvant ISA 206. **IP: intraperitoneal injection with adjuvant ISA 206. | | | | | |

**Table 9**

| Group | Vaccine | No. of live mice/ No. of challenged mice | Neutralization titer |
|---|---|---|---|
| 6 | PE(ΔIII)-HIV gp120 | 4/6 | 1:20 |
| 7 | PE(ΔIII)-HIV gp120-K3 | 1/6 | 1:20 |
| 8 | PE(ΔIIIl)-HIV gp120-41 | 5/6 | 1:20 |
| 9 | PE(ΔIII)-HIV gp 120-41-K3 | 3/6 | 1:20-40 |
| 10 | PBS/adjuvant* | 0/6 | - |

| | | | |
|---|---|---|---|
| *: Adjuvant: ISA 206. | | | |

### EXAMPLE 9

### HIV Gag24, Nef, Tat, and Rev Fusion Proteins

Materials and methods. HIV fusion proteins were tested for their immonogeniticies were: (I) HIV Gag24 fusion protein vaccines, PE(ΔIII)-HIV Gag24-K3 and PE(ΔIII)-HIV Gag24-gp120-41-K3; (II) HIV Nef fusion protein vaccines comprising PE(ΔIII)-HIV Nef-N-K3 and PE(ΔIII)-HIV Nef-C-K3; (III) HIV Tat fusion protein vaccine comprising PE(ΔIII)-HIV tat-K3; and (IV) HIV Rev fusion protein vaccine comprising PE(ΔIII)-HIV Rev-K3. The above (I) to (IV) HIV fusion proteins were constructed using similar methods described in Examples 2 to 4. Briefly, various polypeptide segments (Table 10) were selected from HIV proteins Gag24, Nef, Tat and Rev, respectively.

**Table 10**

| HIV Proteins | Targeted peptide segments | Amino acid sequence | SEQ ID No. |
|---|---|---|---|
| Gag24 | Full length Gag24 | | 151 |
| Nef | Nef- N terminus | | 152 |
| | Nef- C terminus | | 153 |
| Tat | Full length Tat | | 154 |
| Rev | Full length Rev | | 155 |

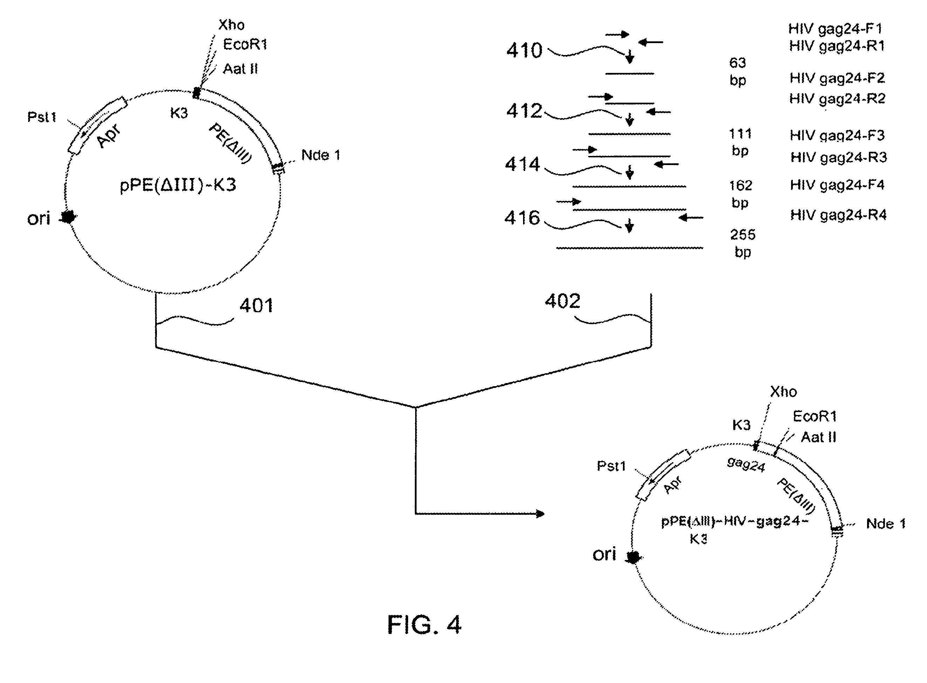

The DNA fragments (Table 11) encoding respective polypeptide segments were synthesized using primers listed in Table 11 by multi-round PCR synthesis method as described previously. Gel electrophoresis experiments were performed to examine the PCR products generated by each primer pair in the multiple-round PCR synthesis of DNA fragments, e.g., **410, 412, 414, 416** (FIG. 4). The PCR synthesized DNA fragments were fused to a PE fragment, cloned and expressed, respectively, using a similar method described in Example 4. The PE fragment is a polypeptide called PE (ΔIII) that contains a binding domain and a translocation domain from *Pseudomonas Exotoxin A* but lacks a cytotoxic domain. For example, the DNA fragment encoding gag24 generated by PCR was digested by restriction enzymes *EcoRI* and *Xho I* to isolate a 225 bp fragment, **402,** which was ligated to a PE(ΔIIIl) fragment within an *EcoRI* and *Xho I*-digested plasmid pPE(ΔIII)-KDEL3, **401**, to generate pPE(ΔIII)-HIV-gag24-K3 (FIG. 4).

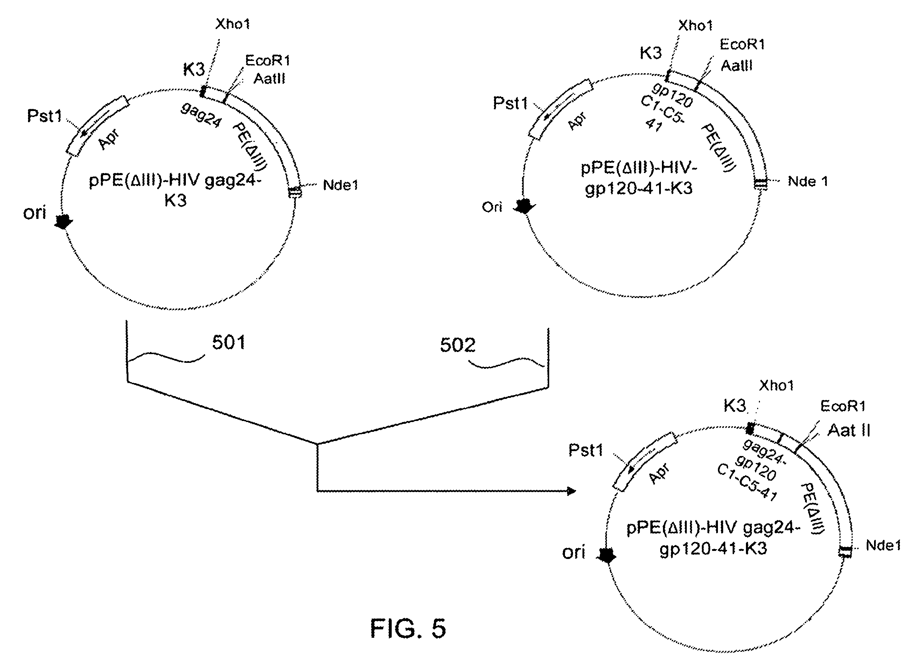

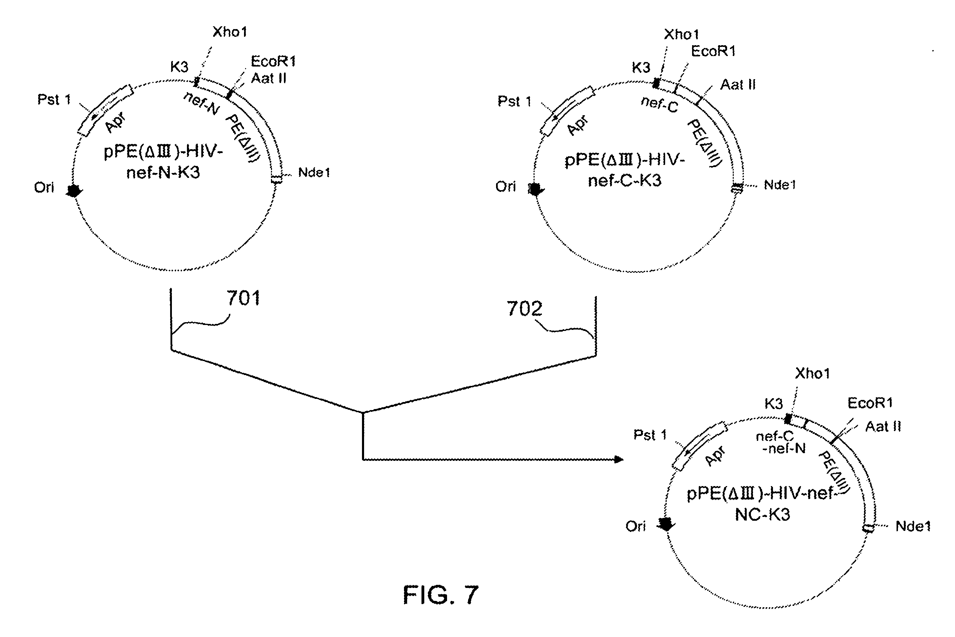

A 1.7 Kb gp120-41 fragment, isolated from *SalI* and *Pst I*-digested pPE(ΔIII)-HIV gp120. 41-K3, **502**, was fused to gag24 by inserting downstream, or C-terminal, to the *gag24* gene within a *PstI, Xho I*-digested plasmid pPE(ΔIII)-HIV gag24-K3, **501**, to generate plasmid pPE(ΔIII)-HIV gag24-gp120-41-K3 (FIG. 5). Plasmid pPE(ΔIII)-HIV nef-C-K3 was digested by Sal I and Pst I to isolate a 1.6 Kb fragment, **702**, followed by ligation to a nef-N fragment within a *Pst I. Xho I-*digested plasmid pPE(ΔIII)-HIV nef-N-KDEL, **701**, to generate pPE(ΔIII)-HIV nef-NC-KDEL (FIG. 7).

The DNA fragment encoding Tat or Rev generated by PCR was digested by restriction enzymes *EcoRI* and *Xho I* to isolate fragment, which was ligated to a PE(ΔIII) fragment within an *EcoRI* and *Xho I* -digested plasmid pPE(ΔIII)-KDEL3. By employing DNA recombinant method, plasmids pPE(ΔIII)-HIV Tat-K3 (FIG. IF), and pPE(ΔIII)-HIV Rev-K3 (FIG. 1E) were generated.

The sequence KDEL3 (i.e., K3) is an endoplasmic reticulum (ER) retention peptide located at the carboxyl terminal portion of the chimeric fusion protein. The sequence listing illustrates the nucleotide sequences of PE(ΔIII)-HIV Gag24-K3, PE(ΔIII)-HIV gag24-gp120-41-K3, PE-(ΔIII)-HIV nef-N-K3, pPE(ΔIII)-HIV nef-C-K3, pPE(ΔIII)- HIV-nef-NC-K3, pPE(III)-HIV rev-K3, pPE(ΔIII)-HIV-tat-K3 as SEQ ID NOs: 236, 238, 240, 242, 244, 246 and 248, and the corresponding amino acid sequences as SEQ ID NOs: 237, 239, 241, 243, 245, 247, and 249, respectively. For clinical applications, any undesired sequence such as oncogen sequences, if present in the bridge between PE(ΔIII) and HIV target peptide (e.g., between EcoRI and AatII), may be deleted without affecting the HIV target antigenic determinants.

**Table 11**

| Target peptide | Nucleotide Sequence ID NO. | primer pairs | Forward Primer | SEQ ID NO. | Reverse Primer | SEQ ID NO. |
|---|---|---|---|---|---|---|
| Gag24 | 156 | P1 | F1 | 161 | R1 | 165 |
| | | P2 | F2 | 162 | R2 | 166 |
| | | P3 | F3 | 163 | R3 | 167 |
| | | P4 | F4 | 164 | R4 | 168 |
| Nef- N terminus | 157 | P1 | F1 | 169 | R1 | 176 |
| | | P2 | F2 | 170 | R2 | 177 |
| | | P3 | F3 | 171 | R3 | 178 |
| | | P4 | F4 | 172 | R4 | 179 |
| | | P5 | F5 | 173 | R5 | 180 |
| | | P6 | F6 | 174 | R6 | 181 |
| | | P7 | F7 | 175 | R7 | 182 |
| | | P8 | F7 | 175 | R8 | 183 |
| | | P9 | F7 | 175 | R9 | 184 |
| Nef- C terminus | 158 | P1 | F1 | 185 | R1 | 189 |
| | | P2 | F2 | 186 | R2 | 190 |
| | | P3 | F3 | 187 | R3 | 191 |
| | | P4 | F4 | 188 | R4 | 192 |
| Tat | 159 | P1 | F1 | 193 | R1 | 200 |
| | | P2 | F2 | 194 | R2 | 201 |
| | | P3 | F3 | 195 | R3 | 202 |
| | | P4 | F4 | 196 | R4 | 203 |
| | | P5 | F5 | 197 | R5 | 204 |
| | | P6 | F6 | 198 | R6 | 205 |
| | | P7 | F7 | 199 | R7 | 206 |
| Rev | 160 | P1 | F1 | 207 | R 1 | 213 |
| | | P2 | F2 | 208 | R2 | 214 |
| | | P3 | F3 | 209 | R3 | 215 |
| | | P4 | F4 | 210 | R4 | 216 |
| | | P5 | F5 | 211 | R5 | 217 |
| | | P6 | F6 | 212 | R5 | 217 |

**Immunization of animals with fusion proteins**. Chimeric fusion proteins as described above were expressed for vaccination. Female mice C57BL/6J aged 6- to 8- week old were purchased from National Taiwan University (Taipei, Taiwan) and bred in Animal Center of National Taiwan University Hospital. Mice were divided into groups and injected three times at two-week intervals with respective fusion proteins or PBS (control group) in ISA 206 oil adjuvant (Table 12). Two weeks after the last immunization, mice were exsanguinated under deep anesthesia and blood and spleens were collected for immunological assays. An ELISA test using a commercial kit was used to determine binding antibody titers.

**Table 12**

| HIV target peptides | Vaccine* | No. ot Mice | Immunization Schedule | | |
|---|---|---|---|---|---|
| | | | First time 0 week | Second time 2 weeks | Third time 4 weeks |
| Placebo | PBS/Adjuvant | 3 | 100 µl | 100 µl | 100 µl |
| Gag24 | PE(ΔIII)-HIV Gag24-K3 | 4 | 100 µg | 100 µg | 100 µg |
| gp120 | PE(ΔIII)-HIV gp120-41-K3 | 4 | 100 µg | 100 µg | 100 µg |
| Gag24-gp120-41 | PE(ΔIII)-HIV Gag24-gp120-41-K3 | 3 | 100 µg | 100 µg | 100 µg |
| Nef | PE(ΔIII)-HIV Nef-N-K3 | 4 | 100 µg (50 µg each) | 100 µg (50 µg each) | 100 µg (50 µg each) |
| | PE(ΔIII)-HIV Nef-C-K3 | | | | |
| Tat | PE(ΔIII)-HIV Tat-K3 | 4 | 100 µg | 100 µg | 100 µg |
| Rev | PE(ΔIII)-HIV Rev-K3 | 4 | 100 µg | 100 µg | 100 µg |

| | | | | | |
|---|---|---|---|---|---|
| *: Vaccines were prepared by mixing fusion proteins or PBS with 50 µl of adjuvant ISA 206 before intramuscular (IM) injection. | | | | | |

**Serum Antibody Test.** Sera from mice were prepared and serially diluted 500 x, 2500 x, 12500 x, and 62500 x using the method described in Example 5. Antibody titers were measured using indirect ELISA analysis. ELISA plates were prepared and coated with corresponding peptides in Tables 13 and 14.

**Cytokine release assay.** Splenocyte cytokine levels in the medium of cultured cells were examined by ELISA to measure levels of cytokines TNF-α, γ-IFN, IL-4, IL-10 and IL-12. Briefly, spleens were aseptically collected from mice and dissociated to harvest splenocytes. Cells were resuspended in RPMI, and mononuclear cells were counted in a hemocytomer. Splenocytes were diluted to an optimal density and cultured in 5x10⁶ cells/well in 6-well plates in 5 ml of RPM I. Immunogen inducer peptides, e.g., Gag24-N and Gag24-C, etc., were respectively added to splenocytes in triplicate. On the second day after the addition of immunogens, supernatants were collected. The amounts of TNF-α, γ-IFN, IL-4, IL-10 and IL-12 produced by splenocyte CD8+ T cells were assayed using quantitative ELISA assay kits (Invitrogen BioSource) by following the manufacturer's protocol with slight modifications.

**Table 13**

| Antigen peptide | Peptide sequence | SEQ ID NO. | Length (a.a.) |
|---|---|---|---|
| gp120-41-N1 | VEKLWVTVYYGVPVWK | 218 | 16 |
| gp20-41-N2 | KVVKIEPLGVAPTKCK | 219 | 16 |
| gp120-41-N3 | APTKCKRRVVQREKR | 220 | 15 |
| gp120-41-C1 | QARVWRYLKDQQLL | 221 | 14 |
| gp120-41-C2 | GIWGCSGKLICCTTAVP | 222 | 17 |
| gp120-41-C3 | AVPWNASSWSNKLDR | 223 | 15 |

**Spleen lymphoid cell proliferation CMI assay.** Cell proliferation ELISA BrdU (colorimetric) assays for CMI reactions were performed. The steps for culturing splenocytes were similar to those used in cytokine release assay except that cells were cultured in 96-well plates. Briefly, immunogens or antigen peptides, e.g., Gag24-N, Gag24-C, etc., were respectively added to cell culture on day-2 to stimulate cell proliferation. ConA (10µg/ml), as a positive control, was added to stimulate cell2s for one day. Cells were pulse-labeled with BrdU on day-3 at 37°C for 12-24 hr. Only proliferating cells incorporated BrdU into their DNA. Cells were fixed with FixDenat solution. The FixDenat solution also denatured the genomic DNA, exposing the incorporated BrdU to immunodetection. The BrdU label in the DNA was located with a peroxidase-conjugated anti-BrdU antibody (anti-BrdU-POD). The bound anti-BrdU-POD was quantitated with a peroxidase substrate TMB by measuring absorbance at OD650 using ELISA plate reader.

**Table 14**

| Antigen peptide | Peptide sequence | SEQ ID NO. | Length (a.a.) |
|---|---|---|---|
| HIV-Tat-N | PVDPRLEPWKHPGSQPRTAC | 224 | 20 |
| HIV-Tat-C | QLRGDPTGPKESKKKVERET | 225 | 20 |
| HIV-Tat-M | SYGRKKRRQRRRAPQDSETH | 226 | 20 |
| HIV-Rev-N | QSNPYPKPEGYRRVRRNRRR | 227 | 20 |
| HIV-Rev-C | NCSESGGTSGTQRVGNPLEK | 228 | 20 |
| HIV-Nef-nl-N | SKLKKGWPTVRQRMDRTE | 229 | 18 |
| HIV-Nef-nl-C | TQGYFPDWQNYTPGPGIR | 230 | 18 |
| HIV-Nef-cl-N | VDPEQVEKANEGDNN | 231 | 15 |
| HIV-Nef-cl-M | ISQHGMDDPEKEVLM | 232 | 15 |
| HIV-Nef-cl-C | QHIAREKHPEYYKDCLGLEK | 233 | 20 |
| HIV-Gag24-N | PEFHMVDRDELKGIGMTN | 234 | 18 |
| HIV-Gag24-C | RMYSPTMTNNPPIPV | 235 | 15 |

Table 15 shows Gag24-specific antibodies titers in immunized mouse Sera. The antibody titer assay indicated that Gag24-N antigenic determinant or epitope peptide was stronger in inducing antibody reactions than the Gag24-C epitope peptide. The ability of Gag24-N peptide in inducing antibody titers was, however, weak when it was in the fusion protein PE(ΔIII)-Gag24-K3. Once the Gag24-N antigenic determinant peptide was modified to include polypeptide gyp120 and gp41 α-helix to form fusion protein PE(ΔIII)-Gag24-gp120-41-K3, its ability of inducing Gag24-N-specific IgG increased significantly. Thus, the peptide Gag24-N could elicit a Th2 cell-dependent, antigenic determinant (or epitope)-specific humoral immune response.

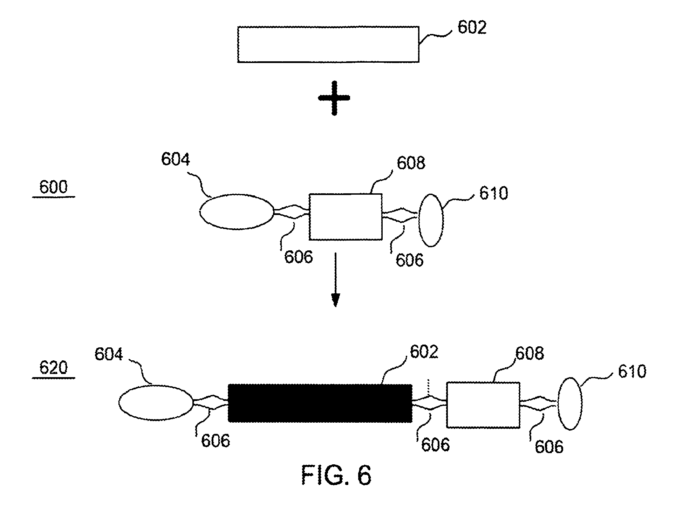

The results from the cell proliferation CMI assay indicated that both fusion proteins, PE(ΔIII)-Gag24-K3 and PE(ΔIII)-Gag24-gp 120-41-K3, after being injected into mice could induce cell-mediated immune response to Gag24 antigen (Table 16). The Gag24 antigen, however, had a low efficacy in inducing cell-mediated immune responses in the fusion protein PE(ΔIII)-Gag24-K3. Once it was modified to fuse with gp120 C1 and C5 domains and gp41 α-helix to form PE(ΔIII)-Gag24-gp120 C 1-C5-gp41-K3, Gag24 antigen's ability in inducing cell-mediated immune responses significantly increased. Thus, PE(ΔIII)-Gag24-gp120 C1-C5-gp41-K3 is much stronger than PE(ΔIII)-Gag24-K3 in inducing Gag24-specific, cell-mediated responses and cytokine release. As shown in FIG. 6, chimeric polypeptide HIV PE(ΔIII)-gp120 C1-C5-gp4 **600** can act as a building unit for connecting other HIV antigenic determinant peptide **602** and thereby markedly enhance cell-mediated immune responses of the inserted HIV antigenic peptide **602,** such as Gag24. Chimeric polypeptide HIV PE(ΔIII)-gp120 C1-C5-gp41 **600** includes PE(ΔIII) **604,** HIV gp120 C1-C5-gp41 **608,** an endoplasmic reticulum retention sequence **610,** with a bridge or linker **606** in-between. The fusion of an HIV antigenic determinant polypeptide **602** with weak CMI responses and HIV gp120 C1-C5-gp41 **608** results in a chimeric PE-HIV fusion protein **620** that exhibits enhanced CMI responses specific to the antigenic determinant **602.**

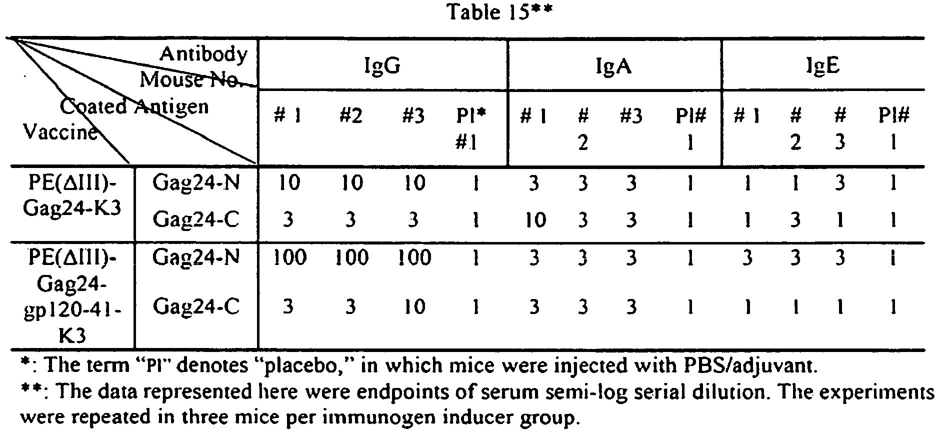

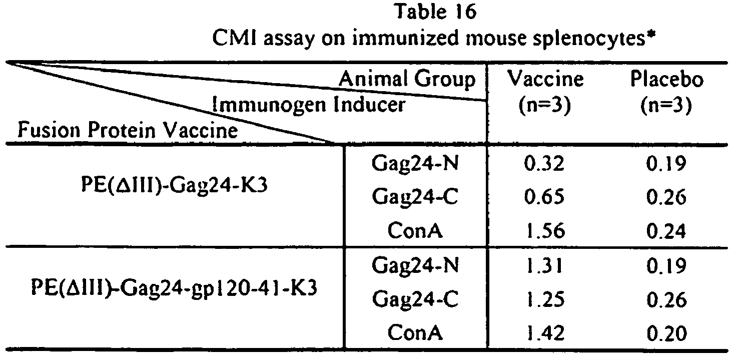

The data from the cytokine induction test (Table 17) showed that both vaccines PE(ΔIII)-Gag24-K3 and PE(ΔIII)-Gag24-gp120-41-K3 after being injected into mice did not induce detectable I L-4, which indicated that they would be better vaccine candidates for HIV. Of the two fusion protein vaccines, PE(ΔIII)-Gag24-gp120-41-K3 was much more effective than PE(ΔIII)-Gag24-K3 in inducing splenocytes to produce large amounts of IL-10 and IL-12. A comparison of Gag24-N and Gag24-C peptides in their cytokine inducing effects showed that in the PE(ΔIII)-Gag24-K3 vaccine group, Gag24-C peptide appeared to have a stronger T-cell-dependent epitope effect than Gag24-N peptide. In the PE(ΔIII)-Gag24-gp120-41-K3 vaccine group, both Gag24-N and Gag24-C peptides were capable of inducing cell-mediated immune responses and had no difference in their effects in inducing cytokine release. The data indicated that fusion protein PE(ΔIII)-Gag24-K3 had a low efficacy in inducing Gag24-specific cytokine release; however, fusion protein PE(ΔIII)-Gag24-gp120-41-K3 had a strong effect in eliciting Gag24-specific immune responses.

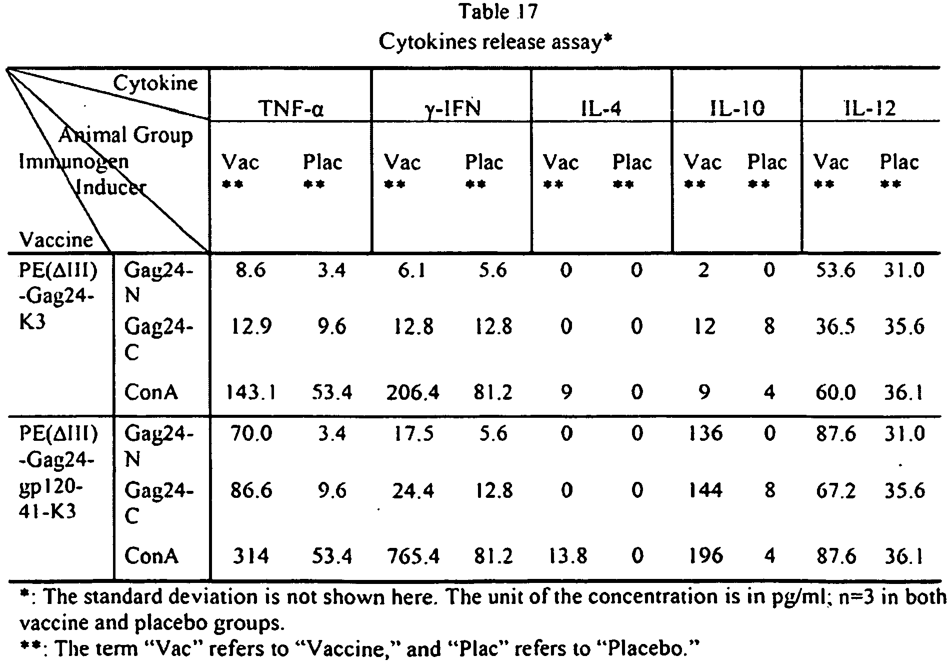

The data from the immunized mice Sera ELISA test indicated that the farthest C-terminal portion of Nef-C antigen determinant peptide had the strongest antibody reaction (Table 18). Based on the antibody- inducing reactions by fusion protein vaccine PE(ΔIII)-Nef-K3, it was concluded that peptide Nef-C-C was one of the Th2 cell-dependent, HIV antigenic determinant sites (Table 18).

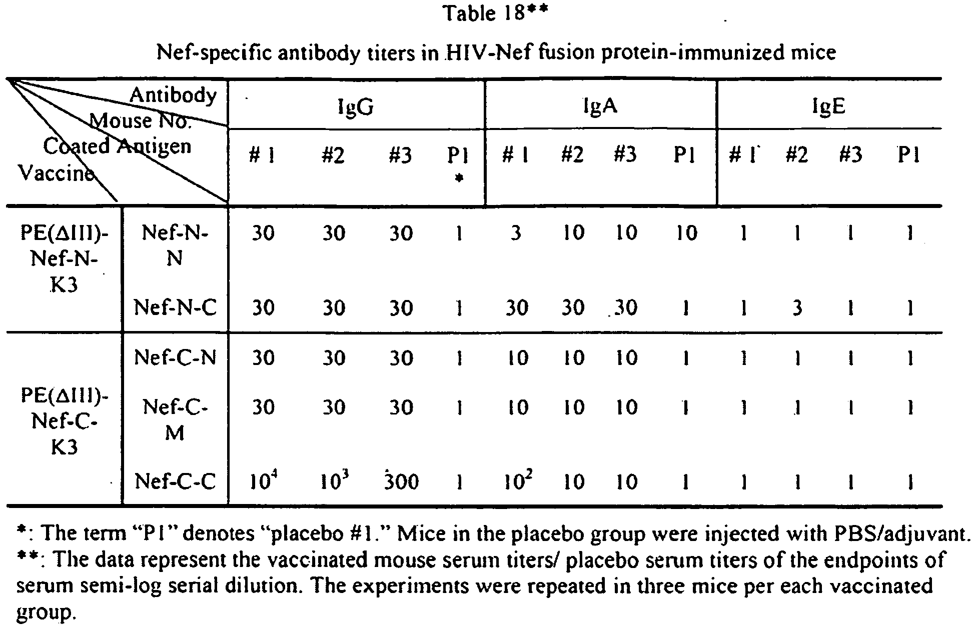

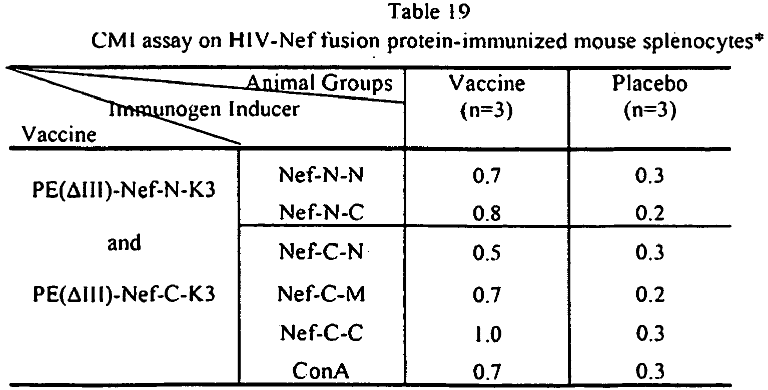

The cell-mediated immune responses in immunized mice indicated that both PE(ΔIII)-Nef-N-K3 and PE(ΔIII)-Nef-C-K3 had Nef- antigen-specific CMI reactions, and among which the Nef-N-C and Nef-C-C antigenic determinant portions induced stronger CMI responses (Table 19).

The data from the cytokine induction test showed that Nef fusion proteins did not induce detectable Nef-specific IL-4, which was an indication that Nef fusion proteins would be better vaccine candidates against HIV. The data also indicated that a vaccine composition comprising fusion proteins PE(ΔIII)-HIV-Nef-N-K3 and PE(ΔIII)-HIV Nef-C could stimulate splenocytes to produce a higher amount of IL-10 (Table 20).

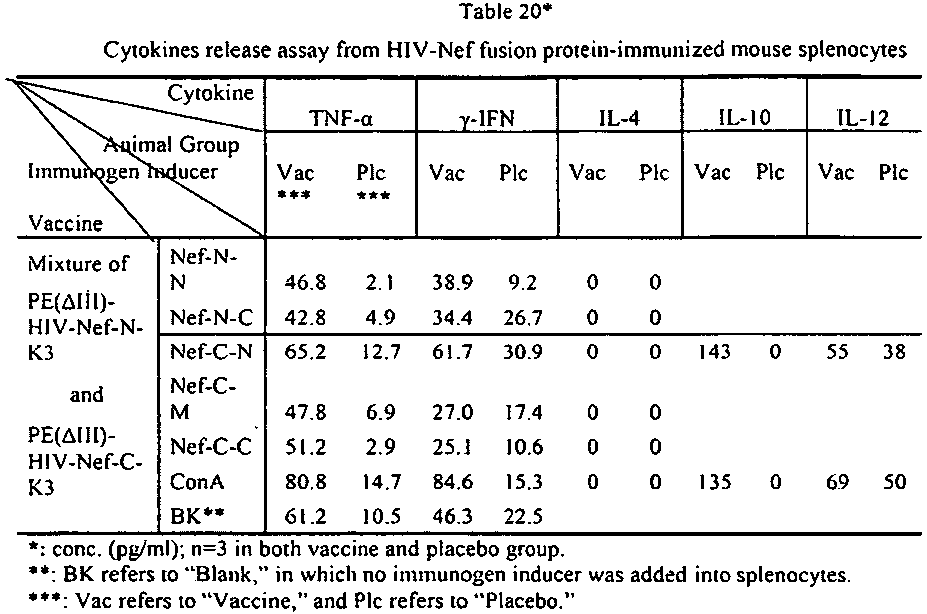

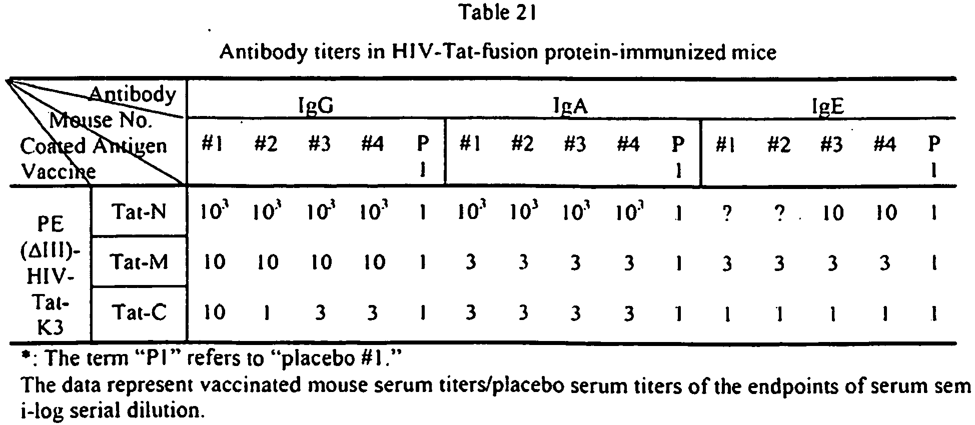

The results from serum antibody assay indicated that the N-terminal portion of HIV- Tat protein could induce remarkable antibody responses, while the mid-segment and C-terminal portions were weak in inducing antibody responses. Thus, the N-terminal portion of HIV Tat protein could elicit Th2 cell-dependent, antigenic determinant- specific humoral immunity (Table 21).

The results from the cell immune response indicated that PE(ΔIII)-Tat-K3 could induce cell mediated immune responses to all Tat protein segments, among which the N-terminus of Tat protein was stronger than mid- and C-terminal segments in inducing cell immune responses (Table 22).

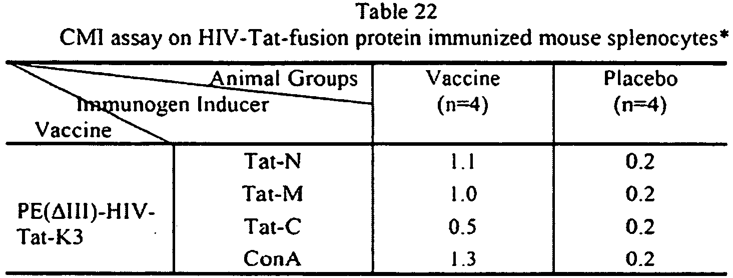

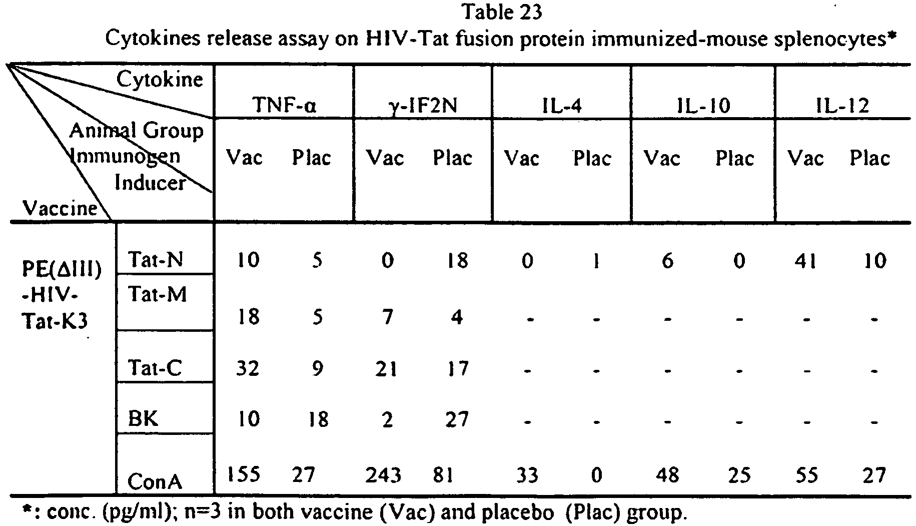

The results from cytokine release assay indicated that HIV Tat fusion protein was not able to induce a detectable level of Tat-specific IL-4. Its effects in inducing γ-IFN and TNF-α release were not obvious, either. The fusion protein PE-(ΔIII)-Tat-K3, however, was able to stimulate splenocytes to produce Tat-N terminus- specific IL-12. Thus, PE-(ΔIII)-Tat-K3 was still effective in inducing a cell immune response that was specific to the N terminal portion of Tat and therefore the N-terminus of Tat could evoke Th 1 cell-dependent, antigenic determinant-specific cell mediated immune responses in the PE delivery system of the present invention (Table 23).

The antibody data from PE(III)-Rev-K3 fusion protein- immunized mice model indicated that the antibody responses to HI V-Rev antigenic determinant peptide was not strong enough to confirm the locus of Th2 cell-dependent, antigenic determinant in Rev protein (Table 24).

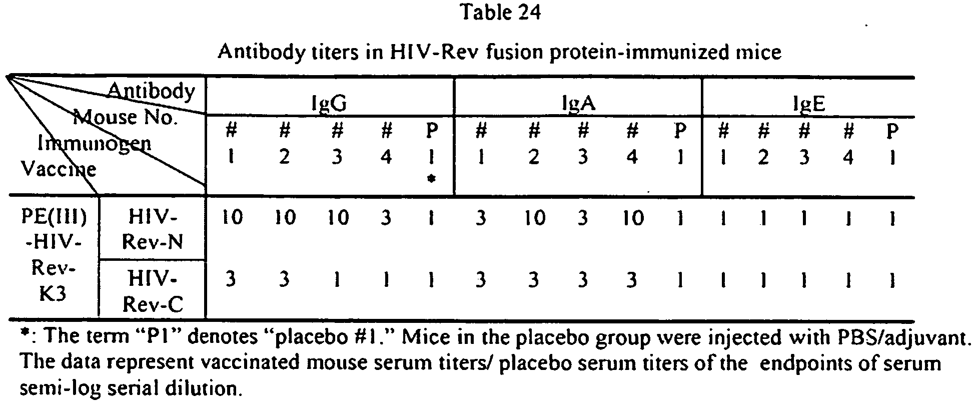

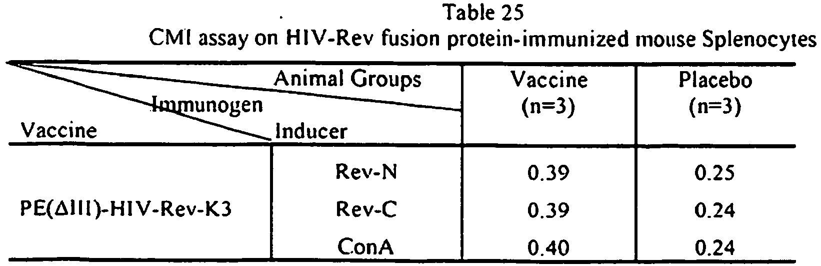

The data from cell immune responses in Table 25 indicated that PE(III)-Rev-K3 fusion protein vaccine was not able to induce cell immune response to Rev antigen. The cytokine release inducing test also gave the similar result. It showed no obvious effects in inducing TNF-α and γ-IFN release (Table 26). Thus, the fusion protein vaccine PE(ΔIII)-Rev-K3 might not be a good component in an HIV vaccine. However, whether Rev is able to elicit a CMI response under the condition of fusion with gp120-41 in a PE delivery system remains to be investigated A plasmid pPE(ΔIII)-HIV-Rev-gp120-41-K3 was constructed for investigation.

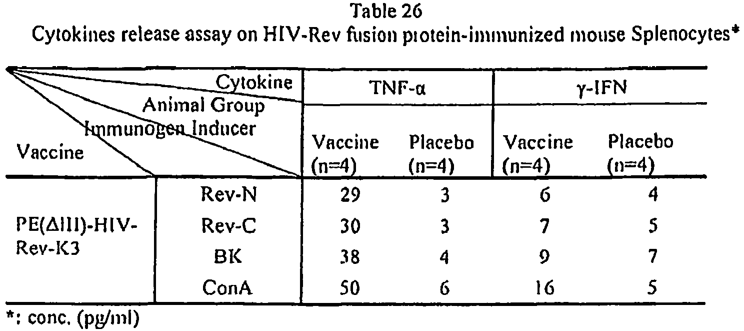

## Claims

1. A chimeric fusion protein comprising:
a) a first polypeptidyl region comprising a *Pseudomonas Exotoxin* A (PE) binding domain and a PE translocation domain, located at the N-terminus of the fusion protein; and
b) a second polypeptidyl region with a fusion peptide of HIV gp120-C1-C5-gp41 with the amino acid sequence of SEQ ID NO: 7.

2. A fusion protein as claimed in claim 1, further comprising an endoplasmic reticulum retention sequence at the C-terminus of the fusion protein.

3. A fusion protein as claimed in claim 2, further comprising an intermediate polypeptidyl region with the amino acid sequence of HIV Gag24 between the first and the second polypeptidyl regions.

4. A fusion protein as claimed in claim 2, wherein the endoplasmic reticulum retention sequence comprises the amino acid sequence KDEL.

5. A fusion protein as claimed in any one of the preceding claims, for use as an immunogen for inducing HIV antigen-specific immune responses.

6. A fusion protein for use as an immunogen as claimed in claim 5, for inducing neutralizing antibodies against HIV-1.

7. Use of a fusion protein as claimed in any one of claims 1 to 4 in the manufacture of a medicament for inducing HIV antigen-specific immune responses.

8. A use as claimed in claim 7, wherein the medicament is for inducing neutralizing antibodies against HIV-1.

## Patentansprüche

1. Chimäres Fusionsprotein, umfassend:
a) einen am N-Terminus des Fusionsproteins liegenden eine Pseudomonas-Exotoxin A (PE)-Bindungsdomäne und eine PE-Translokationsdomäne umfassenden ersten Polypeptidylbereich; und
b) einen zweiten Polypeptidylbereich mit einem Fusionspeptid von HIV-gp120-C1-C5-gp41 mit der Aminosäuresequenz von SEQ ID NO: 7.

2. Fusionsprotein nach Anspruch 1, ferner umfassend eine Endoplasmatisches-Retikulum-Retentionssequenz am C-Terminus des Fusionsproteins.

3. Fusionsprotein nach Anspruch 2, ferner umfassend einen intermediären Polypeptidylbereich mit der Aminosäuresequenz von HIV-Gag24 zwischen dem ersten und dem zweiten Polypeptidylbereich.

4. Fusionsprotein nach Anspruch 2, wobei die Endoplasmatisches-Retikulum-Retentionssequenz die Aminosäuresequenz KDEL umfasst.

5. Fusionsprotein nach einem der vorhergehenden Ansprüche zur Verwendung als Immunogen zur Induktion HIV-Antigen-spezifischer Immunantworten.

6. Fusionsprotein zur Verwendung als Immunogen nach Anspruch 5 zur Induktion neutralisierender Antikörper gegen HIV-1.

7. Verwendung eines Fusionsproteins nach einem der Ansprüche 1 bis 4 bei der Herstellung eines Arzneimittels zur Induktion HIV-Antigen-spezifischer Immunantworten.

8. Verwendung nach Anspruch 7, wobei das Arzneimittel der Induktion neutralisierender Antikörper gegen HIV-1 dient.

## Revendications

1. Protéine de fusion chimère, comprenant :
a) une première région polypeptidique comprenant un domaine de liaison à l'exotoxine A de *Pseudomonas* (PE) et un domaine de translocation de la PE, situé (au niveau du site N-terminal de la protéine de fusion ; et
b) une deuxième région polypeptidique, comportant un peptide de fusion du VIH gp120-C1-C5-gp41 avec la séquence d'acides aminés SEQ ID NO:7.

2. Protéine de fusion telle que revendiquée dans la revendication 1, comprenant en outre une séquence de rétention du réticulum endoplasmique au niveau du site C-terminal de la protéine de fusion.

3. Protéine de fusion telle que revendiquée dans la revendication 2, comprenant en outre une région polypeptidique intermédiaire ayant la séquence d'acides aminés du VIH Gag24 entre la première et la deuxième régions polypeptidiques.

4. Protéine de fusion telle que revendiquée dans la revendication 2, dans laquelle la séquence de rétention du réticulum endoplasmique comprend la séquence d'acides aminés KDEL.

5. Protéine de fusion telle que revendiquée dans l'une quelconque des revendications précédentes, pour utilisation en tant qu'immunogène pour induire des réponses immunes spécifiques de l'antigène du VIH.

6. Protéine de fusion pour utilisation en tant qu'immunogène telle que revendiquée dans la revendication 5, pour induire des anticorps neutralisants contre le VIH-1.

7. Utilisation d'une protéine de fusion telle que revendiquée dans l'une quelconque des revendications 1 à 4 pour la fabrication d'un médicament destiné à induire des réponses immunes spécifiques de l'antigène du VIH.

8. Utilisation telle que revendiquée dans la revendication 7, pour laquelle le médicament est destiné à induire des anticorps neutralisants contre le VIH-1.
